(19) Europäisches Patentamt
European Patent Office
Office européen des brevets

(11) EP 4 711 377 A1

(12) EUROPEAN PATENT APPLICATION
published in accordance with Art. 153(4) EPC

(43) Date of publication:
**18.03.2026 Bulletin 2026/12**

(21) Application number: **24803076.9**

(22) Date of filing: **10.05.2024**

(51) International Patent Classification (IPC):
***C07J 7/00*** (2006.01) ***C07J 5/00*** (2006.01)
***C07J 9/00*** (2006.01) ***A61K 31/58*** (2006.01)
***A61K 31/57*** (2006.01) ***A61K 31/56*** (2006.01)
***A61P 25/00*** (2006.01)

(52) Cooperative Patent Classification (CPC):
**A61K 31/56; A61K 31/57; A61K 31/58; A61P 25/00; C07J 5/00; C07J 7/00; C07J 9/00**

(86) International application number:
**PCT/CN2024/092185**

(87) International publication number:
**WO 2024/230797 (14.11.2024 Gazette 2024/46)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **11.05.2023 CN 202310527779**
**25.10.2023 CN 202311387436**
**29.01.2024 CN 202410117316**

(71) Applicants:
- **Shujing Biopharma Co., Ltd**
  **Shanghai 201208 (CN)**
- **Jiangsu NHWA Pharmaceutical Co., Ltd**
  **Xuzhou, Jiangsu 221000 (CN)**

(72) Inventors:
- **FENG, Jiaquan**
  **Shanghai 201208 (CN)**
- **SHI, Junjie**
  **Shanghai 201208 (CN)**
- **ZOU, Yuanhai**
  **Shanghai 201208 (CN)**
- **WAN, Zehong**
  **Shanghai 201208 (CN)**

(74) Representative: **Henderson, Helen Lee**
**Withers & Rogers LLP**
**2 London Bridge**
**London SE1 9RA (GB)**

# (54) STEROID COMPOUND, PREPARATION METHOD THEREFOR, AND USE THEREOF

(57) The present invention relates to a steroid compound, a preparation method therefor, and a use thereof. Specifically, the present invention provides a compound as shown in general formula (I), a stereoisomer thereof or pharmaceutically-acceptable salt thereof, a preparation method therefor, a pharmaceutical composition containing the compound, and a use thereof as a GABAA receptor modulator in the treatment of a CNS-related disease.

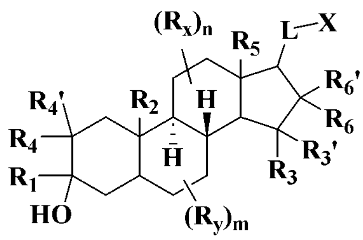

(I)

EP 4 711 377 A1

## Description

The present application claims priority to Chinese Patent Application No. 202310527779.1 filed on May 11, 2023, Chinese Patent Application No. 202311387436.6 filed on October 25, 2023, and Chinese Patent Application No. 202410117316.2 filed on January 29, 2024, which are incorporated herein by reference in their entirety.

TECHNICAL FIELD

The present disclosure relates to the field of pharmaceuticals, and particularly to a steroid compound, a preparation method therefor, and use thereof.

BACKGROUND

The GABAa receptor is a ligand-gated ionotropic receptor, which is primarily located at the apical region of the neuronal postsynaptic membrane and can trigger a fast inhibitory postsynaptic potential (IPSP). It is a heteropentameric protein composed of three homologous subunits. The subunits involved in the formation of the GABAa receptor include α1-α6, β1-β4, γ1-γ3, δ, ε, θ, π, and ρ1-ρ3. Each subunit consists of a large N-terminus, four hydrophobic transmembrane domains, a loop-shaped intracellular domain (containing phosphorylation sites for tyrosine kinases, protein kinase A, and protein kinase C), and a short C-terminus. Most GABAAR subunit combinations are composed of two α, two β, and one γ (δ) to form a heteropentamer. Different subunit compositions have corresponding effects on the physiological and pharmacological functions of the resulting GABAARs. In the mammalian brain, α1-β1-γ2 is the primary GABAAR composition type, while α2-β3-γ2 and α3-β3-γ2 are also relatively common. There is a GABA recognition site on the β subunit of GABAAR. Upon binding to GABA, the receptor is activated, such that the internal Cl- channel opens and selectively allows Cl- to enter the cell, and the cell membrane of the postsynaptic neuron undergoes hyperpolarization, thereby inhibiting electrical firing and generating a postsynaptic inhibitory potential (IPSP). This inhibitory effect produced at the postsynaptic membrane reduces the excitability of the nerve cell.

It is known that the membrane endocytosis and cycling of GABAAR are mediated by its β and γ subunits through binding to the clathrin adaptor protein AP2. Phosphorylation at Tyr and Ser sites on the binding sequence will affect the binding to AP2, thereby regulating the internalization process of the receptor and affecting the GABAergic inhibitory efficiency. Inhibitory dysfunctions in the GABA/GABAAR system may lead to an increased risk of developing neurological diseases.

Depression is a common neuropsychiatric disease characterized primarily by pathological emotional changes, mainly low mood. Globally, an estimated 5% of adults suffer from this disease. It is characterized by persistent sadness and a lack of interest or pleasure in previously rewarding or enjoyed activities. Depression is the leading cause of disability worldwide and greatly increases the global disease burden.

Research results suggest that both synaptic and extrasynaptic GABAa receptors may be involved in the pathogenesis of depression, and different subunits of GABAa receptors play different roles in the pathogenesis of depression. Although studies on changes in hippocampal GABAa receptor expression in depression have yielded inconsistent results, activation of GABAa receptors has been observed to exert antidepressant and anxiolytic effects. Activation of hippocampal GABAa receptors in rats can decrease Glu levels and increase GABA levels, thereby exerting an antidepressant effect. This suggests that modulating Glu and GABA levels may be one of the pathways through which GABAa receptors exert their antidepressant actions. In addition, studies using patch-clamp electrophysiological techniques have found that GABAa receptors can inhibit whole-cell currents mediated by Glu ionotropic receptors, indicating that GABAa receptors also have a regulatory effect on the function of Glu ionotropic receptors. Therefore, activating GABAa receptors exerts an antidepressant effect, and its antidepressant pathway is closely related to the glutamatergic system and GABAergic system.

However, over the past twenty years, there has been minimal innovation in the discovery and development of depression treatments, and the development goal of GABAa receptor modulators is to change patients' expectations by altering the treatment regimen for MDD. Currently, non-Chinese pharmaceutical enterprises, including Sage Therapeutics and Marinus, are investing heavily in developing GABAa receptor modulators.

Neurosteroid drugs targeting GABAa currently under development include: Alfaxalone, Alfadolone, Ganaxolone, Allopregnanolone, Sage-217, etc. Among them, Alfaxalone, Alfadolone, Ganaxolone, and Allopregnanolone have all clinically demonstrated that neurosteroid GABAa ligands can bring significant therapeutic effects. However, unfortunately, due to their high lipophilicity and rapid metabolism in the liver, these drugs exhibit very low oral bioavailability and are limited to injectable administration. Sage Therapeutics' Sage-217, as an oral GABAa receptor positive allosteric modulator, demonstrated significant, consistent, rapid, and sustained relief of depressive symptoms (including anxiety and insomnia), as well as acceptable tolerability and safety in clinical development programs. Thus, it has been granted Breakthrough Therapy designation by the U.S. FDA. However, subsequent investigations revealed that Sage-217 still has

many problems. For example, its highly lipophilic steroid backbone results in poor dissolution performance, and its inability to form salts precludes improvement of this suboptimal dissolution performance. Consequently, in clinical application, a relatively high dose is required to achieve therapeutic efficacy, thereby increasing costs and compromising patient compliance. Additionally, the drug suffers from poor absorption, so that patients need to take the drug with meals in clinical protocols. Most fatally, it has serious side effects. The FDA has issued a black box warning of "excessive sedation" for it. After taking the drug, patients cannot drive or operate heavy machinery within 12 hours. Therefore, the drug can only be taken with dinner and cannot be used for more than 14 days. This problem potentially limits its approval in MDD treatment.

Therefore, there is an urgent need to develop a class of novel steroid compounds, which have improved druggability properties, lower metabolic rates, and higher in vivo exposure, while maintaining reasonable lipophilicity to ensure the ability to cross the blood-brain barrier, and exhibiting reduced maximum agonistic effect on the target to alleviate adverse reactions, so that they can serve as safe and long-term usable brain excitability modulator drugs for the prevention and treatment of CNS-related diseases

CONTENT OF THE PRESENT INVENTION

The technical problem to be solved by the present disclosure is to provide a GABAa receptor modulator with a brand-new structure, and specifically to provide a steroid compound, a preparation method therefor, a pharmaceutical composition thereof, and use thereof. The compound has good positive modulatory effects on the GABAa receptor (e.g., having improved $E_{max}$ and/or $EC_{50}$), and/or improved pharmacokinetic properties (e.g., having a longer half-life $t_{1/2}$, greater exposure AUC, and a higher maximum plasma concentration $C_{max}$), and/or an improved safety window (e.g., having a larger safe administration dose range, or showing smaller probability of side effects occurring at the same dose), and/or improved safety (e.g., having lower toxicity and/or fewer side effects), and/or improved pharmacodynamic effects (e.g., having significant antiepileptic effects and/or significant antidepressant effects).

The present disclosure aims to provide a compound represented by general formula (I), a stereoisomer thereof, or a pharmaceutically acceptable salt thereof:

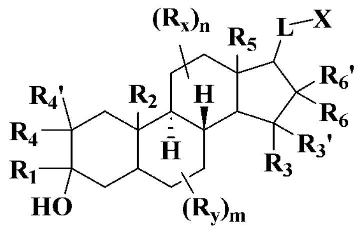

(I)

wherein:

X is hydrogen, $C_{1-6}$ alkyl, $C_{2-6}$ alkenyl, $C_{2-6}$ alkynyl, $C_{1-6}$ alkoxy, $C_{3-8}$ cycloalkyl, 3- to 8-membered heterocyclyl, $C_{6-10}$ aryl, or 5- to 10-membered heteroaryl, wherein the $C_{1-6}$ alkyl, $C_{2-6}$ alkenyl, $C_{2-6}$ alkynyl, $C_{1-6}$ alkoxy, $C_{3-8}$ cycloalkyl, 3- to 8-membered heterocyclyl, $C_{6-10}$ aryl, or 5- to 10-membered heteroaryl is optionally further substituted with one or more $R_A$;

$R_A$ is halogen, hydroxyl, $-NO_2$, -CN, $-NR_{aa}R_{bb}$, $-C(=O)R_{aa}$, $-C(=O)OR_{aa}$, $-OC(=O)R_{aa}$, $-OC(=O)OR_{aa}$, $-C(=O)NR_{aa}R_{bb}$, $-N(R_{aa})C(=O)R_{bb}$, $-OC(=O)NR_{aa}R_{bb}$, $-N(R_{aa})C(=O)OR_{bb}$, $-N(R_{cc})C(=O)NR_{aa}R_{bb}$, $-SR_{aa}$, $-S(=O)R_{aa}$, $-S(=O)_2R_{aa}$, $-S(=O)_2OR_{aa}$, $-OS(=O)_2R_{aa}$, $-S(=O)_2NR_{aa}R_{bb}$, $-S(=O)(=NR_{aa})R_{bb}$, $-N(R_{aa})S(=O)_2R_{bb}$, $-P(=O)R_{aa}R_{bb}$, $C_{1-6}$ alkyl, $C_{2-6}$ alkenyl, $C_{2-6}$ alkynyl, $C_{1-6}$ haloalkyl, $C_{1-6}$ alkoxy, $C_{1-6}$ haloalkoxy, $C_{3-8}$ cycloalkyl, or 3- to 8-membered heterocyclyl, wherein the $C_{1-6}$ alkyl, $C_{2-6}$ alkenyl, $C_{2-6}$ alkynyl, $C_{1-6}$ haloalkyl, $C_{1-6}$ alkoxy, $C_{1-6}$ haloalkoxy, $C_{3-8}$ cycloalkyl, or 3- to 8-membered heterocyclyl is optionally further substituted with one or more substituents selected from deuterium, halogen, hydroxyl, amino, sulfhydryl, nitryl, cyano, $C_{1-6}$ alkyl, $C_{1-6}$ haloalkyl, $C_{1-6}$ alkoxy, $C_{1-6}$ haloalkoxy, $C_{1-6}$ alkylthio, $C_{1-6}$ alkylamino, $C_{3-8}$ cycloalkyl, 3- to 8-membered heterocyclyl, $C_{6-10}$ aryl, and 5- to 10-membered heteroaryl;

$R_{aa}$, $R_{bb}$, and $R_{cc}$ are each independently hydrogen, $C_{1-6}$ alkyl, $C_{1-6}$ haloalkyl, $C_{3-8}$ cycloalkyl, 3- to 8-membered heterocyclyl, $C_{6-10}$ aryl, or 5- to 10-membered heteroaryl, or any two of $R_{aa}$, $R_{bb}$, and $R_{cc}$, together with the atom(s) to which they are attached, form 3- to 8-membered heterocyclyl or a 5- to 10-membered heteroaryl ring;

L is $-(CH_2)_{n1}-$, $-(CH_2)_{n1}O(CH_2)_{n2}-$, $-(CH_2)_{n1}S(CH_2)_{n2}-$, $-(CH_2)_{n1}NH(CH_2)_{n2}-$, $-(CH_2)_{n1}C(O)(CH_2)_{n2}-$, $-(CH_2)_{n1}S(O)(CH_2)_{n2}-$, $-(CH_2)_{n1}C(O)O(CH_2)_{n2}-$, $-(CH_2)_{n1}S(O)_2(CH_2)_{n2}-$, $-(CH_2)_{n1}C(O)NH(CH_2)_{n2}-$, $-(CH_2)_{n1}C(O)(CH_2)_{n2}NH-$, $-(CH_2)_{n1}NHC(O)(CH_2)_{n2}-$, or $-(CH_2)_{n1}NHS(O)_2(CH_2)_{n2}-$;

$R_x$ and $R_y$ are each independently hydrogen, deuterium, halogen, hydroxyl, amino, sulfhydryl, nitryl, cyano, $C_{1-6}$ alkyl,

$C_{1-6}$ haloalkyl, $C_{1-6}$ alkoxy, $C_{1-6}$ haloalkoxy, $C_{3-8}$ cycloalkyl, 3- to 8-membered heterocyclyl, $C_{6-10}$ aryl, or 5- to 6-membered heteroaryl, wherein the $C_{1-6}$ alkyl, $C_{1-6}$ haloalkyl, $C_{1-6}$ alkoxy, $C_{1-6}$ haloalkoxy, $C_{3-8}$ cycloalkyl, 3- to 8-membered heterocyclyl, $C_{6-10}$ aryl, or 5- to 6-membered heteroaryl is optionally further substituted with one or more substituents selected from deuterium, halogen, hydroxyl, amino, sulfhydryl, nitryl, cyano, $C_{1-6}$ alkyl, $C_{1-6}$ haloalkyl, $C_{1-6}$ alkoxy, and $C_{1-6}$ haloalkoxy;

$R_1$, $R_2$, $R_3$, $R_3$', $R_5$, $R_6$, and $R_6$' are each independently hydrogen, deuterium, halogen, hydroxyl, amino, sulfhydryl, nitryl, cyano, $C_{1-6}$ alkyl, $C_{2-6}$ alkenyl, $C_{2-6}$ alkynyl, $C_{1-6}$ haloalkyl, $C_{1-6}$ alkoxy, $C_{1-6}$ haloalkoxy, $-(CH_2)_{n3}R_{dd}$, $-(CH_2)_{n3}OR_{dd}$, $-(CH_2)_{n3}SR_{dd}$, $-(CH_2)_{n3}C(O)R_{dd}$, $-(CH_2)_{n3}C(O)OR_{dd}$, - $(CH_2)_{n3}S(O)R_{dd}$, $-(CH_2)_{n3}S(O)_2R_{dd}$, $-(CH_2)_{n3}NR_{dd}R_{ee}$, $-(CH_2)_{n3}C(O)NR_{dd}R_{ee}$, $-(CH_2)_{n3}S(O)NR_{dd}R_{ee}$, - $(CH_2)_{n3}NR_{dd}C(O)R_{ee}$, $-(CH_2)_{n3}NR_{dd}S(O)R_{ee}$, or $-(CH_2)_{n3}NR_{dd}S(O)_2R_{ee}$;

$R_{dd}$ and $R_{ee}$ are each independently hydrogen, $C_{1-6}$ alkyl, $C_{1-6}$ haloalkyl, $C_{3-8}$ cycloalkyl, 3- to 8-membered heterocyclyl, $C_{6-10}$ aryl, or 5- to 10-membered heteroaryl;

$R_4$ and $R_4$' are each independently hydrogen, halogen, hydroxyl, amino, sulfhydryl, nitryl, cyano, $C_{1-6}$ alkyl, $C_{2-6}$ alkenyl, $C_{2-6}$ alkynyl, $C_{1-6}$ haloalkyl, $C_{1-6}$ alkoxy, $C_{1-6}$ haloalkoxy, or $C_{3-8}$ cycloalkyl; and $R_4$ and $R_4$' are not both hydrogen;

n is an integer from 0 to 5;

m is an integer from 0 to 5;

n1 is an integer from 0 to 3;

n2 is an integer from 0 to 3; and

n3 is an integer from 0 to 3.

In a preferred embodiment, X is hydrogen, $C_{1-6}$ alkyl, $C_{6-10}$ aryl, or 5- to 10-membered heteroaryl containing 1-4 heteroatoms selected from N, O, and S, wherein the $C_{1-6}$ alkyl, $C_{6-10}$ aryl, or 5- to 10-membered heteroaryl containing 1-4 heteroatoms selected from N, O, and S is optionally further substituted with one or more $R_A$; preferably, X is hydrogen, $C_{6-10}$ aryl, or 5- to 10-membered heteroaryl containing 1-4 heteroatoms selected from N, O, and S, wherein the $C_{6-10}$ aryl or 5- to 10-membered heteroaryl containing 1-4 heteroatoms selected from N, O, and S is optionally further substituted with one or more $R_A$.

In a preferred embodiment, $R_A$ is halogen, -CN, $-NR_{aa}R_{bb}$, $-C(=O)R_{aa}$, $-C(=O)OR_{aa}$, - $C(=O)NR_{aa}R_{bb}$, $-S(=O)_2R_{aa}$, $-S(=O)_2NR_{aa}R_{bb}$, $-S(=O)(=NR_{aa})R_{bb}$, $-P(=O)R_{aa}R_{bb}$, $C_{1-6}$ alkyl, $C_{1-6}$ haloalkyl, $C_{1-6}$ alkoxy, $C_{3-8}$ cycloalkyl, or 3- to 8-membered heterocyclyl, preferably halogen, -CN, $-S(=O)_2R_{aa}$, - $S(=O)_2NR_{aa}R_{bb}$, $-S(=O)(=NR_{aa})R_{bb}$, $-P(=O)R_{aa}R_{bb}$, $C_{1-6}$ alkyl, $C_{1-6}$ haloalkyl, or $C_{1-6}$ alkoxy, more preferably halogen, -CN, $-S(=O)_2R_{aa}$, $-S(=O)_2NR_{aa}R_{bb}$, $-S(=O)(=NR_{aa})R_{bb}$, $-P(=O)R_{aa}R_{bb}$, $C_{1-3}$ alkyl, $C_{1-3}$ haloalkyl, or $C_{1-3}$ alkoxy, and further preferably fluorine, chlorine, -CN, methyl, trifluoromethyl, methoxy, $-SO_2CH_3$, - $SO_2NH_2$, $-SCH_3(=O)(=NH)$, $-SCH_3(=O)(=NCH_3)$, or $-P(=O)(CH_3)_2$.

In a preferred embodiment, $R_{aa}$, $R_{bb}$, and $R_{cc}$ are each independently hydrogen or $C_{1-6}$ alkyl, preferably hydrogen or $C_{1-3}$ alkyl, and more preferably hydrogen or methyl.

In a preferred embodiment, L is $-(CH_2)_{n1}-$, $-(CH_2)_{n1}C(O)(CH_2)_{n2}-$, $-(CH_2)_{n1}S(O)_2(CH_2)_{n2}-$, - $(CH_2)_{n1}C(O)NH(CH_2)_{n2}-$, $-(CH_2)_{n1}C(O)(CH_2)_{n2}NH-$, $-(CH_2)_{n1}NHC(O)(CH_2)_{n2}-$, or - $(CH_2)_{n1}NHS(O)_2(CH_2)_{n2}-$, preferably $-C(O)(CH_2)_{n2}-$, $-C(O)NH(CH_2)_{n2}-$, $-C(O)(CH_2)_{n2}NH-$, - $(CH_2)_nNHC(O)-$, or $-(CH_2)_{n1}NHS(O)_2-$, and more preferably $-C(O)(CH_2)_{n2}-$ or $-C(O)(CH_2)_{n2}NH-$.

In a preferred embodiment, $R_x$ and $R_y$ are each independently hydrogen, deuterium, halogen, hydroxyl, $C_{1-6}$ alkyl, $C_{1-6}$ haloalkyl, $C_{1-6}$ alkoxy, or $C_{3-8}$ cycloalkyl, preferably hydrogen, deuterium, halogen, hydroxyl, or $C_{1-6}$ alkyl, and more preferably hydrogen.

In a preferred embodiment, $R_1$, $R_2$, and $R_5$ are each independently hydrogen, deuterium, halogen, hydroxyl, $C_{1-6}$ alkyl, $C_{2-6}$ alkenyl, $C_{2-6}$ alkynyl, $C_{1-6}$ haloalkyl, $C_{1-6}$ alkoxy, $-(CH_2)_{n3}OR_{dd}$, $-(CH_2)_{n3}SR_{dd}$, or - $(CH_2)_{n3}C(O)R_{dd}$, preferably hydrogen, deuterium, $C_{1-3}$ alkyl, $C_{2-4}$ alkenyl, or $C_{2-4}$ alkynyl, more preferably hydrogen or $C_{1-3}$ alkyl, and further preferably hydrogen or methyl.

In a preferred embodiment, $R_3$, $R_3$', $R_6$, and $R_6$' are each independently hydrogen, deuterium, or $C_{1-6}$ alkyl, preferably hydrogen, deuterium, or $C_{1-3}$ alkyl, more preferably hydrogen or $C_{1-3}$ alkyl, and further preferably hydrogen or methyl.

In a preferred embodiment, $R_4$ and $R_4$' are each independently hydrogen or $C_{1-6}$ alkyl, preferably hydrogen or $C_{1-3}$ alkyl, and more preferably hydrogen or methyl; and $R_4$ and $R_4$' are not both hydrogen.

In a preferred embodiment, $R_{dd}$ and $R_{ee}$ are each independently $C_{1-6}$ alkyl or $C_{1-6}$ haloalkyl, preferably $C_{1-3}$ alkyl.

In a preferred embodiment, n is selected from 0, 1, 2, 3, and 4, preferably 0, 1, or 2.

In a preferred embodiment, m is selected from 0, 1, 2, 3, and 4, preferably 0, 1, or 2.

In a preferred embodiment, n1 is selected from 0, 1, and 2, preferably 0 or 1.

In a preferred embodiment, n2 is selected from 0, 1, and 2, preferably 0 or 1, and more preferably 1.

In a preferred embodiment, n3 is selected from 0, 1, and 2, preferably 0 or 1.

In a preferred embodiment, general formula (I) further has a structure represented by general formula (II):

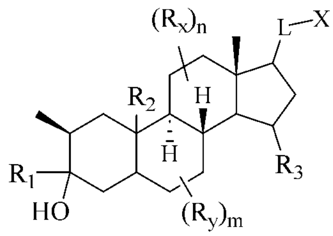

**(II)**

wherein X, L, $R_1$, $R_2$, $R_3$, $R_x$, $R_y$, n, and m are as described above.

In a preferred embodiment, general formula (I) further has a structure represented by general formula (III):

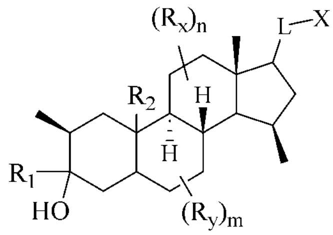

**(III)**

wherein X, L, $R_1$, $R_2$, $R_x$, $R_y$, n, and m are as described above.

In a preferred embodiment, general formula (I) further has a structure represented by general formula (IV):

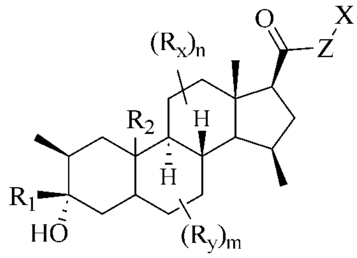

**(IV)**

wherein:

- Z is $-(CH_2)_{n4}-$ or $-NH(CH_2)_{n4}-$, preferably $-(CH_2)_{n4}-$, and more preferably $-CH_2-$;
- $R_1$ is hydrogen, deuterium, halogen, hydroxyl, $C_{1-6}$ alkyl, $C_{2-6}$ alkenyl, $C_{2-6}$ alkynyl, $C_{1-6}$ haloalkyl, $C_{1-6}$ alkoxy, $-(CH_2)_{n3}OR_{dd}$, $-(CH_2)_{n3}SR_{dd}$, or $-(CH_2)_{n3}C(O)R_{dd}$, preferably $C_{1-6}$ alkyl, more preferably $C_{1-3}$ alkyl, and further preferably methyl;
- $R_2$ is hydrogen, deuterium, halogen, hydroxyl, $C_{1-6}$ alkyl, $C_{2-6}$ alkenyl, $C_{2-6}$ alkynyl, $C_{1-6}$ haloalkyl, $C_{1-6}$ alkoxy, $-(CH_2)_{n3}OR_{dd}$, $-(CH_2)_{n3}SR_{dd}$, or $-(CH_2)_{n3}C(O)R_{dd}$, preferably hydrogen or $C_{1-6}$ alkyl, more preferably hydrogen or $C_{1-3}$ alkyl, and further preferably hydrogen or methyl;
- $R_{dd}$ is $C_{1-6}$ alkyl or $C_{1-6}$ haloalkyl, preferably $C_{1-3}$ alkyl;
- n4 is 0, 1, or 2, preferably 0 or 1;
- X, $R_x$, $R_y$, n, and m are as described above.

In a preferred embodiment, general formula (I) further has a structure represented by general formula (V) or general formula (V-A):

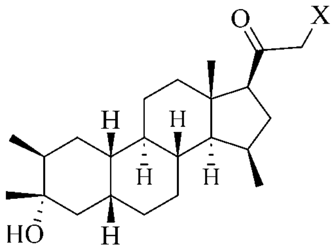

(V)

or

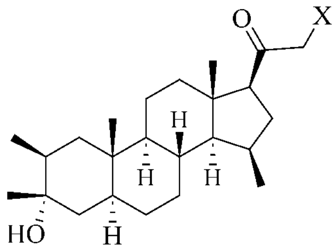

(V-A)

,

wherein:

X is hydrogen or 5- to 10-membered heteroaryl containing 1-4 heteroatoms selected from N, O, and S and optionally substituted with one or more $R_A$, preferably hydrogen or 5- to 10-membered heteroaryl containing 1-4 nitrogen atoms and optionally substituted with one or more $R_A$;

$R_A$ is halogen, hydroxyl, -CN, $-NR_{aa}R_{bb}$, $-C(=O)R_{aa}$, $-C(=O)OR_{aa}$, $-OC(=O)R_{aa}$, $-OC(=O)OR_{aa}$, $-C(=O)NR_{aa}R_{bb}$, $-N(R_{aa})C(=O)R_{bb}$, $-OC(=O)NR_{aa}R_{bb}$, $-N(R_{aa})C(=O)OR_{bb}$, $-N(R_{cc})C(=O)NR_{aa}R_{bb}$, $-SR_{aa}$, $-S(=O)R_{aa}$, $-S(=O)_2R_{aa}$, $-S(=O)_2OR_{aa}$, $-OS(=O)_2R_{aa}$, $-S(=O)_2NR_{aa}R_{bb}$, $-S(=O)(=NR_{aa})R_{bb}$, $-N(R_{aa})S(=O)_2R_{bb}$, $-P(=O)R_{aa}R_{bb}$, $C_{1-6}$ alkyl, $C_{2-6}$ alkenyl, $C_{2-6}$ alkynyl, $C_{1-6}$ haloalkyl, $C_{1-6}$ alkoxy, $C_{1-6}$ haloalkoxy, $C_{3-8}$ cycloalkyl, or 3- to 8-membered heterocyclyl, preferably halogen, -CN, $-S(=O)_2R_{aa}$, $-S(=O)_2NR_{aa}R_{bb}$, $-S(=O)(=NR_{aa})R_{bb}$, $-P(=O)R_{aa}R_{bb}$, $C_{1-6}$ alkyl, $C_{1-6}$ haloalkyl, or $C_{1-6}$ alkoxy, more preferably halogen, -CN, - S(=O)2$R_{aa}$, $-S(=O)_2NR_{aa}R_{bb}$, $-S(=O)(=NR_{aa})R_{bb}$, $-P(=O)R_{aa}R_{bb}$, $C_{1-3}$ alkyl, $C_{1-3}$ haloalkyl, or $C_{1-3}$ alkoxy, and further preferably fluorine, chlorine, -CN, methyl, trifluoromethyl, methoxy, $-SO_2CH_3$, $-SO_2NH_2$, - $SCH_3(=O)(=NH)$, $-SCH_3(=O)(=NCH_3)$, or $-P(=O)(CH_3)_2$;

$R_{aa}$, $R_{bb}$, and $R_{cc}$ are each independently hydrogen, $C_{1-6}$ alkyl, or $C_{1-6}$ haloalkyl, preferably hydrogen or $C_{1-6}$ alkyl, more preferably hydrogen or $C_{1-3}$ alkyl, and further preferably hydrogen or methyl.

In a preferred embodiment, in general formula (Ia), general formula (I), general formula (II), general formula (III), and general formula (IV), X is hydrogen or the following group:

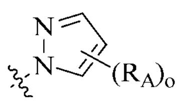 , 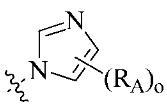 , 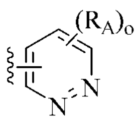 , 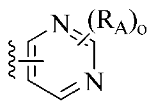 , 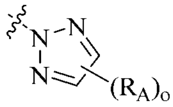 , 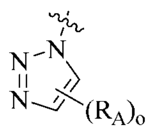 ,

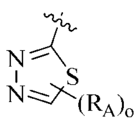 , 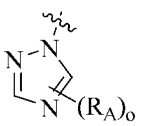 , 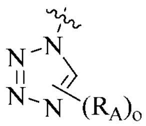 , 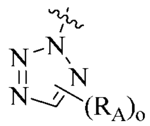 , 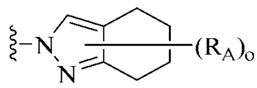 , 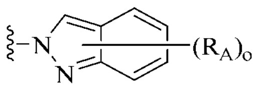 ,

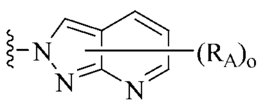 , 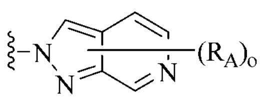 , 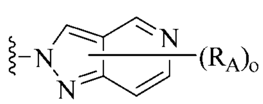 , 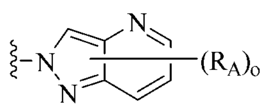 ,

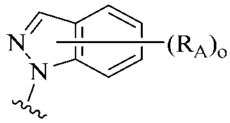 , 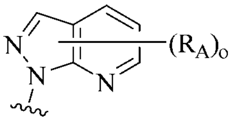 , 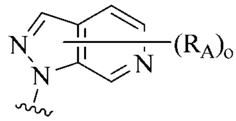 , 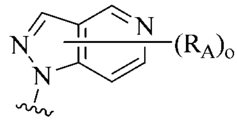 ,

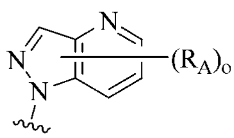 , 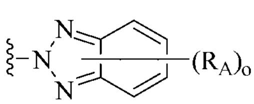 , 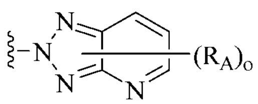 , 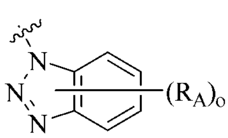 ,

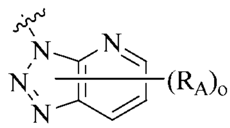, 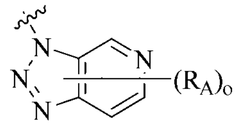, 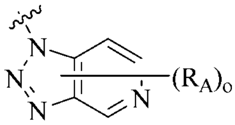, or 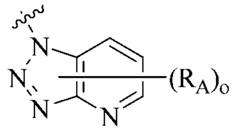,

preferably hydrogen,

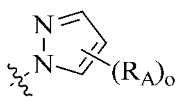, 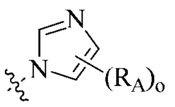, 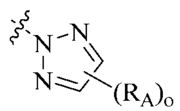, 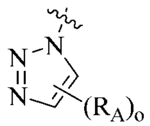,

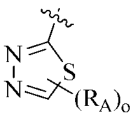, 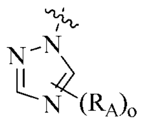, 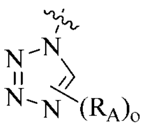, 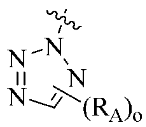, 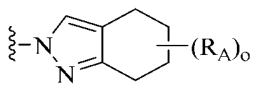, 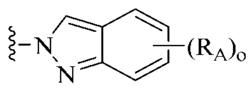,

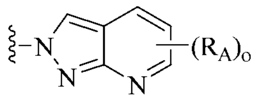, 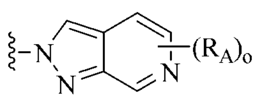, 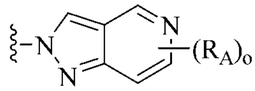, 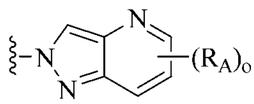,

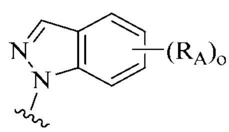, 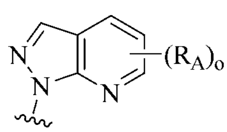, or 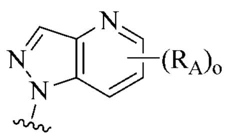;

$R_A$ is halogen, hydroxyl, -CN, $-NR_{aa}R_{bb}$, $-C(=O)R_{aa}$, $-C(=O)OR_{aa}$, $-OC(=O)R_{aa}$, $-OC(=O)OR_{aa}$, - $C(=O)NR_{aa}R_{bb}$, $-N(R_{aa})C(=O)R_{bb}$, $-OC(=O)NR_{aa}R_{bb}$, $-N(R_{aa})C(=O)OR_{bb}$, $-N(R_{cc})C(=O)NR_{aa}R_{bb}$, $-SR_{aa}$, - $S(=O)R_{aa}$, $-S(=O)_2R_{aa}$, $-S(=O)_2OR_{aa}$, $-OS(=O)_2R_{aa}$, $-S(=O)_2NR_{aa}R_{bb}$, $-S(=O)(=NR_{aa})R_{bb}$, $-N(R_{aa})S(=O)_2R_{bb}$, $-P(=O)R_{aa}R_{bb}$, $C_{1-6}$ alkyl, $C_{2-6}$ alkenyl, $C_{2-6}$ alkynyl, $C_{1-6}$ haloalkyl, $C_{1-6}$ alkoxy, $C_{1-6}$ haloalkoxy, $C_{3-8}$ cycloalkyl, or 3- to 8-membered heterocyclyl, preferably halogen, -CN, $-S(=O)_2R_{aa}$, $-S(=O)_2NR_{aa}R_{bb}$, - $S(=O)(=NR_{aa})R_{bb}$, $-P(=O)R_{aa}R_{bb}$, $C_{1-6}$ alkyl, $C_{1-6}$ haloalkyl, or $C_{1-6}$ alkoxy, more preferably halogen, -CN, - $S(=O)2R_{aa}$, $-S(=O)_2NR_{aa}R_{bb}$, $-S(=O)(=NR_{aa})R_{bb}$, $-P(=O)R_{aa}R_{bb}$, $C_{1-3}$ alkyl, $C_{1-3}$ haloalkyl, or $C_{1-3}$ alkoxy, and further preferably fluorine, chlorine, -CN, methyl, trifluoromethyl, methoxy, $-SO_2CH_3$, $-SO_2NH_2$, - $SCH_3(=O)(=NH)$, $-SCH_3(=O)(=NCH_3)$, or $-P(=O)(CH_3)_2$;

$R_{aa}$, $R_{bb}$, and $R_{cc}$ are each independently hydrogen, $C_{1-6}$ alkyl, or $C_{1-6}$ haloalkyl, preferably hydrogen or $C_{1-6}$ alkyl, more preferably hydrogen or $C_{1-3}$ alkyl, and further preferably hydrogen or methyl;

o is selected from 0, 1, 2, 3, and 4, preferably 0, 1, or 2, and more preferably 0 or 1.

In a preferred embodiment of the present disclosure, general formula (I) further has a structure represented by general formula (VI):

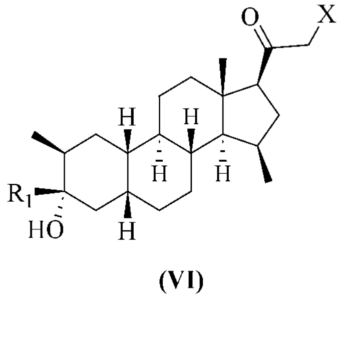

**(VI)**

wherein:

$R_1$ is hydrogen, deuterium, halogen, hydroxyl, $C_{1-6}$ alkyl, $C_{2-6}$ alkenyl, $C_{2-6}$ alkynyl, $C_{1-6}$ haloalkyl, or $C_{1-6}$ alkoxy,

preferably hydrogen, deuterium, or $C_{1-6}$ alkyl, more preferably hydrogen, deuterium, or $C_{1-3}$ alkyl, and further preferably hydrogen, deuterium, or methyl;

X is hydrogen or 5- to 10-membered heteroaryl containing 1-4 heteroatoms selected from N, O, and S and optionally substituted with one or more $R_A$, preferably hydrogen or 5- to 10-membered heteroaryl containing 1-4 nitrogen atoms and optionally substituted with one or more $R_A$, and more preferably hydrogen,

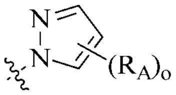 , 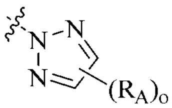 , 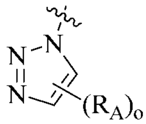 , 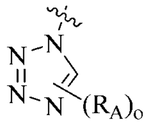 , 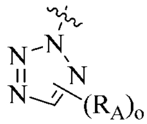 , 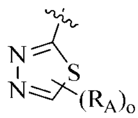 ,

or

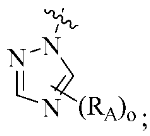 ;

$R_A$ is halogen, hydroxyl, cyano, $C_{1-6}$ alkyl, $C_{2-6}$ alkenyl, $C_{2-6}$ alkynyl, $C_{1-6}$ haloalkyl, $C_{1-6}$ alkoxy, or $C_{1-6}$ haloalkoxy, preferably cyano, $C_{1-6}$ alkyl, or $C_{1-6}$ haloalkyl, more preferably cyano, $C_{1-3}$ alkyl, or $C_{1-3}$ haloalkyl, and further preferably cyano, methyl, or trifluoromethyl;

o is selected from 0, 1, and 2, preferably 0 or 1.

In a preferred embodiment of the present disclosure, general formula (VI) further has a structure represented by general formula (VIa) or (VIb):

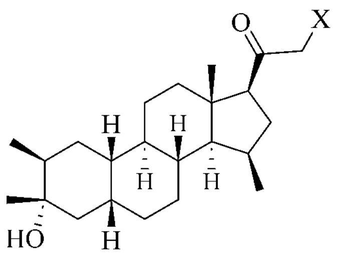

**(VIa)** or

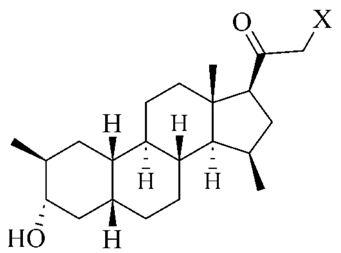

**(VIb)** ,

wherein:

X is hydrogen or 5- to 10-membered heteroaryl containing 1-4 heteroatoms selected from N, O, and S and optionally substituted with one or more $R_A$, preferably hydrogen or 5- to 10-membered heteroaryl containing 1-4 nitrogen atoms and optionally substituted with one or more $R_A$, and more preferably hydrogen,

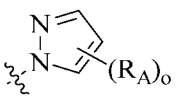 , 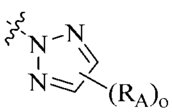 , 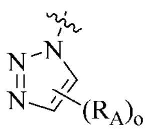 , 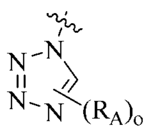 , 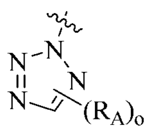 , 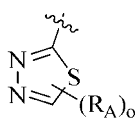 ,

or

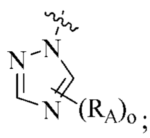 ;

$R_A$ is halogen, hydroxyl, cyano, $C_{1-6}$ alkyl, $C_{2-6}$ alkenyl, $C_{2-6}$ alkynyl, $C_{1-6}$ haloalkyl, $C_{1-6}$ alkoxy, or $C_{1-6}$ haloalkoxy,

preferably cyano, $C_{1-6}$ alkyl, or $C_{1-6}$ haloalkyl, more preferably cyano, $C_{1-3}$ alkyl, or $C_{1-3}$ haloalkyl, and further preferably cyano, methyl, or trifluoromethyl;
o is selected from 0, 1, and 2, preferably 0 or 1.

In a preferred embodiment of the present disclosure, general formula (I) further has a structure represented by general formula (VII):

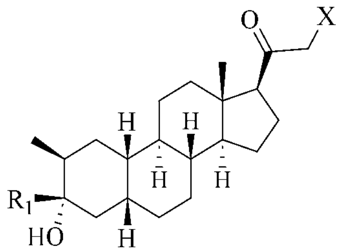

**(VII)**

wherein:

$R_1$ is hydrogen, deuterium, halogen, hydroxyl, $C_{1-6}$ alkyl, $C_{2-6}$ alkenyl, $C_{2-6}$ alkynyl, $C_{1-6}$ haloalkyl, or $C_{1-6}$ alkoxy, preferably hydrogen, deuterium, or $C_{1-6}$ alkyl, more preferably hydrogen, deuterium, or $C_{1-3}$ alkyl, and further preferably hydrogen, deuterium, or methyl;
X is 5- to 10-membered heteroaryl containing 1-4 heteroatoms selected from N, O, and S and optionally substituted with one or more $R_A$, preferably 5- to 10-membered heteroaryl containing 1-4 nitrogen atoms and optionally substituted with one or more $R_A$, and more preferably

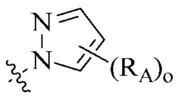 , 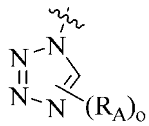 ,

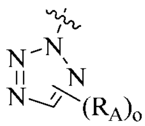 , or 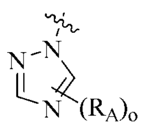 ;

$R_A$ is halogen, hydroxyl, cyano, $C_{1-6}$ alkyl, $C_{2-6}$ alkenyl, $C_{2-6}$ alkynyl, $C_{1-6}$ haloalkyl, $C_{1-6}$ alkoxy, or $C_{1-6}$ haloalkoxy, preferably cyano or $C_{1-6}$ alkyl, more preferably cyano or $C_{1-3}$ alkyl, and further preferably cyano or methyl;
o is selected from 0, 1, and 2, preferably 0 or 1.

In a preferred embodiment of the present disclosure, general formula (VII) further has a structure represented by general formula (VIIa) or (VIIb):

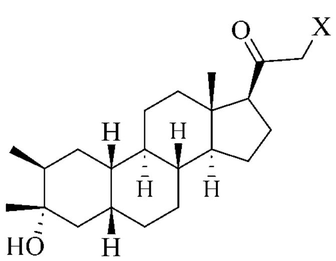

**(VIIa)** or

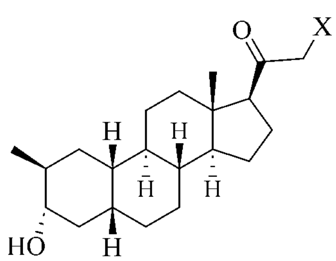

**(VIIb)** ,

wherein:

X is 5- to 10-membered heteroaryl containing 1-4 heteroatoms selected from N, O, and S and optionally substituted with one or more $R_A$, preferably 5- to 10-membered heteroaryl containing 1-4 nitrogen atoms and optionally substituted with one or more $R_A$, and more preferably

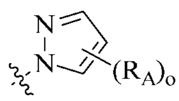, 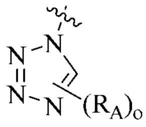,

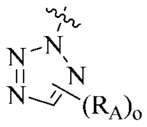, or 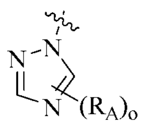;

$R_A$ is halogen, hydroxyl, cyano, $C_{1-6}$ alkyl, $C_{2-6}$ alkenyl, $C_{2-6}$ alkynyl, $C_{1-6}$ haloalkyl, $C_{1-6}$ alkoxy, or $C_{1-6}$ haloalkoxy, preferably cyano or $C_{1-6}$ alkyl, more preferably cyano or $C_{1-3}$ alkyl, and further preferably cyano or methyl;
o is selected from 0, 1, and 2, preferably 0 or 1.

In a preferred embodiment of the present disclosure, general formula (I) further has a structure represented by general formula (VIII):

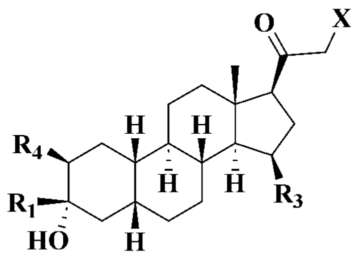

**(VIII)**

wherein:

$R_1$ is hydrogen, deuterium, or $C_{1-6}$ alkyl, preferably hydrogen, deuterium, or $C_{1-3}$ alkyl, and more preferably hydrogen or methyl;
$R_3$ is hydrogen, deuterium, or $C_{1-6}$ alkyl, preferably hydrogen, deuterium, or $C_{1-3}$ alkyl, and more preferably hydrogen or methyl;
$R_4$ is $C_{1-6}$ alkyl, preferably $C_{1-3}$ alkyl, and more preferably methyl;
X is 5-membered heteroaryl containing 1-4 nitrogen atoms and optionally substituted with one or more $R_A$, preferably

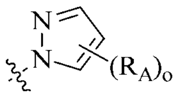, 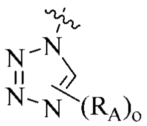, 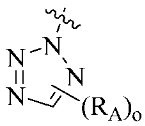, 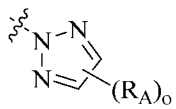, 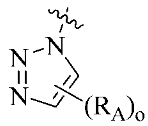,

or

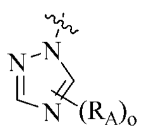;

$R_A$ is halogen, -CN, $C_{1-6}$ alkyl, $C_{1-6}$ haloalkyl, $C_{1-6}$ alkoxy, or $C_{3-8}$ cycloalkyl, preferably halogen, -CN, $C_{1-3}$ alkyl, $C_{1-3}$ haloalkyl, or $C_{1-3}$ alkoxy, and more preferably fluorine, chlorine, -CN, methyl, ethyl, trifluoromethyl, or methoxy;
o is selected from 0, 1, and 2.

In a preferred embodiment of the present disclosure, general formula (VIII) further has a structure represented by general formula (VIII-A) or (VIII-B):

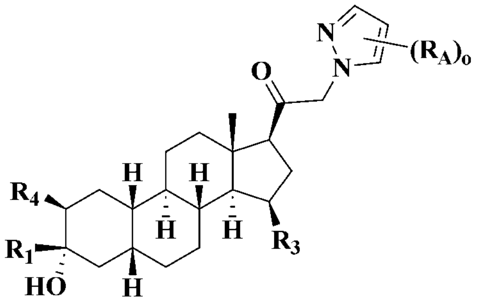

(VIII-A)

or

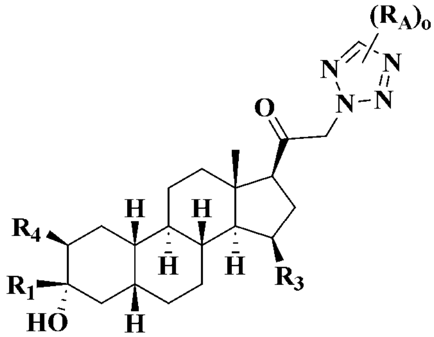

(VIII-B)

,

wherein:

$R_1$ is hydrogen, deuterium, or $C_{1-6}$ alkyl, preferably hydrogen, deuterium, or $C_{1-3}$ alkyl, and more preferably hydrogen or methyl;

$R_3$ is hydrogen, deuterium, or $C_{1-6}$ alkyl, preferably hydrogen, deuterium, or $C_{1-3}$ alkyl, and more preferably hydrogen or methyl;

$R_4$ is $C_{1-6}$ alkyl, preferably $C_{1-3}$ alkyl, and more preferably methyl;

$R_A$ is halogen, -CN, $C_{1-6}$ alkyl, $C_{1-6}$ haloalkyl, $C_{1-6}$ alkoxy, or $C_{3-8}$ cycloalkyl, preferably halogen, -CN, $C_{1-3}$ alkyl, $C_{1-3}$ haloalkyl, or $C_{1-3}$ alkoxy, and more preferably fluorine, chlorine, -CN, methyl, ethyl, trifluoromethyl, or methoxy;

o is selected from 0, 1, and 2.

In a preferred embodiment of the present disclosure, the compound is selected from:

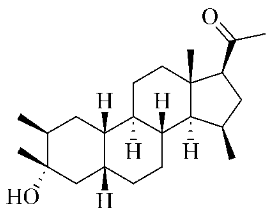

1 ,

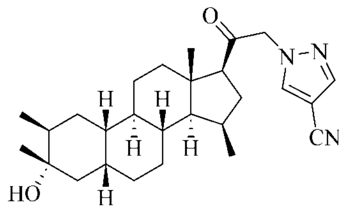

2 ,

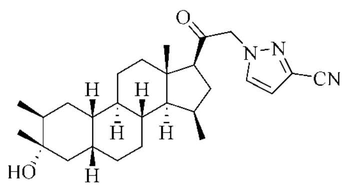

3 ,

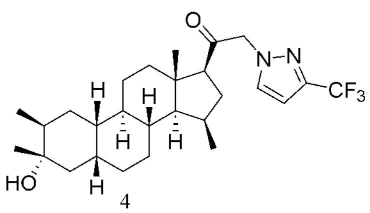

4 ,

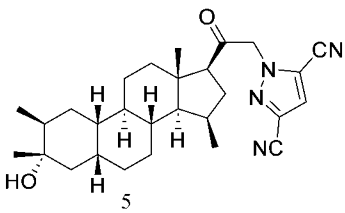

5 ,

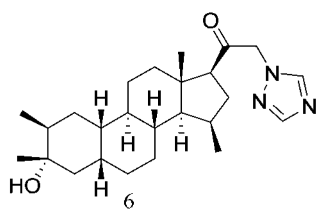

6 ,

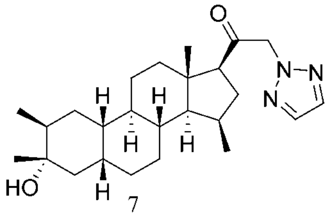

7 ,

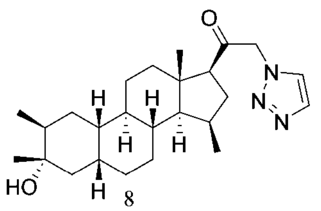

8 ,

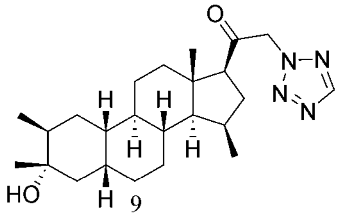

9 ,

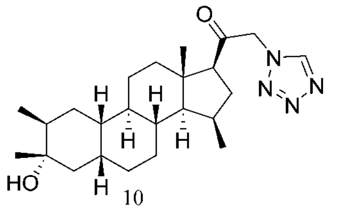
10,

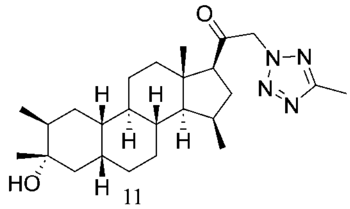
11,

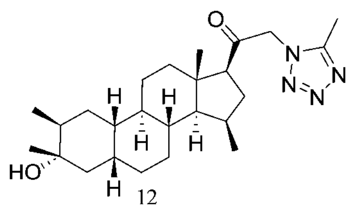
12,

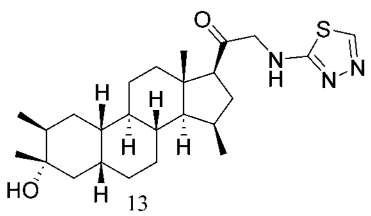
13,

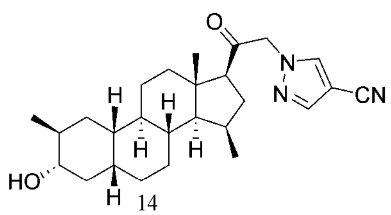
14,

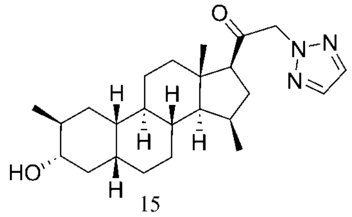
15,

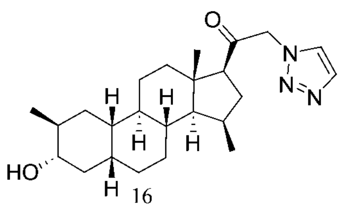
16,

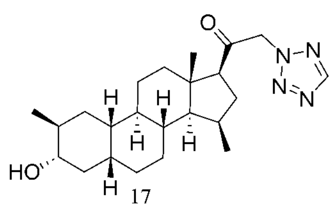
17,

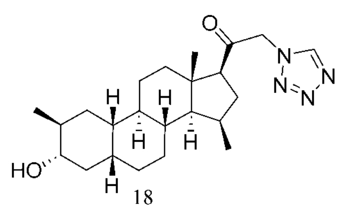
18,

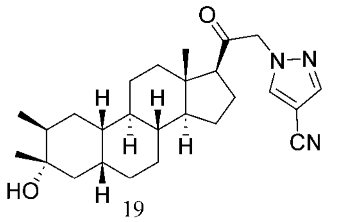
19,

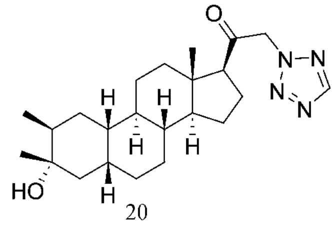
20,

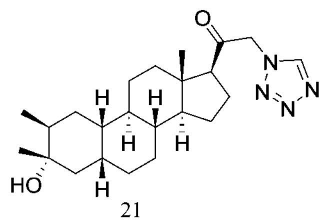
21,

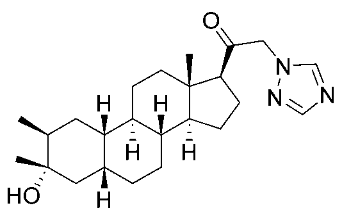
22,

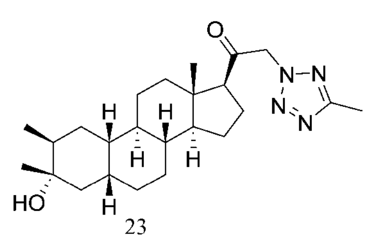
23,

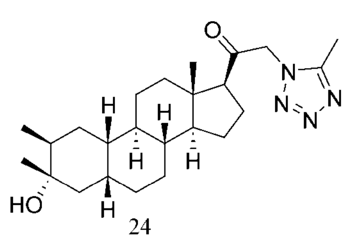
24,

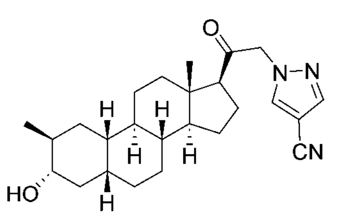
25,

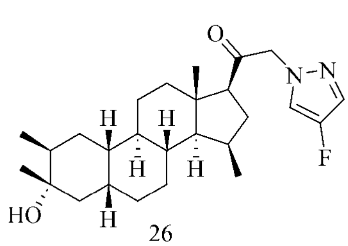
26,

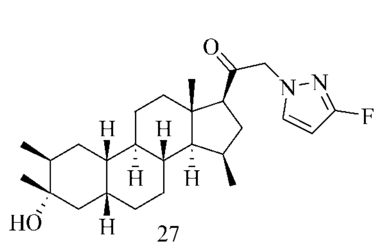
27,

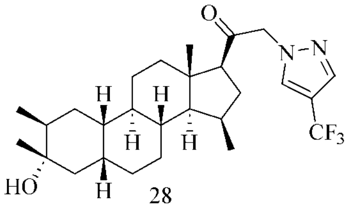
28
,
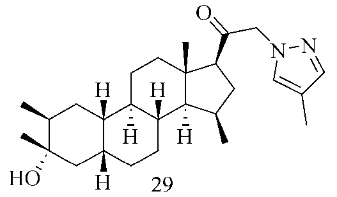
29
,
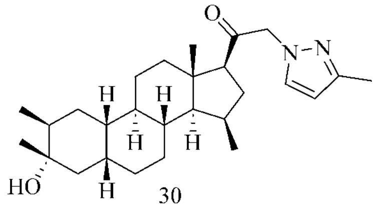
30
,
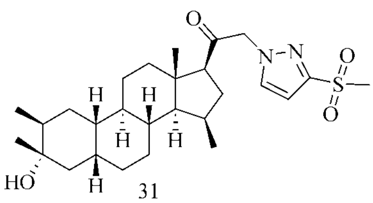
31
,
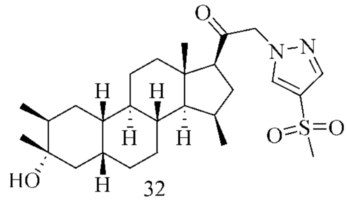
32
,
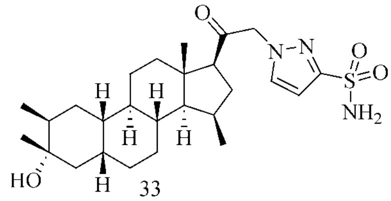
33
,
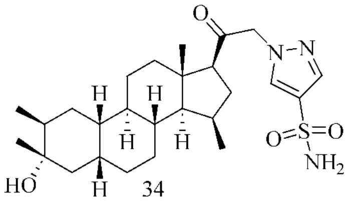
34
,
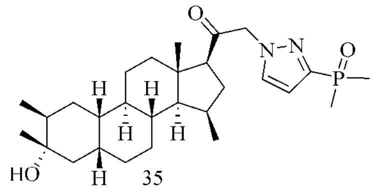
35
,
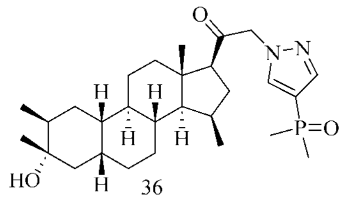
36
,
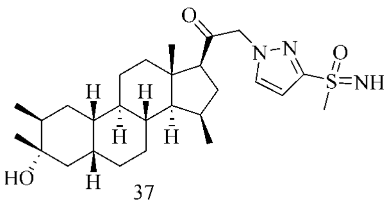
37
,
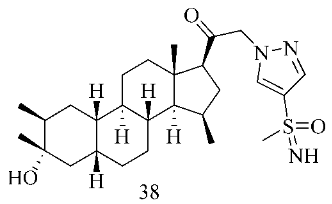
38
,
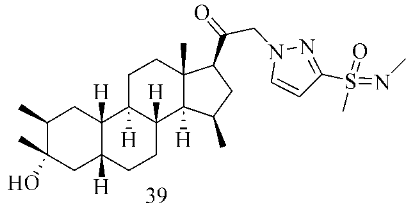
39
,
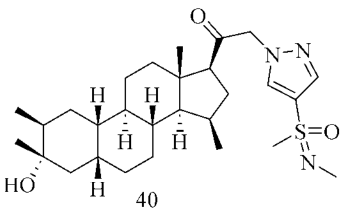
40
,
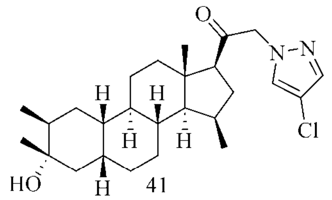
41
,
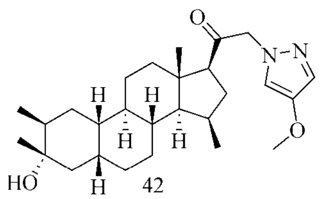
42
,
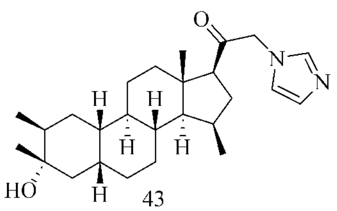
43
,
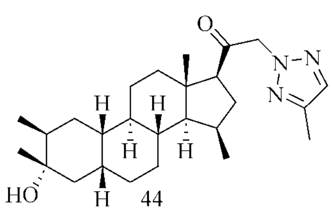
44
,
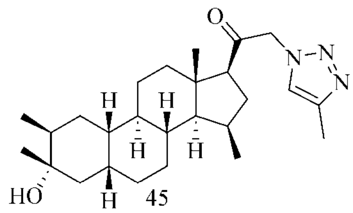
45
,
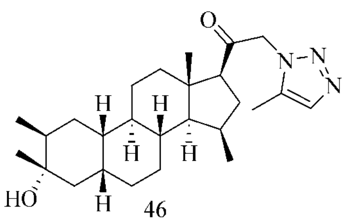
46
,
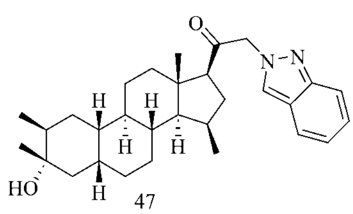
47
,
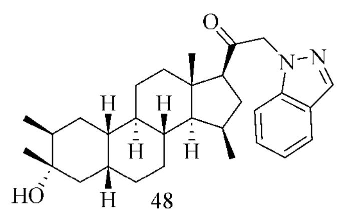
48
,

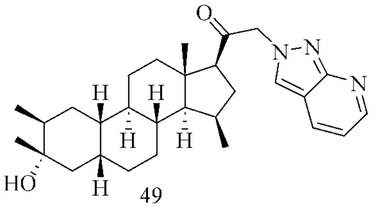
49
,
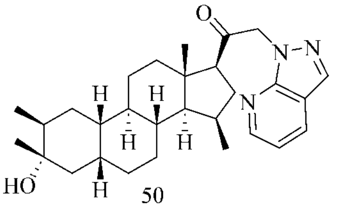
50
,
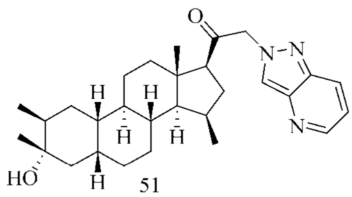
51
,

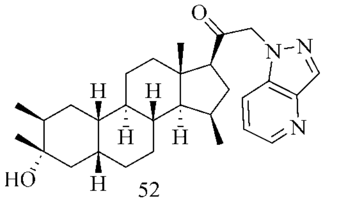
52
,
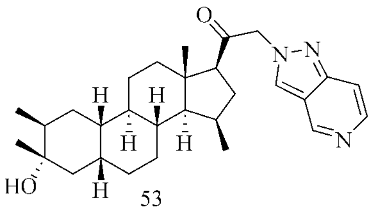
53
,
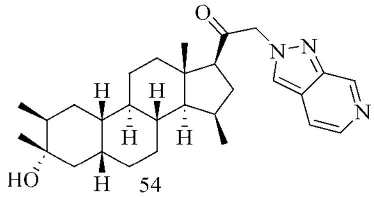
54
,

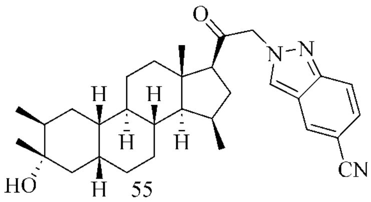
55
,
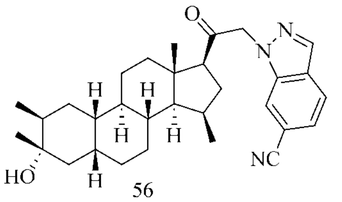
56
,
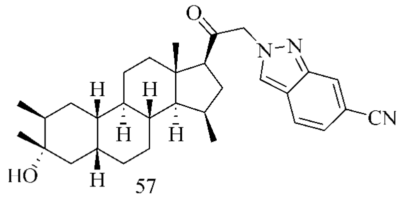
57
,

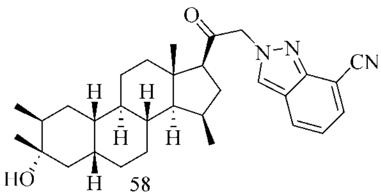
58
,
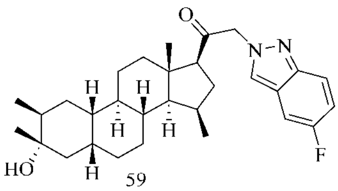
59
,
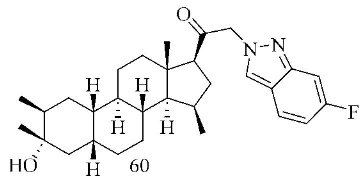
60
,

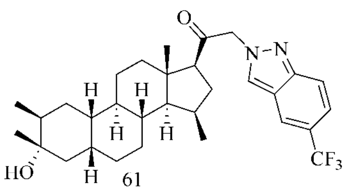
61
,
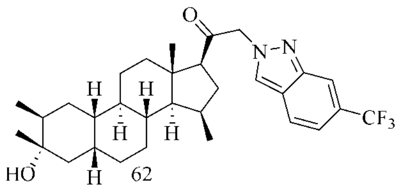
62
,
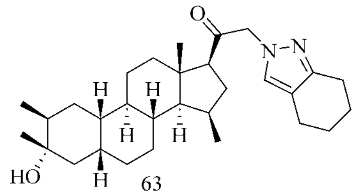
63
,

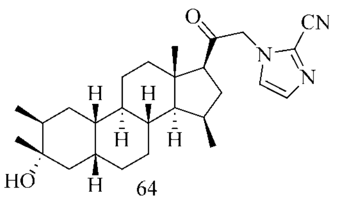
64
,
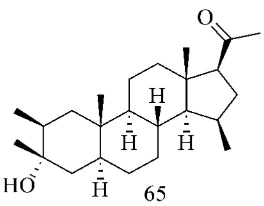
65
,
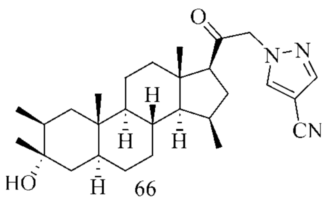
66
,

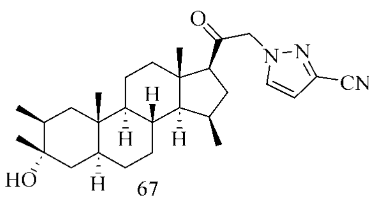
67
, and
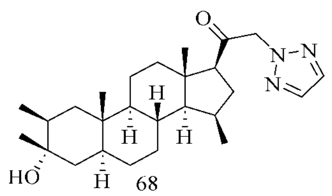
68
.

The present disclosure further provides a pharmaceutical composition, which comprises a therapeutically

effective amount of the compound represented by any one of the general formulas described above, the stereoisomer thereof, a tautomer thereof, or the pharmaceutically acceptable salt thereof, and at least one pharmaceutical adjuvant among pharmaceutically acceptable carriers, diluents or excipients.

In some embodiments of the present disclosure, the pharmaceutical composition may be administered in any of the following ways: oral administration, spray inhalation, rectal administration, nasal administration, buccal administration, topical administration, parenteral administration such as subcutaneous, intravenous, intramuscular, intraperitoneal, intrathecal, intraventricular, intrasternal, or intracranial injection or infusion, or administration by means of an implantable reservoir. Among them, oral administration is the preferred way.

When administered orally, the compound of the present application may be prepared into any orally acceptable formulation form, including, but not limited to, tablets, capsules, aqueous solutions, or aqueous suspensions. For tablets, carriers generally include lactose and corn starch, and additionally, a lubricant such as magnesium stearate may be added. Diluents used in capsule formulations generally include lactose and dried corn starch. Aqueous suspension formulations are generally used by mixing the active ingredient with suitable emulsifying and suspending agents. If needed, a certain amount of sweetening, flavoring, or coloring agents may be added to the above oral formulation forms.

The present disclosure further provides use of the compound represented by any one of the general formulas described above, the stereoisomer thereof, the tautomer thereof, or the pharmaceutically acceptable salt thereof, or the pharmaceutical composition in the preparation of a medicament.

The present disclosure further provides use of the compound represented by any one of the general formulas described above, the stereoisomer thereof, the tautomer thereof, or the pharmaceutically acceptable salt thereof, or the pharmaceutical composition in the preparation of a GABAa receptor modulator medicament.

The present disclosure further provides use of the compound represented by any one of the general formulas described above, the stereoisomer thereof, the tautomer thereof, or the pharmaceutically acceptable salt thereof, or the pharmaceutical composition in the preparation of a medicament for treating a CNS-related disease.

The present disclosure further provides use of the compound represented by any one of the general formulas described above, the stereoisomer thereof, the tautomer thereof, or the pharmaceutically acceptable salt thereof, or the pharmaceutical composition in the treatment of a CNS-related disease.

In some embodiments of the present disclosure, the CNS-related disease is selected from: sleep disorder, mood disorder, premenstrual dysphoric disorder, schizophrenia spectrum disorder, convulsive disorder, memory disorder and/or cognitive disorder, movement disorder, personality disorder, autism spectrum disorder, depression, postpartum depression, major depressive disorder, perimenopausal depression, anxiety disorder, premenstrual anxiety disorder, epilepsy, pain, traumatic brain injury, vascular disease, substance use disorder and/or withdrawal syndrome, and tinnitus.

In some embodiments of the present disclosure, the CNS-related disease is depression.

In some embodiments of the present disclosure, the CNS-related disease is postpartum depression.

In some embodiments of the present disclosure, the CNS-related disease is major depressive disorder.

In some embodiments of the present disclosure, the CNS-related disease is perimenopausal depression.

In some embodiments of the present disclosure, the CNS-related disease is anxiety disorder.

In some embodiments of the present disclosure, the CNS-related disease is premenstrual dysphoric disorder.

In some embodiments of the present disclosure, the CNS-related disease is epilepsy.

Detailed Description of the Present Disclosure

Unless otherwise stated, the terms used in the specification and claims have the following meanings.

The compound of the present disclosure may exist in the form of a specific geometric isomer or stereoisomer. All such compounds are contemplated herein, including cis and trans isomers, (-)- and (+)-enantiomers, (R)- and (S)-enantiomers, diastereomers, (D)-isomers, (L)-isomers, and racemic mixtures and other mixtures thereof, such as enantiomerically or diastereomerically enriched mixtures, all of which fall within the scope of the present disclosure. Additional asymmetric carbon atoms may be present in substituents such as alkyl. All these isomers and mixtures thereof are encompassed within the scope of the present disclosure. In certain embodiments, preferred compounds are those isomer compounds that exhibit superior biological activity. Purified or partially purified isomers and stereoisomers, or racemic or diastereomeric mixtures of the compounds of the present disclosure are also encompassed within the scope of the present disclosure. Purification and separation of such substances can be accomplished by standard techniques known in the art.

The term "alkyl" refers to a linear or branched saturated aliphatic hydrocarbon group consisting of carbon atoms and hydrogen atoms, which is attached to the rest of the molecule via a single bond. The "alkyl" may have 1-8 carbon atoms, i.e., "$C_1$-$C_8$ alkyl", e.g., $C_{1-4}$ alkyl, $C_{1-3}$ alkyl, $C_{1-2}$ alkyl, $C_3$ alkyl, $C_4$ alkyl, $C_{1-6}$ alkyl, or $C_{3-6}$ alkyl. Non-limiting examples of the alkyl include, but are not limited to, methyl, ethyl, propyl, butyl, pentyl, hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, etc., or isomers thereof. The alkyl may be optionally substituted or unsubstituted. When it is substituted, the substituent may attach at any available attachment site, where the substituent may be one or more groups such as alkyl, alkenyl, alkynyl, alkoxy, alkylthio, halogen, sulfhydryl,

hydroxyl, nitryl, amino, cyano, carboxyl, oxo, cycloalkyl, heterocyclyl, aryl, heteroaryl, etc.

The term "alkylene" refers to alkyl in which one hydrogen atom is further replaced. For example, the term "$C_{1-8}$ alkylene" refers to alkylene having 1-8 carbon atoms, e.g., methylene, ethylene, propylene, butylene, pentylene, hexylene, 1-methylethylene, 2-methylethylene, methylpropylene, ethylpropylene, etc. The alkylene may be optionally substituted or unsubstituted. When it is substituted, the substituent may attach at any available attachment site, where the substituent may be one or more groups such as alkyl, alkenyl, alkynyl, alkoxy, alkylthio, halogen, sulfhydryl, hydroxyl, nitryl, amino, cyano, carboxyl, oxo, cycloalkyl, heterocyclyl, aryl, heteroaryl, etc. The "methylene" refers to $-CH_2-$, the "ethylene" refers to - $(CH_2)_2-$, the "propylene" refers to $-(CH_2)_3-$, the "butylene" refers to $-(CH_2)_4-$, and so on.

The term "cycloalkyl" refers to a saturated or partially unsaturated monocyclic or polycyclic hydrocarbon substituent. The cycloalkyl contains 3 to 20 carbon atoms, i.e., "$C_3$-$C_{20}$ cycloalkyl", e.g., $C_{3-18}$ cycloalkyl, $C_{3-16}$ cycloalkyl, $C_{3-12}$ cycloalkyl, $C_{3-8}$ cycloalkyl, $C_{3-6}$ cycloalkyl, $C_{3-5}$ cycloalkyl, $C_{3-4}$ cycloalkyl, $C_{4-8}$ cycloalkyl, $C_{4-6}$ cycloalkyl, or $C_{5-6}$ cycloalkyl, preferably $C_{3-8}$ cycloalkyl, $C_{3-6}$ cycloalkyl, $C_{3-5}$ cycloalkyl, or $C_{3-4}$ cycloalkyl. Non-limiting examples of the monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc. The polycyclic cycloalkyl includes spiro cycloalkyl, fused cycloalkyl, and bridged cycloalkyl. The cycloalkyl may be optionally substituted or unsubstituted. When it is substituted, the substituent may be one or more groups such as alkyl, alkenyl, alkynyl, alkoxy, alkylthio, alkylamino, halogen, sulfhydryl, hydroxyl, nitryl, amino, cycloalkyl, heterocycloalkyl, aryl, heteroaryl, cycloalkoxy, heterocycloalkoxy, cycloalkylthio, heterocycloalkylthio, oxo, carboxyl, carboxylate group, etc.

The term "heterocyclyl" refers to a saturated or unsaturated monocyclic or polycyclic hydrocarbon substituent containing 3 to 20 ring atoms, wherein one or more of the ring atoms are heteroatoms selected from nitrogen, oxygen, and $S(O)_m$ (wherein m is an integer from 0 to 2), but excluding a ring moiety of -O-O-, -O-S-, or -S-S-, and the remaining ring atoms are carbon atoms. The term includes 3- to 20-membered heterocyclyl, e.g., 3- to 18-membered heterocyclyl, 3- to 16-membered heterocyclyl, 3- to 12-membered heterocyclyl, 3- to 8-membered heterocyclyl, 3- to 6-membered heterocyclyl, 3- to 5-membered heterocyclyl, 3- to 4-membered heterocyclyl, 4- to 8-membered heterocyclyl, 4- to 6-membered heterocyclyl, or 5- to 6-membered heterocyclyl, preferably 3- to 8-membered heterocyclyl, 3- to 6-membered heterocyclyl, 3- to 5-membered heterocyclyl, 3- to 4-membered heterocyclyl, 4- to 8-membered heterocyclyl, 4- to 6-membered heterocyclyl, or 5- to 6-membered heterocyclyl, optionally containing 1-4 heteroatoms, 1-3 heteroatoms, or 1-2 heteroatoms, wherein the heteroatom is optionally an N, O, or S atom, but excluding a ring moiety of - O-O-, -O-S-, or -S-S-. 3- to 8-membered heterocyclyl containing 1-4 heteroatoms selected from N, O, and S is particularly preferred. Non-limiting examples of the monocyclic heterocyclyl include oxetanyl, thietanyl, pyrrolidinyl, imidazolidinyl, tetrahydrofuranyl, tetrahydrothienyl, tetrahydropyranyl, dihydroimidazolyl, dihydrofuranyl, dihydropyrazolyl, piperidinyl, piperazinyl, morpholinyl, 1,3-dioxolanyl, 2,2-difluoro-1,3-dioxolanyl, azepinyl, etc. Non-limiting examples of polycyclic heterocyclyl include spiro heterocyclyl, fused heterocyclyl, and bridged heterocyclyl, wherein the spiro heterocyclyl, fused heterocyclyl, or bridged heterocyclyl involved is optionally attached to other groups via single bonds, or further ortho-fused to other cycloalkyl, heterocyclyl, aryl, and heteroaryl via any two or more atoms on the ring. The heterocyclyl may be optionally substituted or unsubstituted. When it is substituted, the substituent may attach at any available attachment site, where the substituent may be one or more groups such as alkyl, alkenyl, alkynyl, alkoxy, alkylthio, halogen, sulfhydryl, hydroxyl, nitryl, amino, cyano, carboxyl, oxo, cycloalkyl, heterocyclyl, aryl, heteroaryl, etc.

The term "aryl" refers to a 6- to 14-membered, preferably 6- to 10-membered, all-carbon monocyclic or fused polycyclic (i.e., rings that share a pair of adjacent carbon atoms) group having a conjugated electron system, such as phenyl and naphthyl, more preferably, phenyl. The aryl may be optionally substituted or unsubstituted. When it is substituted, the substituent may attach at any available attachment site, where the substituent may be one or more groups such as alkyl, alkenyl, alkynyl, alkoxy, alkylthio, halogen, sulfhydryl, hydroxyl, nitryl, amino, cyano, carboxyl, oxo, cycloalkyl, heterocyclyl, aryl, heteroaryl, etc. When the aryl is substituted with a substituent, the substituent is not further substituted.

The term "heteroaryl" refers to a monocyclic or fused polycyclic heteroaromatic system containing 1 to 4 heteroatoms and 5 to 14 ring atoms, wherein the heteroatom is selected from oxygen, sulfur, nitrogen, etc. The heteroaryl is preferably 5- to 14-membered heteroaryl, more preferably 5- to 10-membered heteroaryl, e.g., 5- to 6-membered heteroaryl, 5- to 6-membered heteroaryl-fused 5- to 6-membered heteroaryl, 5- to 6-membered heteroaryl-fused 5- to 6-membered cycloalkyl, 5- to 6-membered heteroaryl-fused $C_{6-10}$ aryl, $C_{6-10}$ aryl-fused 5- to 6-membered heteroaryl, or the like, further preferably 5- to 6-membered heteroaryl, 5- to 6-membered heteroaryl-fused 5- to 6-membered heteroaryl, 5- to 6-membered heteroaryl-fused 5- to 6-membered cycloalkyl, 5- to 6-membered heteroaryl-fused phenyl, or phenyl-fused 5- to 6-membered heteroaryl, and particularly preferably 5- to 10-membered heteroaryl containing 1-4 heteroatoms selected from N, O, and S, such as pyrrolyl, imidazolyl, furanyl, pyranyl, thienyl, thiazolyl, thiadiazolyl, pyrazolyl, oxazolyl, oxadiazolyl, triazolyl, tetrazolyl, pyridyl, pyrimidinyl, pyridazinyl, pyrazinyl, thienophenyl, thienopyridinyl, pyrazolophenyl, pyrazolopyridinyl, pyrazolocyclohexyl, pyridothienyl, pyridopyrrolyl, benzothienyl, indolyl, and indazolyl. The heteroaryl may be optionally substituted or unsubstituted. When it is substituted, the substituent may attach at any available attachment site, where the substituent may be one or more groups such as alkyl, alkenyl, alkynyl, alkoxy, alkylthio,

halogen, sulfhydryl, hydroxyl, nitryl, amino, cyano, carboxyl, oxo, cycloalkyl, heterocyclyl, aryl, heteroaryl, etc. When the heteroaryl is substituted with a substituent, the substituent is not further substituted.

The "hetero" in the heteroaryl or heterocyclyl means that the group contains one or more ring atoms independently selected from heteroatoms such as N, O, and $S(O)_m$ (wherein m is an integer from 0 to 2), preferably 1-4 ring atoms selected from N, O, and S, and preferably 1-3 ring atoms selected from N, O, and S.

The term "alkoxy" refers to -O-(alkyl) or -O-(unsubstituted cycloalkyl), wherein the alkyl and cycloalkyl are as defined above. Non-limiting examples of the alkoxy include: methoxy, ethoxy, propoxy, butoxy, cyclopropyloxy, cyclobutyloxy, cyclopentyloxy, cyclohexyloxy, etc. The alkoxy may be optionally substituted or unsubstituted. When it is substituted, the substituent may attach at any available attachment site, where the substituent may be one or more groups such as alkyl, alkenyl, alkynyl, alkoxy, alkylthio, halogen, sulfhydryl, hydroxyl, nitryl, amino, cyano, carboxyl, oxo, cycloalkyl, heterocyclyl, aryl, heteroaryl, etc. When the alkoxy is substituted with a substituent, the substituent is not further substituted.

The term "alkylthio" refers to -S-(alkyl) or -S-(unsubstituted cycloalkyl), wherein the alkyl and cycloalkyl are as defined above. Non-limiting examples of the alkylthio include: methylthio, ethylthio, propylthio, butylthio, cyclopropylthio, cyclobutylthio, cyclopentylthio, cyclohexylthio, etc. The alkylthio may be optionally substituted or unsubstituted. When it is substituted, the substituent may attach at any available attachment site, where the substituent may be one or more groups such as alkyl, alkenyl, alkynyl, alkoxy, alkylthio, halogen, sulfhydryl, hydroxyl, nitryl, amino, cyano, carboxyl, oxo, cycloalkyl, heterocyclyl, aryl, heteroaryl, etc. When the alkylthio is substituted with a substituent, the substituent is not further substituted.

The term "alkylamino" refers to -NH-(alkyl or unsubstituted cycloalkyl) or -N-(alkyl or unsubstituted cycloalkyl) (alkyl or unsubstituted cycloalkyl), wherein the alkyl and cycloalkyl are as defined above. Non-limiting examples of the alkylamino include: methylamino, ethylamino, propylamino, butylamino, cyclopropylamino, cyclobutylamino, cyclopentylamino, cyclohexylamino, etc. The alkylamino may be optionally substituted or unsubstituted. When it is substituted, the substituent may attach at any available attachment site, where the substituent may be one or more groups such as alkyl, alkenyl, alkynyl, alkoxy, alkylthio, halogen, sulfhydryl, hydroxyl, nitryl, amino, cyano, carboxyl, oxo, cycloalkyl, heterocyclyl, aryl, heteroaryl, etc. When the alkylamino is substituted with a substituent, the substituent is not further substituted.

The term "halo", "halogen", or "halogenated" should be understood to refer to a fluorine (F), chlorine (Cl), bromine (Br) or iodine (I) atom, preferably a fluorine, chlorine or bromine atom.

The term "haloalkyl" refers to alkyl substituted with one or more halogens, wherein the alkyl is as defined above. Non-limiting examples of halomethyl include: fluoromethyl, chloromethyl, bromomethyl, iodomethyl, difluoromethyl, chlorofluoromethyl, dichloromethyl, bromofluoromethyl, trifluoromethyl, chlorodifluoromethyl, dichlorofluoromethyl, trichloromethyl, bromodifluoromethyl, bromochlorofluoromethyl, dibromofluoromethyl, etc., preferably fluoromethyl, difluoromethyl, and trifluoromethyl. Non-limiting examples of haloethyl include: 2-fluoroethyl, 2-chloroethyl, 2-bromoethyl, 2,2-difluoroethyl, 2-chloro-2-fluoroethyl, 2,2-dichloroethyl, 2-bromo-2-fluoroethyl, 2,2,2-trifluoroethyl, 2-chloro-2,2-difluoroethyl, 2,2-dichloro-2-fluoroethyl, 2,2,2-trichloroethyl, 2-bromo-2,2-difluoroethyl, 2-bromo-2-chloro-2-fluoroethyl, 2-bromo-2,2-dichloroethyl, 1,1,2,2-tetrafluoroethyl, pentafluoroethyl, 1-chloro-1,2,2,2-tetrafluoroethyl, 2-chloro-1,1,2,2-tetrafluoroethyl, 1,2-dichloro-1,2,2-trifluoroethyl, 2-bromo-1,1,2,2-tetrafluoroethyl, etc., preferably 2-fluoroethyl, 2-chloroethyl, 2-bromoethyl, and 2,2-difluoroethyl.

The term "haloalkoxy" refers to alkoxy substituted with one or more halogens, wherein the alkoxy is as defined above. Non-limiting examples of halomethoxy include: fluoromethoxy, chloromethoxy, bromomethoxy, iodomethoxy, difluoromethoxy, chlorofluoromethoxy, dichloromethoxy, bromofluoromethoxy, trifluoromethoxy, chlorodifluoromethoxy, dichlorofluoromethoxy, trichloromethoxy, bromodifluoromethoxy, bromochlorofluoromethoxy, dibromofluoromethoxy, etc., preferably fluoromethoxy, difluoromethoxy, and trifluoromethoxy. Non-limiting examples of haloethoxy include: 2-fluoroethoxy, 2-chloroethoxy, 2-bromoethoxy, 2,2-difluoroethoxy, 2-chloro-2-fluoroethoxy, 2,2-dichloroethoxy, 2-bromo-2-fluoroethoxy, 2,2,2-trifluoroethoxy, 2-chloro-2,2-difluoroethoxy, 2,2-dichloro-2-fluoroethoxy, 2,2,2-trichloroethoxy, 2-bromo-2,2-difluoroethoxy, 2-bromo-2-chloro-2-fluoroethoxy, 2-bromo-2,2-dichloroethoxy, 1,1,2,2-tetrafluoroethoxy, pentafluoroethoxy, 1-chloro-1,2,2,2-tetrafluoroethoxy, 2-chloro-1,1,2,2-tetrafluoroethoxy, 1,2-dichloro-1,2,2-trifluoroethoxy, 2-bromo-1,1,2,2-tetrafluoroethoxy, etc., preferably 2-fluoroethoxy, 2-chloroethoxy, 2-bromoethoxy, and 2,2-difluoroethoxy.

The term "alkenyl" refers to alkyl as defined above consisting of at least two carbon atoms and at least one carbon-carbon double bond. The "alkenyl" may have 2-6 carbon atoms, such as vinyl, 1-propenyl, 2-propenyl, 1-, 2- or 3-butenyl, etc. The alkenyl may be optionally substituted or unsubstituted. When it is substituted, the substituent may be one or more groups such as alkyl, alkenyl, alkynyl, alkoxy, alkylthio, alkylamino, halogen, sulfhydryl, hydroxyl, nitryl, amino, cycloalkyl, heterocycloalkyl, aryl, heteroaryl, cycloalkoxy, heterocycloalkoxy, cycloalkylthio, heterocycloalkylthio, etc.

The term "alkynyl" refers to alkyl as defined above consisting of at least two carbon atoms and at least one carbon-carbon triple bond. The "alkynyl" may have 2-6 carbon atoms, wherein alkynyl may be further substituted with other related groups such as: alkyl, alkenyl, alkynyl, alkoxy, alkylthio, alkylamino, halogen, sulfhydryl, hydroxyl, nitryl, amino, cycloalkyl, heterocycloalkyl, aryl, heteroaryl, cycloalkoxy, heterocycloalkoxy, cycloalkylthio, heterocycloalkylthio, carboxyl, carboxylate group, etc.

"Hydroxyl" refers to -OH.

"Amino" refers to $-NH_2$.

"Cyano" refers to -CN.

"Nitryl" refers to $-NO_2$.

"Carboxyl" refers to -C(O)OH.

"Oxo" refers to =O.

The terms "comprise", "include", "have", "contain", or "involve" and other variations thereof herein are inclusive or open-ended and do not exclude additional unrecited elements or method steps. It should be understood by those skilled in the art that the terms described above such as "comprise" encompass the meaning of "consist of".

The term "one or more" or the similar expression "at least one" may indicate, for example, 1, 2, 3, 4, 5, 6, 7, 8, 9, 10, or more.

When the lower and upper limits of a range of numerical values are disclosed, any numerical value falling within the range and any included range are specifically disclosed. In particular, each range of values disclosed herein is to be understood as indicating each numerical value and range encompassed within a relatively broad range.

As used herein, "Z" and "-Z-" both refer to the same particular group, which can be used interchangeably.

The expression "m-n" as used herein refers to the range of m to n as well as to sub-ranges consisting of point values therein and the point values. For example, the expression "$C_2$-$C_8$" or "$C_{2-8}$" encompasses a range of 2 to 8 carbon atoms and should be understood as also encompassing any sub-range and each point value therein, e.g., $C_2$-$C_5$, $C_3$-$C_4$, $C_2$-$C_6$, $C_3$-$C_6$, $C_4$-$C_6$, $C_4$-$C_7$, $C_4$-$C_8$, etc., as well as $C_2$, $C_3$, $C_4$, $C_5$, $C_6$, $C_7$, $C_8$, etc. For example, the expression "$C_3$-$C_{10}$" or "$C_{3-10}$" should also be understood in a similar manner, for example, it can encompass any sub-range and point value therein, e.g., $C_3$-$C_9$, $C_6$-$C_9$, $C_6$-$C_8$, $C_6$-$C_7$, $C_7$-$C_{10}$, $C_7$-$C_9$, $C_7$-$C_8$, $C_8$-$C_9$, etc., as well as $C_3$, $C_4$, $C_5$, $C_6$, $C_7$, $C_8$, $C_9$, $C_{10}$, etc. For another example, the expression "$C_1$-$C_6$" or "$C_{1-6}$" encompasses a range of 1 to 6 carbon atoms and should be understood as also encompassing any sub-range and each point value therein, e.g., $C_2$-$C_5$, $C_3$-$C_4$, $C_1$-$C_2$, $C_1$-$C_3$, $C_1$-$C_4$, $C_1$-$C_5$, $C_1$-$C_6$, etc., as well as $C_1$, $C_2$, $C_3$, $C_4$, $C_5$, $C_6$, etc. For another example, the expression "three- to ten-membered" should be understood as encompassing any sub-range and each point value therein, e.g., three- to five-membered, three- to six-membered, three- to seven-membered, three- to eight-membered, four- to five-membered, four- to six-membered, four- to seven-membered, four- to eight-membered, five- to seven-membered, five- to eight-membered, six- to seven-membered, six- to eight-membered, nine- to ten-membered, etc., as well as three-membered, four-membered, five-membered, six-membered, seven-membered, eight-membered, nine-membered, ten-membered, etc. Other similar expressions herein should also be understood in a similar manner.

The expressions "X is selected from A, B, or C", "X is selected from A, B, and C", "X is A, B, or C", "X is A, B, and C", and the like as used herein all carry the same meaning, indicating that X may be any one or more of A, B, and C.

The expression "$-(CY_1Y_2)_n-$" as used herein means that all $Y_1$ or $Y_2$ attached to C may be identical or different, i.e., all $Y_1$ may be different groups, all $Y_2$ may be different groups, all $Y_1$ may also be identical groups, and all $Y_2$ may also be identical groups.

The term "optional" or "optionally" refers to that the subsequently described event or circumstance may occur or may not occur. The description includes instances where the event or circumstance occurs and instances where the event or circumstance does not occur. For example, "cycloalkyl optionally substituted with alkyl" means that alkyl may, but does not necessarily, exist. The description includes instances where the cycloalkyl is substituted with alkyl and instances where the cycloalkyl is not substituted with alkyl.

The terms "substitution" and "substituted" mean that one or more (e.g., one, two, three, or four) hydrogens on a specified atom are replaced by a choice from the designated groups, with the proviso that the normal valency of the specified atom in the present case is not exceeded and that the replacement results in a stable compound. A combination of substituents and/or variables is permissible only if the combination results in a stable compound. When it is stated that a certain substituent is absent, it should be understood that the substituent may be one or more hydrogen atoms, provided that the structure enables the compound to reach a stable state. When it is stated that each carbon atom in a group may optionally be replaced by a heteroatom, the proviso is that the normal valency of all atoms in the group in the present case is not exceeded, and that a stable compound is formed.

If a substituent is described as "optionally substituted", the substituent may be unsubstituted or may be substituted. If an atom or group is described as optionally substituted with one or more substituents in the list of substituents, one or more hydrogens on the atom or group can be replaced by an independently selected and optional substituent. When the substituent is oxo (namely =O), it means that two hydrogen atoms are replaced. Unless otherwise specified, as used herein, the attachment site of a substituent may be from any suitable position of the substituted group.

When a bond of a substituent is shown as passing through a bond connecting two atoms in a ring, the substituent may be bonded to any one of the ring-forming atoms in the substitutable ring.

When any variable (e.g., R), as well as labeled variables (e.g., $R_1$, $R_2$, $R_3$, $R_4$, $R_5$, $R_6$, $R_7$, etc.), occurs more than once in a composition or structure of a compound, the variable is independently defined in each case at each occurrence.

For example, if a group is substituted with 0, 1, 2, 3, or 4 R substituents, the group may optionally be substituted with up to four R substituents, and the options for each R substituent in each case are independent of each other.

The term "pharmaceutically acceptable" substance refers to those substances which are, within the scope of sound medical judgment, suitable for use in contact with the tissues of patients without undue toxicity, irritation, allergic response, and other problems, commensurate with a reasonable benefit/risk ratio, and effective for their intended use.

The term "pharmaceutically acceptable salt" refers to salts of the compounds of the present disclosure, which are safe and effective and possess the desired biological activities when used in mammals.

The term "pharmaceutical composition" refers to a composition containing one or more of the compounds described herein or physiologically/pharmaceutically acceptable salts thereof, and other components such as physiologically/pharmaceutically acceptable carriers or excipients. The pharmaceutical composition is intended to promote the administration to an organism, so as to facilitate the absorption of the active ingredient, thereby exerting its biological activity.

The term "pharmaceutically acceptable carrier" refers to those substances that do not have a significant irritating effect on organisms and do not impair the biological activity and properties of the active compound. The term "pharmaceutically acceptable carrier" includes, but is not limited to, a glidant, a sweetener, a diluent, a preservative, a dye/colorant, a flavoring agent, a surfactant, a wetting agent, a dispersant, a disintegrant, a stabilizer, a solvent, or an emulsifier.

The terms "administration" or "administering" and the like refer to methods that enable a compound or composition to be delivered to a desired site of biological action. These methods include, but are not limited to, oral administration or parenteral administration (including intraventricular, intravenous, subcutaneous, intraperitoneal, intramuscular, and intravascular injection or infusion), topical administration, rectal administration, etc., especially injection or oral administration.

As used herein, the term "treat", "treating", or "treatment" includes alleviating, relieving, or ameliorating a disease or symptom, preventing other symptoms, ameliorating or preventing underlying metabolic factors of a symptom, inhibiting a disease or symptom, such as arresting the development of a disease or symptom, relieving a disease or symptom, promoting remission of a disease or symptom, or halting signs of a disease or symptom, and extends to include prevention. The term "treat", "treating", or "treatment" also includes achieving a therapeutic benefit and/or a prophylactic benefit. The therapeutic benefit refers to eradication or amelioration of a condition being treated. In addition, the therapeutic benefit is achieved by eradicating or ameliorating one or more physiological signs associated with an underlying disease, such that amelioration of the underlying disease in the patient can be observed, although the patient may still be afflicted with the underlying disease. The prophylactic benefit refers to the use of a composition by a patient to prevent the risk of a certain disease, or the administration of a composition to a patient when the patient develops one or more physiological conditions of a disease, although the disease has not yet been diagnosed.

The term "active ingredient", "therapeutic agent", "active substance", or "active agent" refers to a chemical entity that is effective in treating or preventing a target disorder, disease, or condition. The term "neuropsychiatric disease" refers to a generic term for neurological diseases and psychiatric diseases, including neurological diseases and/or psychiatric diseases.

For a drug, drug unit or active ingredient, the term "effective amount", "therapeutically effective amount", or "prophylactically effective amount" refers to an amount of a drug or pharmaceutical agent that is sufficient to provide the desired effect with acceptable side effects. The determination of the effective amount varies from person to person. It depends on the age and general condition of an individual, as well as the specific active substance used. The appropriate effective amount in a case may be determined by those skilled in the art in the light of routine tests.

As used herein, the term "individual" includes a human or non-human animal. An exemplary human individual includes a human individual (referred to as a patient) with a disease (e.g., a disease described herein) or a normal individual. In the present disclosure, the "non-human animal" includes all vertebrates, such as non-mammals (e.g., birds, amphibians, and reptiles) and mammals, such as non-human primates, livestock and/or domesticated animals (e.g., sheep, dogs, cats, cows, pigs, etc.).

The following detailed description of the present disclosure is intended to illustrate non-limiting embodiments and to enable others skilled in the art to more fully understand the technical solutions of the present disclosure, their principles, and their practical applications, so that others skilled in the art may modify and implement the present disclosure in various forms to allow it to be optimally adapted to the requirements of particular use.

Beneficial Effects

The compounds of the present disclosure exhibit good binding ability to the GABAa receptor, have activity against neuropsychiatric diseases, and also possess relatively good druggability properties, relatively low metabolic rates, and suitable lipophilicity. These characteristics ensure their ability to cross the blood-brain barrier and provide therapeutic or prophylactic effects against neuropsychiatric diseases, thereby offering better patient compliance in clinical practice.

In some embodiments, the compounds of the present disclosure have good positive modulatory effects on the

GABAa receptor (e.g., having improved Emax and/or EC50). In some embodiments, the compounds of the present disclosure have improved safety (e.g., having lower toxicity and/or fewer side effects). In some embodiments, the compounds of the present disclosure have an improved safety window (e.g., having larger safe administration dose ranges, or showing smaller probability of side effects occurring at the same dose). In some embodiments, the compounds of the present disclosure have improved pharmacokinetic properties (e.g., having longer half-lives t1/2, greater exposure AUC, and higher maximum plasma concentrations Cmax). In some embodiments, the compounds of the present disclosure have improved pharmacodynamic effects (e.g., having significant antiepileptic effects and/or significant antidepressant effects). In some embodiments, the compounds of the present disclosure have good patient compliance and/or superior druggability properties such as being less likely to develop tolerance.

Specifically, the results of an in vitro GABAa receptor cellular activity assay of the present disclosure show that the compounds of the present disclosure are effective GABAa receptor positive modulators with excellent positive modulatory effects. They have lower Emax (i.e., low efficacy ceiling at high concentrations), indicating potentially fewer in vivo side effects; in combination with lower EC50 (i.e., lower effective dose), they demonstrated potential for a larger therapeutic window. The results of an in vivo efficacy experiment in a PTZ-induced epilepsy mouse model show that the compounds of the present disclosure can prolong the survival rate of mice with PTZ-induced convulsions, exhibiting protective effects against acute epileptic seizures in the mice, and some of the compounds still exhibit partial protective effects at an extremely low dose; additionally, some of the compounds can also significantly prolong the latency period of clonic seizures and generalized tonic-clonic seizures and reduce their number of occurrences, demonstrating significant antiepileptic effects. The results of an in vivo efficacy experiment by forced swimming and sucrose preference tests in mice show that the compound of the present disclosure can significantly reduce the immobility time of C57 mice in the forced swimming test and significantly improve the sucrose preference degree of C57 mice in the sucrose preference test, demonstrating significant antidepressant effects. The results of an in vivo pharmacokinetic experiment in mice show that the compounds of the present disclosure have longer half-lives t1/2, greater exposure AUC, and higher maximum plasma concentrations Cmax, demonstrating good metabolic properties. The results of an acute toxicity experiment and side effect observation in mice show that the compounds of the present disclosure have higher maximum tolerated doses (MTDs), superior safety, and weaker side effects, demonstrating value for further clinical development.

DETAILED DESCRIPTION OF THE PREFERRED EMBODIMENT

Embodiments of the present disclosure will be described in detail below with reference to examples, but those skilled in the art will appreciate that the following examples are only illustrative of the present disclosure and should not be construed as limiting the scope of the present disclosure. Experimental procedures without specified conditions in the examples were conducted according to conventional conditions or conditions recommended by the manufacturers. Reagents or instruments without specified manufacturers used herein are conventional products that are commercially available. The proportions or percentages used herein are calculated by weight, unless otherwise specified.

**Example**

The compound structures of the present disclosure were determined by nuclear magnetic resonance (NMR) and/or liquid chromatography-mass spectrometry (LC-MS).

The NMR chemical shifts ($\delta$) are given in parts per million (ppm). The NMR determination was conducted by using an AVANCE III600 nuclear magnetic resonance apparatus, with deuterated dimethyl sulfoxide (DMSO-$d_6$), deuterated methanol ($CD_3OD$), and deuterated chloroform ($CDCl_3$) as determination solvents, and tetramethylsilane (TMS) as an internal standard.

The LC-MS determination was conducted by using a Japan Shimadzu LCMS2020 mass spectrometer. The HPLC determination was conducted by using a Japan Shimadzu LC20A liquid chromatograph.

Yantai Jiangyou silica gel plates were adopted as thin layer chromatography silica gel plates. The specification adopted in the TLC was 0.2 mm $\pm$ 0.03 mm, and the specification adopted in the thin layer chromatography for product separation and purification was 0.4 mm-0.5 mm.

**Example 1**

**1-((2S,3S,5R,8R,9R,10S,13S,14S,15R,17S)-3-Hydroxy-2,3,13,15-tetramethylhexadecahydro-1H-cyclopenta[a]phenanthren-17-yl)ethan-1-one**

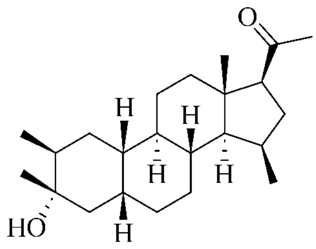

1

Synthesis scheme:

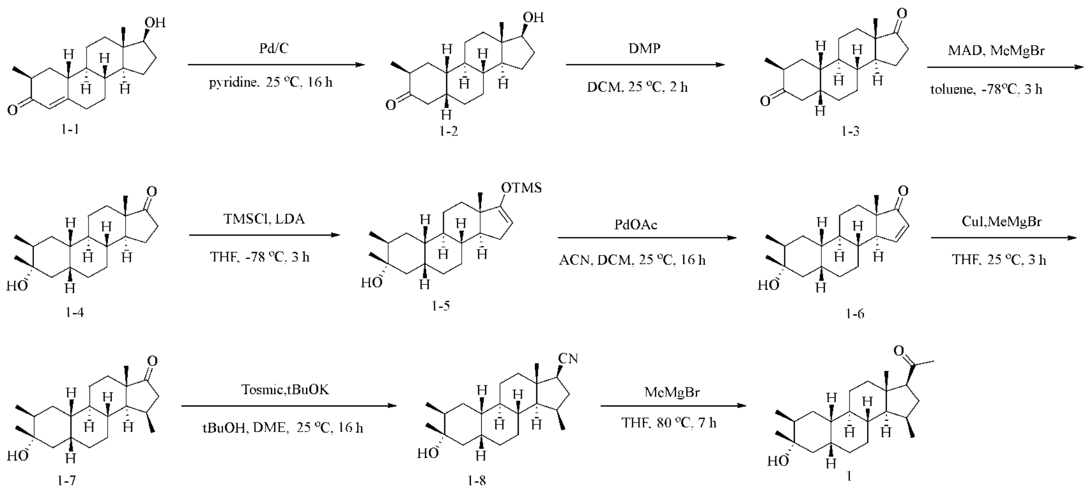


**Step 1: Preparation of (2S,5R,8R,9R,10S,13S,14S,17S)-17-hydroxy-2,13-dimethylhexadecahydro-3H-cyclopenta[a]phenanthren-3-one**

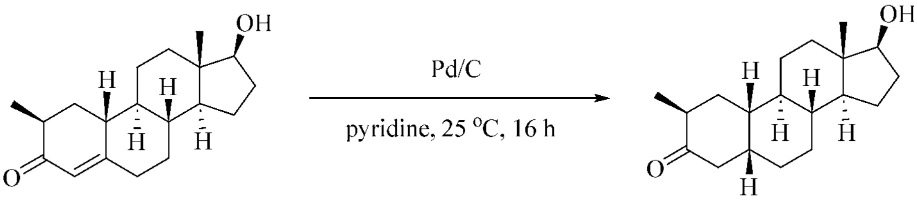


(2S,8R,9S,10R,13S,14S,17S)-17-Hydroxy-2,13-dimethyl-1,2,6,7,8,9,10,11,12,13,14,15,16,17-tetradecahydro-3H-cyclopenta[a]phenanthren-3-one (5.00 g, 17.3 mmol) was dissolved in a solution of pyridine (60.0 mL), and 10% wet palladium on carbon (340 mg) was added. The reaction system was purged with hydrogen and stirred at room temperature for 16 h. The reaction mixture was filtered, and the organic phase was concentrated to give a crude product (6.60 g, yield: 100%, crude product), which was directly used in the next step.

**Step 2: Preparation of (2S,5R,8R,9R,10S,13S,14S)-2,13-dimethyltetradecahydro-3H-cyclopenta[a]phenanthrene-3,17(2H)-dione**

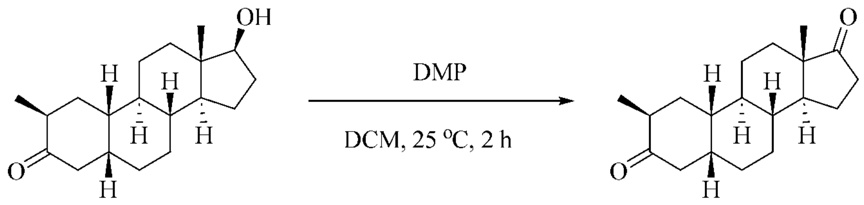


(2S,5R,8R,9R,10S,13S,14S,17S)-17-Hydroxy-2,13-dimethylhexadecahydro-3H-cyclopenta[a]phenanthren-3-one (6.60 g, 22.7 mmol) was dissolved in dichloromethane (87.0 mL), and DMP (14.5 g, 34.1 mmol) was added. The reaction system was stirred at room temperature for 2 h. The reaction mixture was filtered, and the organic phase was concentrated to give a crude product, which was then separated and purified by column chromatography (petroleum ether/ethyl acetate: 50/1-3/1) to give (2S,5R,8R,9R,10S,13S,14S)-2,13-dimethyltetradecahydro-3H-cyclopenta[a]phenanthrene-3,17(2H)-dione (5.50 g, yield: 84.0%).

$^1H$ NMR (400 MHz, $CDCl_3$) δ 2.62 (t, *J* = 13.6Hz, 1H), 2.51 - 2.44 (m, 1H), 2.39 - 2.30 (m, 1H), 2.25-1.16 (m, 19H), 0.99 (d, *J* = 6.4Hz, 3H), 0.91 (s, 3H).

**Step 3: Preparation of (2S,3S,5R,8R,9R,10S,13S,14S)-3-hydroxy-2,3,13-trimethylhexadecahydro-17H-cyclopenta[a]phenanthren-17-one**

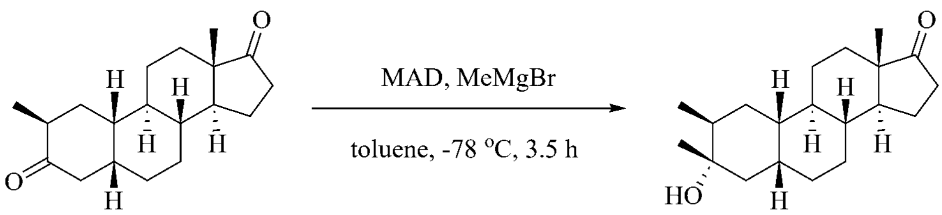


(2S,5R,8R,9R,10S,13S,14S)-2,13-Dimethyltetradecahydro-3H-cyclopenta[a]phenanthrene-3,17(2H)-dione (5.50 g, 18.9 mmol) was dissolved in a solution of dry toluene (200 mL). The system was purged with nitrogen and cooled to -78 °C. A 0.4 M solution of methylaluminum bis(2,6-di-tert-butyl-4-methylphenoxide) (142 mL, 56.8 mmol) was slowly added, and the reaction system was stirred at -78 °C for 30 min. Methylmagnesium bromide (3.0 M, 20 mL, 60.0 mmol) was slowly added dropwise, and the reaction system was stirred at -78 °C for 3 h.After the reaction was completed as detected by TLC, the reaction was quenched with saturated ammonium chloride, and the mixture was extracted with ethyl acetate. The organic phase was washed with water and saturated brine, dried over anhydrous sodium sulfate, filtered, and concentrated to dryness by rotary evaporation, and the crude product was separated by column chromatography (elution with petroleum ether:ethyl acetate = 2:1) to give (2S,3S,5R,8R,9R,10S,13S,14S)-3-hydroxy-2,3,13-trimethylhexadecahydro-17H-cyclopenta[a]phenanthren-17-one (4.90 g, yield: 84.0%).

$^1H$ NMR (400 MHz, $CDCl_3$) δ 2.47 - 2.40 (m, 1H), 2.13 - 2.03 (m, 1H), 1.96 - 1.88 (m, 1H), 1.83 - 1.03 (m, 23H), 0.88 - 0.86 (m, 6H).

**Step 4: Preparation of (2S,3S,5R,8R,9R,10S,13S,14S)-2,3,13-trimethyl-17-((trimethylsilyl)oxy)-2,3,4,5,6,7,8,9,10,11,12,13,14,15-tetradecahydro-1H-cyclopenta[a]phenanthren-3-ol**

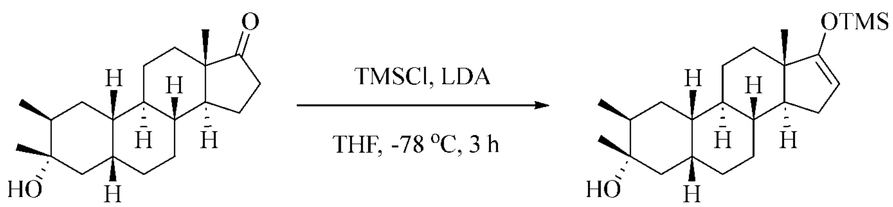


To a dry 100 mL round-bottom flask was added 2.0 M lithium diisopropylamide (32.2 mL, 64.4 mmol). The system was purged with nitrogen and cooled to -78 °C. (2S,3S,5R,8R,9R,10S,13S,14S)-3-Hydroxy-2,3,13-trimethylhexadecahydro-17H-cyclopenta[a]phenanthren-17-one (4.90 g, 16.1 mmol) was dissolved in 35 mL of anhydrous tetrahydrofuran,

and the resulting solution was slowly added dropwise to the reaction system. The reaction system was then stirred for 1 h, and chlorotrimethylsilane (3.50 mL, 40.3 mmol) was added to the reaction system. The reaction mixture was allowed to react at -78 °C for 2 h and then stirred at 0 °C for 15 min. After the reaction was completed as detected by TLC, the reaction was quenched with saturated ammonium chloride, and the mixture was extracted with ethyl acetate. The organic phase was washed with water and saturated brine, dried over anhydrous sodium sulfate, filtered, and concentrated to dryness by rotary evaporation to give a crude product of (2S,3S,5R,8R,9R,10S,13S,14S)-2,3,13-trimethyl-17-((trimethylsilyl) oxy)-2,3,4,5,6,7,8,9,10,11,12,13,14,15-tetradecahydro-1H-cyclopenta[a]phenanthren-3-ol (6.06 g, yield: 100% crude), which was directly used in the next step.

Step **5: Preparation of (2S,3S,5R,8R,9R,10S,13S,14S)-3-hydroxy-2,3,13-trimethyl-1,2,3,4,5,6,7,8,9,10,11,12,13,14-tetradecahydro-17H-cyclopenta[a]phenanthren-17-one**

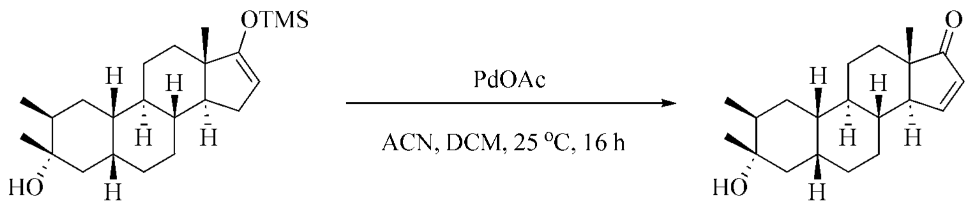


(2S,3S,5R,8R,9R,10S,13S,14S)-2,3,13-Trimethyl-17-((trimethylsilyl)oxy)-2,3,4,5,6,7,8,9,10,11,12,13,14,15-tetradecahydro-1H-cyclopenta[a]phenanthren-3-ol (6.06 g, 16.1 mmol) was dissolved in anhydrous dichloromethane (120 mL) and anhydrous acetonitrile (40.0 mL), and the resulting solution was purged with nitrogen. Palladium acetate (3.50 g, 9.17 mmol) was then added, and the reaction system was stirred at room temperature overnight. The reaction mixture was filtered, and the organic phase was concentrated to give a crude product, which was then separated and purified by column chromatography (petroleum ether/ethyl acetate: 50/1-5/1) to give (2S,3S,5R,8R,9R,10S,13S,14S)-3-hydroxy-2,3,13-trimethyl-1,2,3,4,5,6,7,8,9,10,11,12,13,14-tetradecahydro-17H-cyclopenta[a]phenanthren-17-one (2.20 g, yield: 45.0%).

$^1$H NMR (400 MHz, $CDCl_3$) δ 7.52 (dd, *J* = 6.0, 1.1 Hz, 1H), 6.02 (dd, *J* = 6.0, 3.2 Hz, 1H), 2.37 - 2.33 (m, 1H), 1.91 - 1.21 (m, 17H), 1.15 - 1.10 (m, 4H), 1.07 (s, 3H), 0.87 (d, *J* = 6.7 Hz, 3H).

**Step 6: Preparation of (2S,3S,5R,8R,9R,10S,13S,14S,15R)-3-hydroxy-2,3,13,15-tetramethylhexadecahydro-17H-cyclopenta[a]phenanthren-17-one**

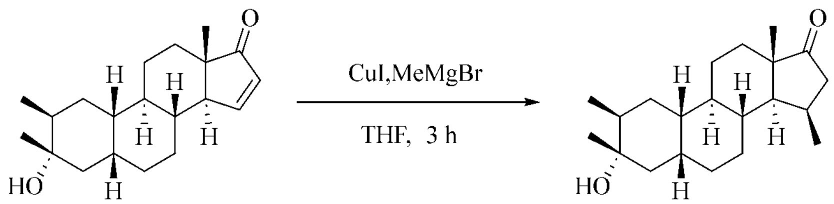


To a dry 100 mL round-bottom flask was added 3.0 M methylmagnesium bromide (9.69 mL, 29.1 mmol). The system was purged with nitrogen and cooled to 0 °C. Cuprous iodide (4.16 g, 21.8 mmol) was added to the reaction system, and the reaction system was stirred at 0 °C for 1 h. (2S,3S,5R,8R,9R,10S,13S,14S)-3-Hydroxy-2,3,13-trimethyl-1,2,3,4,5,6,7,8,9,10,11,12,13,14-tetradecahydro-17H-cyclopenta[a]phenanthren-17-one (2.20 g, 7.27 mmol) was dissolved in 25 mL of anhydrous tetrahydrofuran, and the resulting solution was slowly added dropwise to the reaction system. The reaction system was then stirred for another 2 h. After the reaction was completed as detected by TLC, the reaction was quenched with saturated ammonium chloride, and the mixture was extracted with ethyl acetate. The organic phase was washed with water and saturated brine, dried over anhydrous sodium sulfate, filtered, and concentrated to dryness by rotary evaporation, and the crude product was separated by column chromatography (elution with petroleum ether:ethyl acetate = 3:1) to give (2S,3S,5R,8R,9R,10S,13S,14S,15R)-3-hydroxy-2,3,13,15-tetramethylhexadecahydro-17H-cyclopenta[a]phenanthren-17-one (1.50 g, yield: 65.0%).

$^1$H NMR (400 MHz, $CDCl_3$) δ 2.50 - 2.40 (m, 2H), 2.24 (d, J = 17.6 Hz, 1H), 1.91 - 1.09 (m, 25H), 1.03 (s, 3H), 0.87 (d, J = 6.7 Hz, 3H).

**Step 7: Preparation of (2S,3S,5R,8R,9R,10S,13S,14S,15R,17S)-3-hydroxy-2,3,13,15-tetramethylhexadecahydro-1H-cyclopenta[a]phenanthrene-17-carbonitrile**

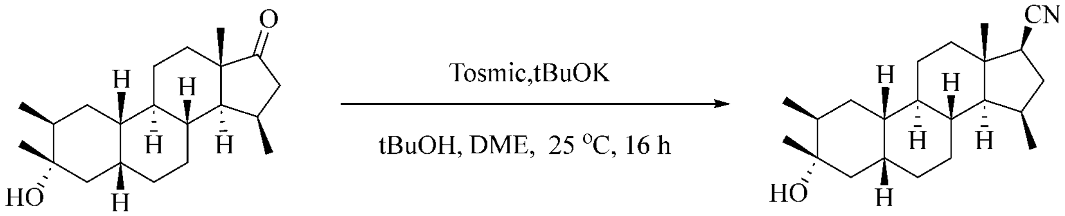


To a dry 100 mL round-bottom flask were added potassium tert-butoxide (5.29 g, 47.2 mmol) and tert-butanol (30.0 mL). The system was purged with nitrogen. (25,3S,5R,8R,9R,10S,13S,14S,15R)-3-Hydroxy-2,3,13,15-tetramethylhexadecahydro-17H-cyclopenta[a]phenanthren-17-one (1.50 g, 4.71 mmol) was dissolved in 15 mL of ethylene glycol dimethyl ether, and the resulting solution was slowly added dropwise to the reaction system. The reaction system was then stirred for 30 min. p-Toluenesulfonylmethyl isocyanide (1.84 g, 9.46 mmol) was dissolved in 15 mL of ethylene glycol dimethyl ether, and the resulting solution was slowly added dropwise to the reaction system. The reaction system was then stirred at room temperature overnight. After the reaction was completed as detected by TLC, water (50 mL) was added to quench the reaction, and the aqueous phase was extracted with ethyl acetate (50 mL × 2). The organic phases were combined, dried over anhydrous sodium sulfate, filtered, and concentrated to give a crude product, which was then separated and purified by column chromatography (petroleum ether/ethyl acetate: 50/1-3/1) to give (2S,3S,5R,8R,9R,10S,13S,14S,15R,17S)-3-hydroxy-2,3,13,15-tetramethylhexadecahydro-1H-cyclopenta[a]phenanthrene-17-carbonitrile (0.95 g, yield: 61.3%).

$^1H$ NMR (400 MHz, $CDCl_3$) δ 2.51 - 2.48 (m, 1H), 2.26 - 2.21 (m, 1H), 1.89 - 1.21 (m, 18H), 1.16 - 1.09 (m, 6H), 1.06 (s, 3H), 1.00 (d, *J* = 7.4 Hz, 3H), 0.87 (d, *J* = 6.7 Hz, 3H).

**Step 8: Preparation of 1-((2S,35,5R,8R,9R,10S,13S,14S,15R,17S)-3-hydroxy-2,3,13,15-tetramethylhexadecahydro-1H-cyclopenta[a]phenanthren-17-yl)ethan-1-one**

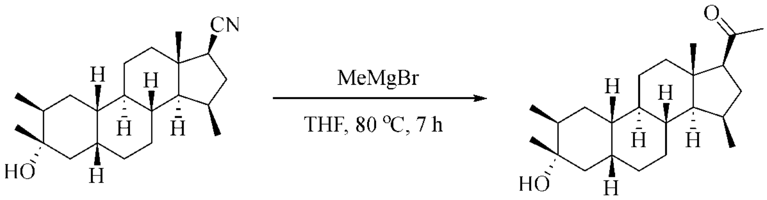


(2S,3S,5R,8R,9R,10S,13S,14S,15R,17S)-3-Hydroxy-2,3,13,15-tetramethylhexadecahydro-1H-cyclopenta[a]phenanthrene-17-carbonitrile (950 mg, 2.88 mmol) was dissolved in a solution of dry tetrahydrofuran (100 mL). The system was purged with nitrogen, and 3.0 M methylmagnesium bromide (28.5 mL, 85.5 mmol) was slowly added dropwise. The reaction system was then stirred at 80 °C for 7 h. After the reaction was completed as detected by TLC, the reaction was quenched with saturated ammonium chloride, and the mixture was extracted with ethyl acetate. The organic phase was washed with water and saturated brine, dried over anhydrous sodium sulfate, filtered, and concentrated to dryness by rotary evaporation, and the crude product was separated by column chromatography (elution with petroleum ether:ethyl acetate = 4:1) to give 1-((2S,3S,5R,8R,9R,10S,13S,14S,15R,17S)-3-hydroxy-2,3,13,15-tetramethylhexadecahydro-1H-cyclopenta[a]phenanthren-17-yl)ethan-1-one (540 mg, yield: 54.0%).

$^1H$ NMR (400 MHz, $CDCl_3$) δ 2.49 (dd, J = 10.4, 8.6 Hz, 1H), 2.20 - 2.13 (m, 1H), 2.11 (s, 3H), 2.09 - 2.00 (m, 1H), 1.96 - 1.91 (m, 1H), 1.86 - 1.21 (m, 16H), 1.15 - 1.02 (m, 6H), 0.96 (d, J = 7.2 Hz, 3H), 0.87 (d, J = 6.7 Hz, 3H), 0.77 (s, 3H).

**Example 2**

**1-(2-((2S,3S,5R,8R,9R,10S,13S,14S,15R,17S)-3-Hydroxy-2,3,13,15-tetramethylhexadecahydro-1H-cyclopenta[a]phenanthren-17-yl)-2-oxoethyl)-1H-pyrazole-4-carbonitrile**

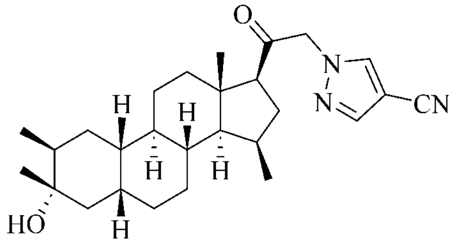

2

Synthesis scheme:

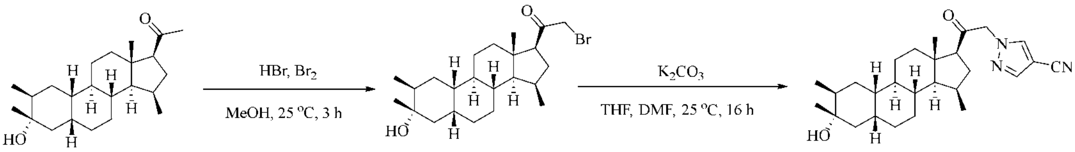


1 2-1 2

**Step 1: Preparation of 2-bromo-1-((2S,3S,5R,8R,9R,10S,13S,14S,15R,17S)-3-hydroxy-2,3,13,15-tetramethylhexadecahydro-1H-cyclopenta[a]phenanthren-17-yl)ethan-1-one**

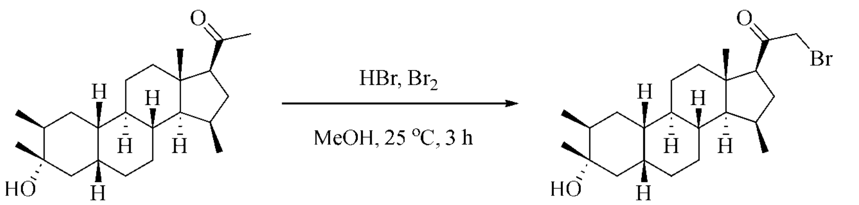


1-((2S,3S,5R,8R,9R,10S,13S,14S,15R,17S)-3-Hydroxy-2,3,13,15-tetramethylhexadecahydro-1H-cyclopenta[a]phenanthren-17-yl)ethan-1-one (150 mg, 0.43 mmol) was dissolved in methanol (5 mL), and a drop of hydrogen bromide was added, followed by the addition of liquid bromine (75.6 mg, 0.48 mmol). The reaction system was stirred at room temperature for 3 h. Water (20.0 mL) was added, and the aqueous phase was extracted with ethyl acetate (20.0 mL × 2). The organic phases were combined, dried over anhydrous sodium sulfate, filtered, and concentrated to give a crude product (150 mg, crude product), which was directly used in the next step.

**Step 2: Preparation of 1-(2-((2S,3S,5R,8R,9R,10S,13S,14S,15R,17S)-3-hydroxy-2,3,13,15-tetramethylhexadecahydro-1H-cyclopenta[a]phenanthren-17-yl)-2-oxoethyl)-1H-pyrazole-4-carbonitrile**

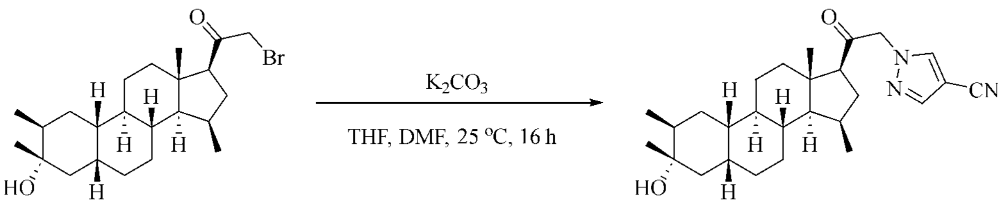

2-Bromo-1-((2S,3S,5R,8R,9R,10S,13S,14S,15R,17S)-3-hydroxy-2,3,13,15-tetramethylhexadecahydro-1H-cyclopenta[a]phenanthren-17-yl)ethan-1-one (150 mg, 0.35 mmol), 1H-pyrazole-4-carbonitrile (48.0 mg, 0.52 mmol), and potassium carbonate (72.5 mg, 0.52 mmol) were dissolved in anhydrous tetrahydrofuran (5.00 mL) and N,N-dimethylformamide (1.00 mL), and the reaction system was stirred at room temperature overnight. The reaction mixture was then filtered, and the filtrate was concentrated. The crude product was separated by a high-performance liquid chromatography column to give 1-(2-((2S,3S,5R,8R,9R,10S,13S,14S,15R,17S)-3-hydroxy-2,3,13,15-tetramethylhexadecahydro-1H-cyclopenta[a]phenanthren-17-yl)-2-oxoethyl)-1H-pyrazole-4-carbonitrile (51.0 mg, yield: 33.0%).

$^1$H NMR (400 MHz, $CDCl_3$) δ 7.86 (s, 1H), 7.81 (s, 1H), 5.03 - 4.88 (m, 2H), 2.55 (dd, *J* = 10.2, 8.4 Hz, 1H), 2.27 - 2.10 (m, 2H), 2.03 - 1.81 (m, 5H), 1.75 - 1.25 (m, 11H), 1.17 - 1.03 (m, 7H), 0.98 (d, *J* = 7.0 Hz, 3H), 0.87 (d, *J* = 6.7 Hz, 3H), 0.83 (s, 3H).

$^{13}$C NMR (101 MHz, $CDCl_3$) δ 201.98, 142.38, 136.01, 113.23, 93.17, 74.11, 61.69, 61.40, 57.43, 45.52, 43.08, 41.29, 41.22, 39.41, 39.26, 35.94, 35.09, 34.09, 33.53, 31.32, 30.25, 26.09, 25.21, 20.47, 18.25, 16.81, 15.13.

LC-MS m/z (ESI): 420.25 $[M+H-18]^+$.

**Example 3**

**1-(2-((2S,3S,5R,8R,9R,10S,13S,14S,15R,17S)-3-Hydroxy-2,3,13,15-tetramethylhexadecahydro-1H-cyclopenta [a]phenanthren-17-yl)-2-oxoethyl)-1H-pyrazole-3-carbonitrile**

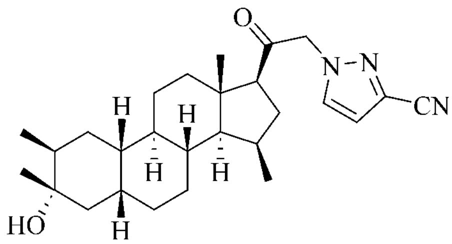

3

Synthesis scheme

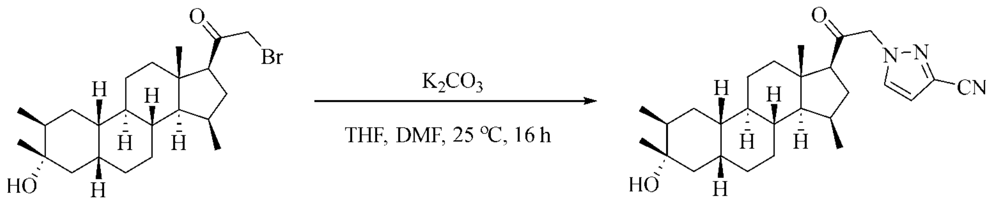


The synthesis method was the same as that in Example 2, except that the 1H-pyrazole-4-carbonitrile in step 2 was replaced by **1H-pyrazole-3-carbonitrile.**

$^1$H NMR (400 MHz, $CDCl_3$) δ 7.48 (d, *J* = 2.4 Hz, 1H), 6.73 (d, *J* = 2.4 Hz, 1H), 5.04 - 4.90 (m, 2H), 2.55 (dd, *J* = 10.3, 8.3 Hz, 1H), 2.26 - 2.09 (m, 2H), 2.00 - 1.95 (m, 1H), 1.90 - 1.81 (m, 3H), 1.76 - 1.23 (m, 12H), 1.18 - 1.03 (m, 7H), 0.98 (d, *J* = 7.1 Hz, 3H), 0.88 (d, *J* = 6.7 Hz, 3H), 0.83 (s, 3H).

$^{13}$C NMR (101 MHz, $CDCl_3$) δ 202.16, 132.19, 125.17, 113.89, 111.96, 74.12, 62.08, 61.36, 57.42, 45.52, 43.09, 41.26, 41.22, 39.41, 39.26, 35.96, 35.10, 34.09, 33.54, 31.33, 30.25, 26.09, 25.22, 20.45, 18.25, 16.80, 15.12.

LC-MS m/z (ESI): 420.20 $[M+H-18]^+$.

**Example 4**

**1-((2S,3S,5R,8R,9R,10S,13S,14S,15R,17S)-3-Hydroxy-2,3,13,15-tetramethylhexadecahydro-1H. cyclopenta[a]phenanthren-17-yl)-2-(3-(trifluoromethyl)-1H-pyrazol-1-yl)ethan-1-one**

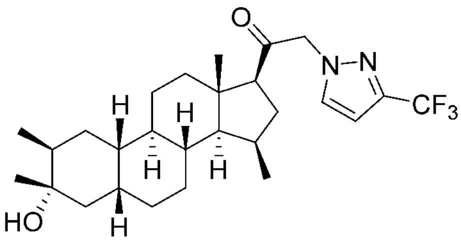

Synthesis scheme

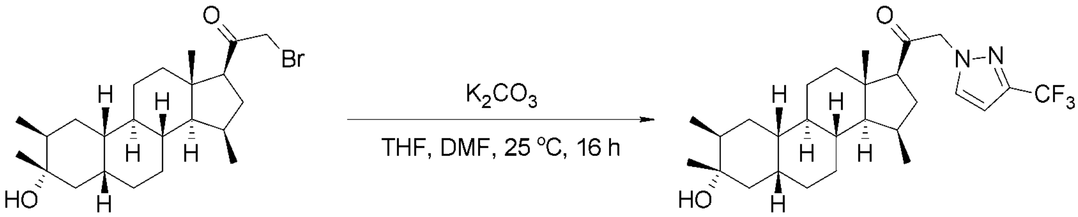


The synthesis method was the same as that in Example 2, except that the 1H-pyrazole-4-carbonitrile in step 2 was replaced by **3-(trifluoromethyl)-1H-pyrazole.**

$^1$H NMR (400 MHz, $CDCl_3$) δ 7.47 (dt, *J* = 2.0, 1.0 Hz, 1H), 6.59 (d, *J*= 2.4 Hz, 1H), 4.97 (d, *J* = 4.6 Hz, 2H), 2.54 (dd, *J* = 10.5, 8.1 Hz, 1H), 2.29 - 2.10 (m, 2H), 1.97 (m, 1H), 1.92 - 1.05 (m, 20H), 0.98 (d, *J* = 7.0 Hz, 3H), 0.88 (d, *J* = 6.7 Hz, 3H), 0.84 (s, 3H).

$^{13}$C NMR (101 MHz, $CDCl_3$) δ 202.58, 142.57 (q, $J_{C-F}$ = 38.0 Hz), 132.26, 122.71 (q, $J_{C-F}$ = 145.0 Hz), 105.01, 74.12, 61.87, 61.29, 57.36, 45.34, 43.10, 41.24, 41.21, 39.41, 39.26, 35.96, 35.10, 34.09, 33.60, 31.34, 30.24, 26.09, 25.20, 20.42, 18.26, 16.75, 15.12.

MS m/z (ESI): 479.2 [M-H]$^-$.

**Example 5**

**1-(2-((2S,3S,5R,8R,9R,10S,13S,14S,15R,17S)-3-Hydroxy-2,3,13,15-tetramethylhexadecahydro-1H-cyclopenta [a]phenanthren-17-yl)-2-oxoethyl)-1H-pyrazole-3,5-dicarbonitrile**

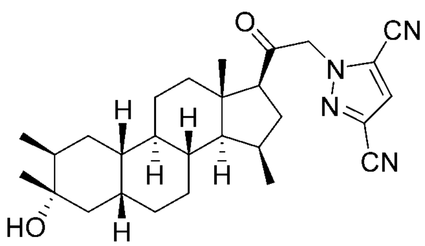

Synthesis scheme

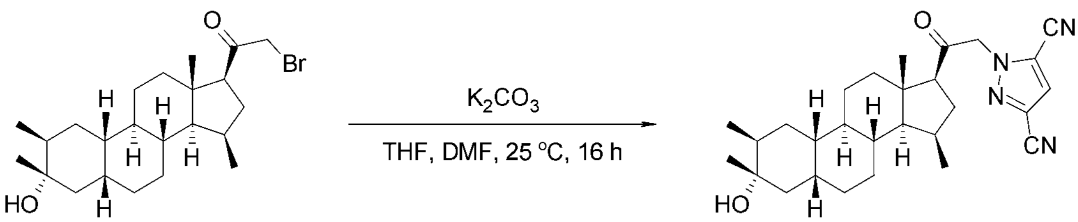

The synthesis method was the same as that in Example 2, except that the 1H-pyrazole-4-carbonitrile in step 2 was replaced by **1H-pyrazole-3,5-dicarbonitrile.**

$^1$H NMR (400 MHz, $CDCl_3$) δ 7.21 (s, 1H), 5.16 (d, *J* = 1.6 Hz, 2H), 2.61 - 2.57 (m, 1H), 2.28 - 2.17 (m, 2H), 2.02 - 1.85 (m, 4H), 1.77 - 1.07 (m, 19H), 0.99 (d, *J* = 6.8 Hz, 3H), 0.88 - 0.86 (m, 6H).

$^{13}$C NMR (101 MHz, $CDCl_3$) δ 200.10, 125.91, 118.36, 118.13, 111.80, 108.72, 74.13, 61.95, 61.33, 57.51, 45.86, 43.08, 41.21, 39.42, 39.25, 35.94, 35.08, 34.08, 33.57, 31.31, 30.30, 26.09, 25.23, 20.48, 18.23, 16.66, 15.12.

MS m/z (ESI): 461.15 [M-H]$^-$.

**Example 6**

**1-((2S,3S,5R,8R,9R,10S,13S,14S,15R,17S)-3-Hydroxy-2,3,13,15-tetramethylhexadecahydro-1H-cyclopenta[a]phenanthren-17-yl)-2-(1H-1,2,4-triazol-1-yl)ethan-1-one**

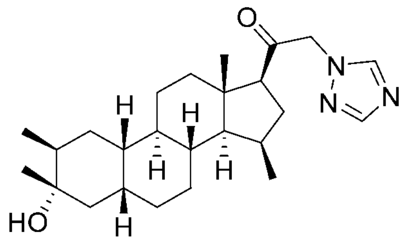

Synthesis scheme

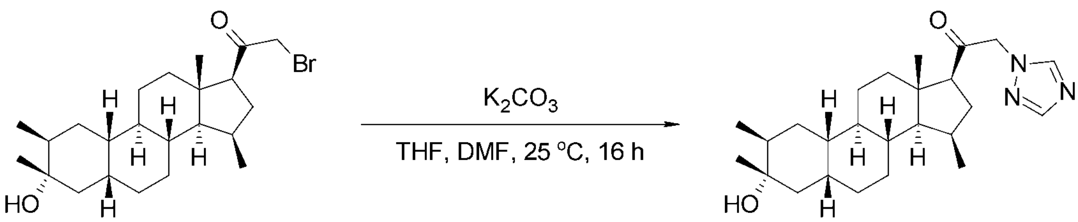


The synthesis method was the same as that in Example 2, except that the 1H-pyrazole-4-carbonitrile in step 2 was replaced by **1H-1,2,4-triazole.**

$^1$H NMR (400 MHz, $CDCl_3$) δ 8.14 (s, 1H), 7.96 (s, 1H), 5.04 - 4.91 (m, 2H), 2.59 - 2.55 (m, 1H), 2.26 - 2.13 (m, 2H), 2.00 - 1.84 (m, 4H), 1.76 - 1.04 (m, 19H), 0.98 (d, *J* = 6.8 Hz, 3H), 0.87 (d, *J* = 6.8 Hz, 3H), 0.84 (s, 3H).

$^{13}$C NMR (101 MHz, $CDCl_3$) δ 201.99, 151.82, 144.53, 74.11, 61.38, 58.76, 57.42, 45.47, 43.10, 41.27, 41.22, 39.42, 39.27, 35.96, 35.10, 34.10, 33.56, 31.33, 30.25, 26.09, 25.22, 20.45, 18.26, 16.80, 15.13.

MS m/z (ESI): 414.40 [M+H]$^+$.

**Example 7**

**1-((2S,3S,5R,8R,9R,10S,13S,14S,15R,17S)-3-Hydroxy-2,3,13,15-tetramethylhexadecahydro-1H-cyclopenta[a]phenanthren-17-yl)-2-(2H-1,2,3-triazol-2-yl)ethan-1-one**

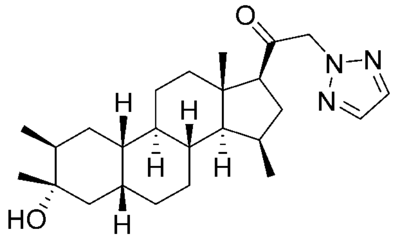

**Example 8**

**1-((2S,3S,5R,8R,9R,108,13S,14S,15R,17S)-3-Hydroxy-2,3,13,15-tetramethylhexadecahydro-1H-cyclopenta[a]phenanthren-17-yl)-2-(1H-1,2,3-triazol-1-yl)ethan-1-one**

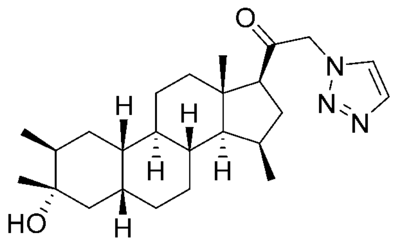

Synthesis scheme

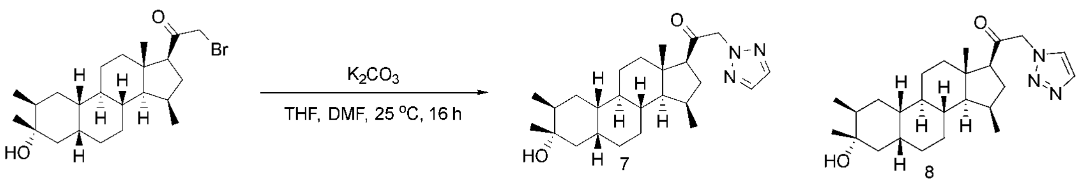


7

8

The synthesis method was the same as that in Example 2, except that the 1H-pyrazole-4-carbonitrile in step 2 was replaced by **1H-1,2,3-triazole,** and finally column chromatography separation was performed to give product 1 (compound 7) and product 2 (compound 8).

Compound 7:

$^1$H NMR (400 MHz, $CDCl_3$) δ 7.69 (s, 2H), 5.24 (d, *J* = 1.2 Hz, 2H), 2.52 (dd, *J* = 10.5, 8.1 Hz, 1H), 2.26 - 2.07 (m, 2H), 2.05 - 1.96 (m, 1H), 1.92 - 1.27 (m, 17H), 1.12 (s, 3H), 0.97 (d, *J* = 7.1 Hz, 3H), 0.89 - 0.85 (m, 6H).
$^{13}$C NMR (101 MHz, $CDCl_3$) δ 202.61, 135.03, 74.13, 63.87, 60.88, 57.37, 45.34, 43.11, 41.23, 41.14, 39.41, 39.28, 35.96, 35.12, 34.11, 33.63, 31.35, 30.26, 26.10, 25.21, 18.25, 16.71, 15.12.
MS m/z (ESI): 414.3 $[M+H]^+$.

Compound 8:

$^1$H NMR (400 MHz, $CDCl_3$) δ 7.76 (d, *J* = 1.0 Hz, 1H), 7.65 (d, *J* = 1.1 Hz, 1H), 5.29 - 5.11 (m, 2H), 2.60 (dd, *J* = 10.5, 8.1 Hz, 1H), 2.31 - 2.12 (m, 2H), 2.07 - 1.97 (m, 1H), 1.93 - 1.83 (m, 3H), 1.79 - 1.12 (m, 16H), 0.98 (d, *J* = 7.0 Hz, 3H), 0.88 (d, *J* = 6.8 Hz, 3H), 0.84 (s, 3H).
$^{13}$C NMR (101 MHz, $CDCl_3$) δ 201.69, 134.01, 125.04, 74.14, 61.46, 58.89, 57.40, 45.52, 43.08, 41.25, 41.21, 39.41, 39.26, 35.96, 35.09, 34.09, 33.56, 31.33, 30.26, 26.08, 25.21, 20.42, 18.26, 16.79, 15.11.
MS m/z (ESI): 396.2 $[M+H-H_2O]^+$.

**Example 9**

**1-((2S,3S,5R,8R,9R,10S,13S,14S,15R,17S)-3-Hydroxy-2,3,13,15-tetramethylhexadecahydro-1H-cyclopenta[a]phenanthren-17-yl)-2-(2H-tetrazol-2-yl)ethan-1-one**

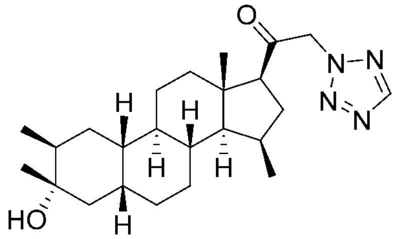

**Example 10**

**1-((2S,3S,5R,8R,9R,10S,13S,14S,15R,17S)-3-Hydroxy-2,3,13,15-tetramethylhexadecahydro-1H-cyclopenta[a]phenanthren-17-yl)-2-(1H-tetrazol-1-yl)ethan-1-one**

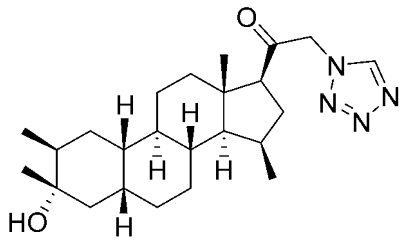

Synthesis scheme

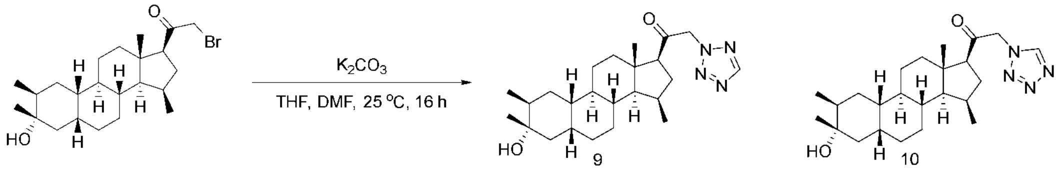

9
10

The synthesis method was the same as that in Example 2, except that the 1H-pyrazole-4-carbonitrile in step 2 was replaced by **1H-tetrazole,** and finally column chromatography separation was performed to give product 1 (compound 9) and product 2 (compound 10).

Compound 9:

$^1$H NMR (400 MHz, $CDCl_3$) δ 8.57 (s, 1H), 5.45 (s, 2H), 2.66 - 2.56 (m, 1H), 2.28 - 2.15 (m, 2H), 2.03 - 1.11 (m, 23H), 0.98 (d, *J* = 6.8 Hz, 3H), 0.91 - 0.86 (m, 6H).
$^{13}$C NMR (101 MHz, $CDCl_3$) δ 200.10, 153.22, 74.12, 61.42, 61.34, 57.43, 45.58, 43.11, 41.23, 41.19, 39.42, 39.27, 35.96, 35.10, 34.10, 33.57, 31.33, 30.28, 26.09, 25.22, 20.45, 18.23, 16.73, 15.11.
MS m/z (ESI): 413.15 $[M-H]^-$.

Compound 10:

$^1$H NMR (400 MHz, $CDCl_3$) δ 8.74 (s, 1H), 5.33 - 5.17 (m, 2H), 2.67 - 2.60 (m, 1H), 2.29 - 2.16 (m, 2H), 2.00 - 1.08 (m, 23H), 0.99 (d, *J* = 6.8 Hz, 3H), 0.88 (d, *J* = 6.4 Hz, 3H), 0.84 (s, 3H).
$^{13}$C NMR (101 MHz, $CDCl_3$) δ 200.07, 143.63, 74.11, 61.70, 57.46, 56.74, 45.71, 43.07, 41.31, 41.21, 39.42,

39.26, 35.94, 35.08, 34.08, 33.55, 31.31, 30.28, 26.07, 25.20, 20.48,18.24, 16.83, 15.10.

MS m/z (ESI): 413.15 [M-H]$^-$.

**Example 11**

**1-((2S,3S,5R,8R,9R,10S,13S,14S,15R,17S)-3-Hydroxy-2,3,13,15-tetramethylhexadecahydro-1H-cyclopenta[a] phenanthren-17-yl)-2-(5-methyl-2H-tetrazol-2-yl)ethan-1-one**

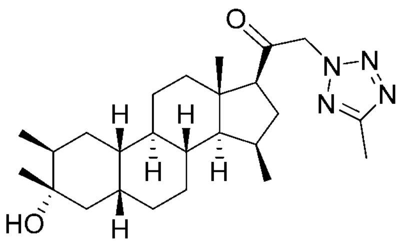

**Example 12**

**1-((2S,3S,5R,8R,9R,108,13S,14S,15R,17S)-3-Hydroxy-2,3,13,15-tetramethylhexadecahydro-1H-cyclopenta[a] phenanthren-17-yl)-2-(5-methyl-1H-tetrazol-1-yl)ethan-1-one**

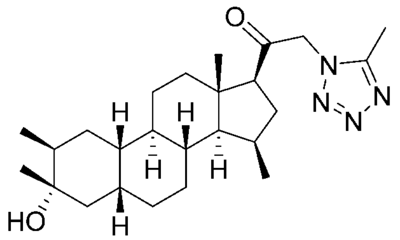

Synthesis scheme

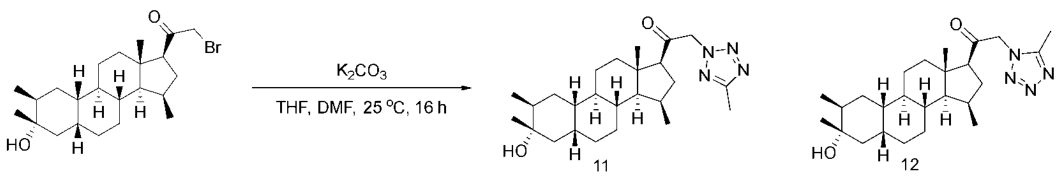


The synthesis method was the same as that in Example 2, except that the 1H-pyrazole-4-carbonitrile in step 2 was replaced by **5-methyl-1H-tetrazole,** and finally column chromatography separation was performed to give product 1 (compound 11) and product 2 (compound 12).

Compound 11:

$^1$H NMR (400 MHz, $CDCl_3$) δ 5.35 (s, 2H), 2.57 (s, 3H), 2.30 - 2.12 (m, 2H), 2.00 (m, 1H)1.95 -1.05 (m,23 H), 0.98 (d, *J* = 6.9 Hz, 3H), 0.92 - 0.84 (m, 6H).

$^{13}$C NMR (101 MHz, $CDCl_3$) δ 200.42, 163.38, 77.34, 77.02, 76.71, 74.13, 61.27, 57.39, 45.51, 43.09, 41.22, 39.41, 35.95, 35.09, 34.09, 33.56, 31.32, 30.26, 26.09, 25.20, 20.44, 18.24, 16.73, 15.11, 10.94.

MS m/z (ESI): 429.3 [M+H]$^+$.

Compound 12:

$^1H$ NMR (400 MHz, $CDCl_3$) δ 5.19 - 5.02 (m, 2H), 2.61 (dd, *J* = 10.4, 8.0 Hz, 1H), 2.47 (s, 3H), 2.33 - 2.12 (m, 2H), 2.07 - 1.96 (m, 1H), 1.94 - 1.81 (m, 3H), 1.78 - 1.06 (m, 19H), 0.99 (d, *J* = 7.0 Hz, 3H), 0.93 - 0.81 (m, 6H).
$^{13}C$ NMR (101 MHz, $CDCl_3$) δ 200.60, 152.42, 74.11, 61.41, 57.46, 56.08, 46.35, 43.72, 41.19, 39.85, 39.24, 35.93, 35.07, 34.08, 33.51, 31.30, 30.70, 30.27, 26.89, 25.23, 20.48, 18.23, 16.84, 15.12, 8.91.
MS m/z (ESI): 429.3 $[M+H]^+$.

**Example 13**

**2-((1,3,4-Thiadiazol-2-yl)amino)-1-((2S,3S,5R,8R,9R,10S,13S,14S,15R,17S)-3-hydroxy-2,3,13,15-tetramethylhexadecahydro-1H-cyclopenta[a]phenanthren-17-yl)ethan-1-one**

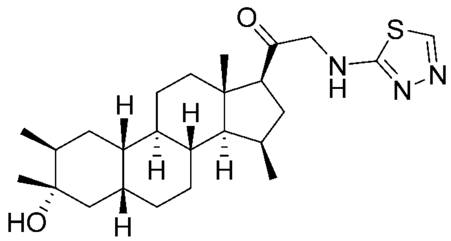

Synthesis scheme

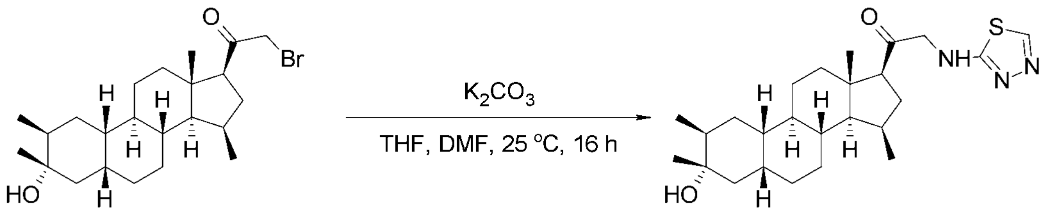


The synthesis method was the same as that in Example 2, except that the 1H-pyrazole-4-carbonitrile in step 2 was replaced by **1,3,4-thiadiazole-2-amino.**
$^1H$ NMR (400 MHz, $CDCl_3$) δ 8.45 (s, 1H), 7.66 (s, 1H), 4.78 (s, 2H), 2.57 (dd, *J* = 10.5, 8.0 Hz, 1H), 2.29 - 1.20 (m, 17H), 1.10 (d, *J* = 9.5 Hz, 6H), 0.97 (d, *J* = 7.0 Hz, 3H), 0.87 (dd, *J* = 6.8, 3.1 Hz, 3H), 0.84 (s, 3H).
$^{13}C$ NMR (101 MHz, $CDCl_3$) δ 203.75, 161.72, 133.15, 74.14, 60.91, 57.43, 57.34, 45.22, 43.15, 41.27, 41.01, 39.42, 39.30, 39.28, 35.98, 35.15, 34.13, 33.50, 31.38, 30.24, 26.13, 25.27, 20.39, 18.29, 16.61, 16.46, 15.12.
MS m/z (ESI): 446.2 $[M+H]^+$.

**Example 14**

**1-(2-((2S,3S,5R,8R,9R,10S,13S,14S,15R,17S)-3-Hydroxy-2,13,15-trimethylhexadecahydro-1H-cyclopenta[a] phenanthren-17-yl)-2-oxoethyl)-1H-pyrazole-4-carbonitrile**

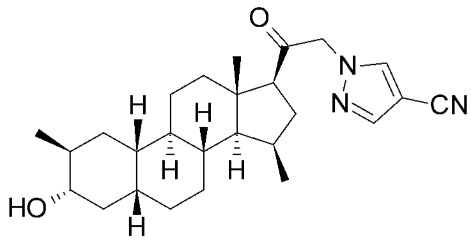

Synthesis scheme

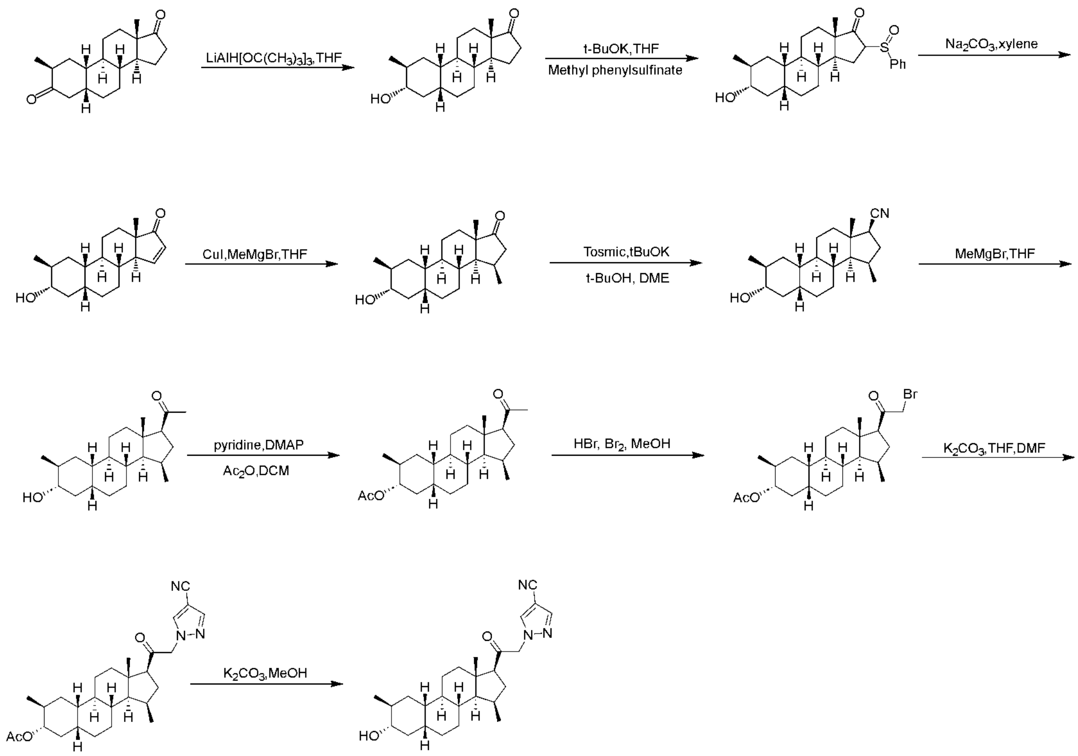


**Step 1: Preparation of (2S,3S,5R,8R,9R,10S,13S,14S)-3-hydroxy-2,13-dimethylhexadecahydro-17H-cyclopenta[a]phenanthren-17-one**

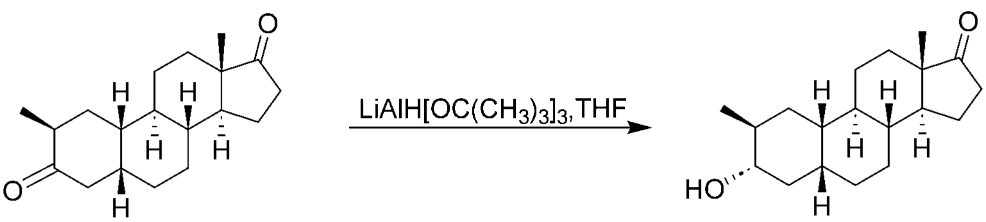


(2S,5R,8R,9R,10S,13S,14S)-2,13-Dimethyltetradecahydro-3H-cyclopenta[a]phenanthrene-3,17(2H)-dione (20.0 g, 69.4 mmol) was dissolved in tetrahydrofuran (1000 mL). The resulting solution was cooled to -70 °C, and then a solution of lithium tri-tert-butoxyaluminum hydride (31.4 g, 123.5 mmol) in tetrahydrofuran (400 mL) was added. The mixture was warmed to -50 °C and allowed to react for 3 h. An aqueous ammonium chloride solution was added to quench the reaction, and the mixture was extracted with ethyl acetate, dried over anhydrous sodium sulfate, filtered, and concentrated. The residue was subjected to column chromatography (DCM/EA = 0-30%) to give the product (2S,3S,5R,8R,9R,10S,13S,14S)-3-hydroxy-2,13-dimethylhexadecahydro-17H-cyclopenta[a]phenanthren-17-one (9.0

g, yield: 45%).

$^{1}H$ NMR (400 MHz, $CDCl_3$) δ 3.20 - 3.14 (m, 1H), 2.61 - 2.34 (m, 2H), 2.10 - 0.92 (m, 24H), 0.86 (s, 3H).

**Step 2: Preparation of (2S,3S,5R,8R,9R,10S,13S,14S)-3-hydroxy-2,13-dimethyl-16-(phenylsulfinyl)hexadecahydro-17H-cyclopenta[a]phenanthren-17-one**

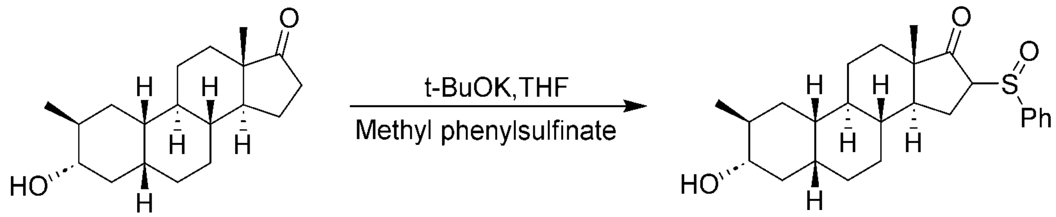


(2S,3S,5R,8R,9R,10S,13S,14S)-3-Hydroxy-2,13-dimethylhexadecahydro-17H-cyclopenta[a]phenanthren-17-one (9.0 g, 31.0 mmol) was added to a solution of potassium tert-butoxide (6.9 g, 61.6 mmol) in tetrahydrofuran (180 mL). The mixture was stirred at room temperature for 10 min under a nitrogen atmosphere, and then methyl benzenesulfinate (9.6 g, 61.5 mmol) was added. The resulting mixture was stirred at room temperature for another 1 h. Water was added to quench the reaction, and the mixture was extracted with ethyl acetate, dried over anhydrous sodium sulfate, filtered, and concentrated. The residue was subjected to column chromatography (DCM/EA = 0-30%) to give the product (25,3S,5R,8R,9R,10S,13S,14S)-3-hydroxy-2,13-dimethyl-16-(phenylsulfinyl)hexadecahydro-17H-cyclopenta [a]phenanthren-17-one (7.8 g, yield: 60%).

MS m/z (ESI): 415 $[M+H]^+$.

**Step 3: Preparation of (2S,3S,5R,8R,9R,10S,13S,14S)-3-hydroxy-2,13-dimethyl-1,2,3,4,5,6,7,8,9,10,11,12,13,14-tetradecahydro-17H-cyclopenta[a]phenanthren-17-one**

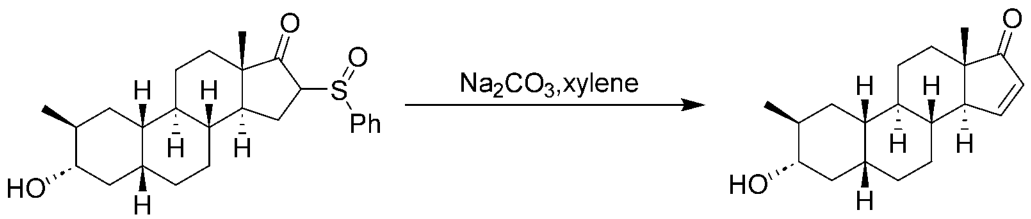


(2S,3S,5R,8R,9R,10S,13S,14S)-3-Hydroxy-2,13-dimethyl-16-(phenylsulfinyl)hexadecahydro-17H-cyclopenta[a]phenanthren-17-one (7.8 g, 18.8 mmol) was dissolved in xylene (124 mL), and anhydrous sodium carbonate (29.0 g, 27.4 mmol) was added. The mixture was stirred at 125 °C for 24 h, filtered to remove the sodium carbonate, and then concentrated. The residue was subjected to column chromatography (DCM/EA = 0-30%) to give the product (2S,3S,5R,8R,9R,10S,13S,14S)-3-hydroxy-2,13-dimethyl-1,2,3,4,5,6,7,8,9,10,11,12,13,14-tetradecahydro-17H-cyclopenta[a]phenanthren-17-one (2.6 g, yield: 48%).

$^{1}H$ NMR (400 MHz, $CDCl_3$) δ 7.53 - 7.51 (m, 1H), 6.04 - 6.01 (m, 1H), 3.22 - 3.16 (m, 1H), 2.38 - 2.34(m, 1H), 1.91 - 1.21(m, 18H), 1.07 (s, 3H), 0.97 (d, $J$ = 6.4 Hz, 3H).

**Step 4: Preparation of (2S,3S,5R,8R,9R,10S,13S,14S,15R)-3-hydroxy-2,13,15-trimethylhexadecahydro-17H-cyclopenta[a]phenanthren-17-one**

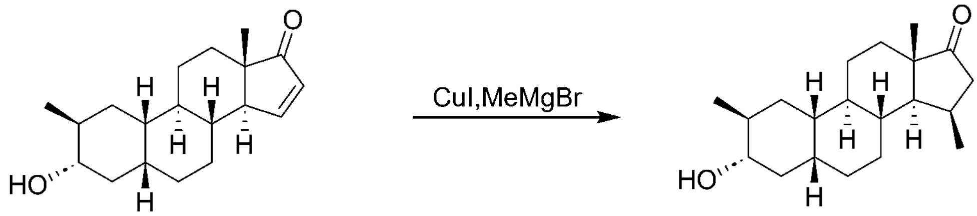


Cuprous iodide (4.9 g, 25.7 mmol) was added to a 3 M solution of methyl Grignard reagent (11.46 mL) in tetrahydrofuran under an ice bath, and the reaction system was stirred at 0 °C for 1 h. A solution of (2S,3S,5R,8R,9R,10S,13S,14S)-3-hydroxy-2,13-dimethyl-1,2,3,4,5,6,7,8,9,10,11,12,13,14-tetradecahydro-17H-cyclopenta[a]phenanthren-17-one (2.6 g, 9.02 mmol) in tetrahydrofuran (32 mL) was then added, and the mixture was stirred under the ice bath for 2 h. After the reaction was completed, an aqueous ammonium chloride solution was added to quench the reaction, and the mixture was extracted with ethyl acetate. The organic phase was then dried over anhydrous sodium sulfate and concentrated. The residue was subjected to column chromatography (DCM/EA = 0-30%) to give the product (2S,3S,5R,8R,9R,10S,13S,14S,15R)-3-hydroxy-2,13,15-trimethylhexadecahydro-17H-cyclopenta[a]phenanthren-17-one (2.3 g, yield: 84%).

$^1$H NMR (400 MHz, $CDCl_3$) δ 3.21 - 3.15(m, 1H), 2.50 - 2.38(m, 2H), 2.30 - 2.22(m,1H), 1.90 - 1.18(m,19H), 1.10 (d, *J* = 7.2 Hz, 3H), 1.03 (s, 3H), 0.97 (d, *J* = 6.4 Hz, 3H).

**Step 5: Preparation of (2S,3S,5R,8R,9R,10S,13S,14S,15R,17S)-3-hydroxy-2,13,15. trimethylhexadecahydro-1H-cyclopenta[a]phenanthrene-17-carbonitrile**

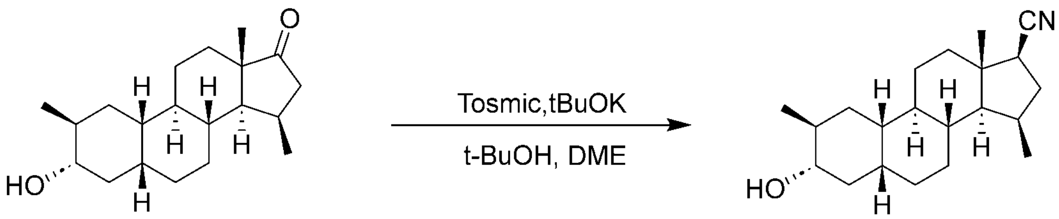


To a dry 100 mL round-bottom flask were added potassium tert-butoxide (8 g, 71.29 mmol) and tert-butanol (46 mL). The system was purged with nitrogen. (2S,3S,5R,8R,9R,10S,13S,14S,15R)-3-Hydroxy-2,13,15-trimethylhexadecahydro-17H-cyclopenta[a]phenanthren-17-one (2.3 g, 7.6 mmol) was dissolved in ethylene glycol dimethyl ether (12 mL), and the resulting solution was slowly added dropwise to the reaction system. The reaction system was then stirred for 30 min. p-Toluenesulfonylmethyl isocyanide (2.8 g, 14.4 mmol) was dissolved in ethylene glycol dimethyl ether (12 mL), and the resulting solution was slowly added dropwise to the reaction system. The reaction system was then stirred at room temperature overnight. After the reaction was completed as detected by TLC, water was added to quench the reaction, and the mixture was extracted with ethyl acetate. The organic phase was then dried over anhydrous sodium sulfate and concentrated. The residue was subjected to column chromatography (DCM/EA = 0-30%) to give the product (2S,3S,5R,8R,9R,10S,13S,14S,15R,17S)-3-hydroxy-2,13,15-trimethylhexadecahydro-1H-cyclopenta[a]phenanthrene-17-carbonitrile (2.0 g, yield: 84%).

$^1$H NMR (400 MHz, $CDCl_3$) δ 3.21 - 3.15(m, 1H), 2.53 - 2.43(m, 1H), 2.27 - 2.20(m,2H), 1.89 - 1.12(m,20H), 1.06 (s, 3H), 1.01 - 0.96(m, 6H).

**Step 6: Preparation of 1-((2S,3S,5R,8R,9R,10S,13S,14S,15R,17S)-3-hydroxy-2,13,15-trimethylhexadecahydro-1H-cyclopenta[a]phenanthren-17-yl)ethan-1-one**

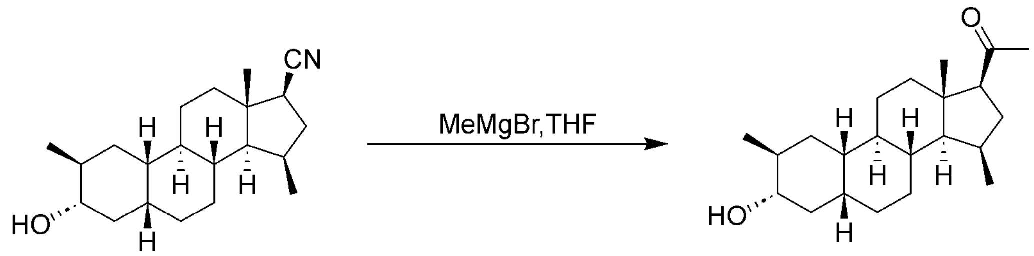


(2S,3S,5R,8R,9R,10S,13S,14S,15R,17S)-3-Hydroxy-2,13,15-trimethylhexadecahydro-1H-cyclopenta[a]phenanthrene-17-carbonitrile (2.0 g, 6.3 mmol) was dissolved in a solution of dry tetrahydrofuran (250 mL). The system was purged with nitrogen, and 3.0 M methylmagnesium bromide (60 mL) was slowly added dropwise. The reaction system was then stirred at 80 °C for 7 h. After the reaction was completed as detected by TLC, the reaction was quenched with saturated ammonium chloride, and the mixture was extracted with ethyl acetate. The organic phase was then dried over anhydrous sodium sulfate and concentrated. The residue was subjected to column chromatography (DCM/EA = 0-30%) to give the product 1-((2S,3S,5R,8R,9R,10S,13S,14S,15R,17S)-3-hydroxy-2,13,15-trimethylhexadecahydro-1H-cyclopenta[a]phenanthren-17-yl)ethan-1-one (1.3 g, yield: 62%).

$^1$H NMR (400 MHz, $CDCl_3$) δ 3.22 - 3.15(m, 1H), 2.51 - 2.46(m, 1H), 2.11(s, 3H), 2.05 - 1.03(m,22H), 0.98 - 0.95(m, 6H), 0.77 (s, 3H).

**Step** 7: **Preparation** of **(2S,3S,5R,8R,9R,10S,13S,14S,15R,17S)-17-acetyl-2,13,15-trimethylhexadecahydro-1H-cyclopenta[a]phenanthren-3-yl acetate**

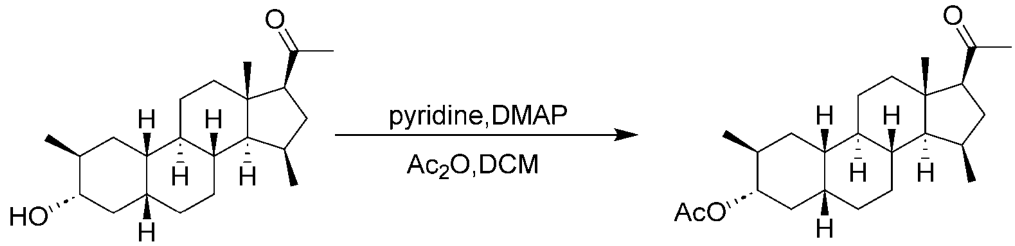


1-((2S,3S,5R,8R,9R,10S,13S,14S,15R,17S)-3-Hydroxy-2,13,15-trimethylhexadecahydro-1H-cyclopenta[a]phenanthren-17-yl)ethan-1-one (300 mg, 0.9 mmol) was dissolved in dichloromethane (20 mL), and DMAP (12 mg, 1.4 mmol), pyridine (1.2 mL), and acetic anhydride (0.8 mL) were added. The reaction system was stirred at room temperature for 2 h. The reaction mixture was washed with water and then dried over anhydrous sodium sulfate, and the organic phase was filtered and concentrated to give a crude product of (2S,3S,5R,8R,9R,10S,13S,14S,15R,17S)-17-acetyl-2,13,15-trimethylhexadecahydro-1H-cyclopenta[a]phenanthren-3-yl acetate (338 mg), which was directly used in the next step.

**Step 8: Preparation of (2S,3S,5R,8R,9R,10S,13S,14S,15R,17S)-17-(2-bromoacetyl)-2,13,15-trimethylhexadecahydro-1H-cyclopenta[a]phenanthren-3-yl acetate**

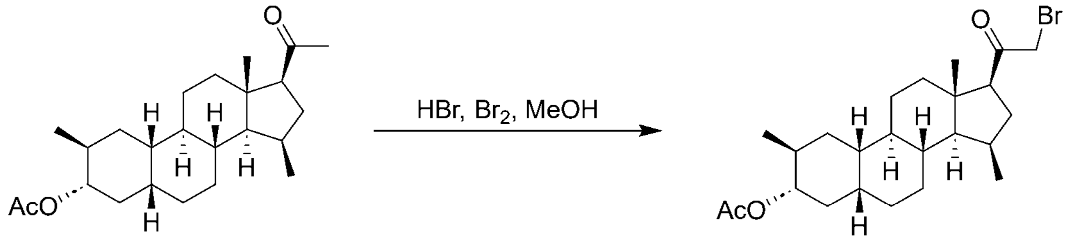


(2S,3S,5R,8R,9R,10S,13S,14S,15R,17S)-17-Acetyl-2,13,15-trimethylhexadecahydro-1H-cyclopenta[a]phenanthren-3-yl acetate (338 mg, 0.9 mmol) was dissolved in methanol (5.0 mL), and a drop of hydrogen bromide was added, followed by the addition of liquid bromine (0.15 mL). The reaction system was stirred at room temperature for 2 h. A

saturated aqueous sodium sulfite solution was added to quench the reaction, and the mixture was extracted with ethyl acetate. The organic phase was then dried over anhydrous sodium sulfate and concentrated to give a crude product of (2S,3S,5R,8R,9R,10S,13S,14S,15R,17S)-17-(2-bromoacetyl)-2,13,15-trimethylhexadecahydro-1H-cyclopenta[a]phenanthren-3-yl acetate (408 mg), which was directly used in the next step.

**Step 9: Preparation of (2S,3S,5R,8R,9R,10S,13S,14S,15R,17S)-17-(2-(4-cyano-1H-pyrazol-1-yl)acetyl)-2,13,15-trimethylhexadecahydro-1H-cyclopenta[a]phenanthren-3-yl acetate**

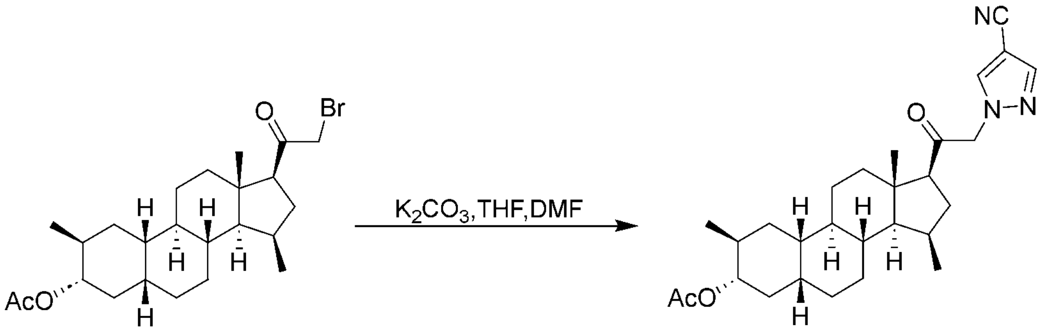


(2S,3S,5R,8R,9R,10S,13S,14S,15R,17S)-17-(2-Bromoacetyl)-2,13,15-trimethylhexadecahydro-1H-cyclopenta[a]phenanthren-3-yl acetate (408 mg, 0.9 mmol), 1H-pyrazole-4-carbonitrile (120.0 mg, 1.3 mmol), and potassium carbonate (181.3 mg, 1.3 mmol) were dissolved in anhydrous tetrahydrofuran (15.0 mL) and N,N-dimethylformamide (3.0 mL), and the reaction system was stirred at room temperature overnight. The reaction mixture was then filtered, diluted with ethyl acetate, washed sequentially with water and saturated brine, dried over anhydrous sodium sulfate, and concentrated to give a crude product of (2S,3S,5R,8R,9R,10S,13S,14S,15R,17S)-17-(2-(4-cyano-1H-pyrazol-1-yl)acetyl)-2,13,15-trimethylhexadecahydro-1H-cyclopenta[a]phenanthren-3-yl acetate (418 mg), which was directly used in the next step.

**Step 10: Preparation of 1-(2-((2S,3S,5R,8R,9R,10S,13S,14S,15R,17S)-3-hydroxy-2,13,15-trimethylhexadecahydro-1H-cyclopenta[a]phenanthren-17-yl)-2-oxoethyl)-1H-pyrazole-4-carbonitrile**

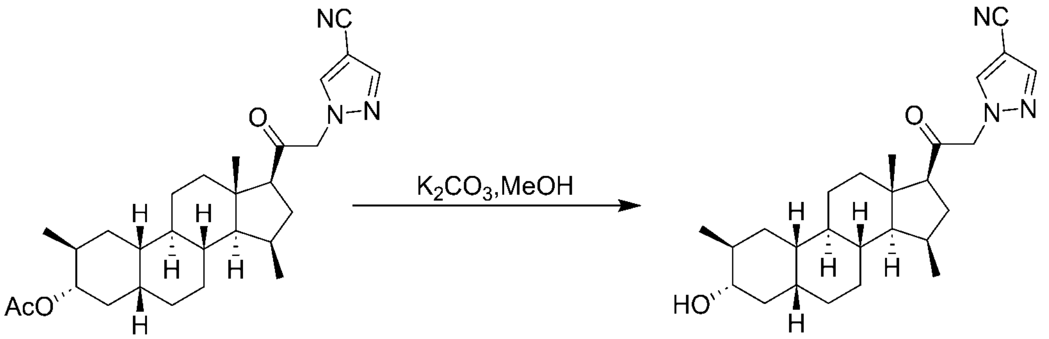


The crude product of (2S,3S,5R,8R,9R,10S,13S,14S,15R,17S)-17-(2-(4-cyano-1H-pyrazol-1-yl)acetyl)-2,13,15-trimethylhexadecahydro-1H-cyclopenta[a]phenanthren-3-yl acetate (418 mg, 0.9 mmol) and potassium carbonate (373 mg, 2.7 mmol) were dissolved in methanol (10.0 mL), and the reaction system was stirred at room temperature for 3 h. The reaction mixture was then filtered, and the filtrate was concentrated. The crude product was separated by a high-performance liquid chromatography column to give 1-(2-((2S,3S,5R,8R,9R,10S,13S,14S,15R,17S)-3-hydroxy-2,13,15-trimethylhexadecahydro-1H-cyclopenta[a]phenanthren-17-yl)-2-oxoethyl)-1H-pyrazole-4-carbonitrile (160 mg, yield: 42.0%).

$^1H$ NMR (400 MHz, $CDCl_3$) δ 7.86 (s, 1H), 7.81 (s, 1H), 5.03 - 4.88 (m, 2H), 3.22 - 3.16 (m, 1H), 2.58 - 2.53 (m, 1H), 2.26 - 2.11 (m, 2H), 2.00 - 1.80(m, 4H), 1.72 - 1.01 (m, 16H), 0.99- 0.97 (m, 6H), 0.83 (s, 3H).

$^{13}C$ NMR (101 MHz, $CDCl_3$) δ 202.02, 142.40, 136.05, 113.25, 93.16, 77.15, 61.70, 61.40, 57.40, 45.54, 41.27,

40.98, 39.80, 39.47, 36.20, 36.04, 35.39, 33.65, 33.53, 31.28, 30.26, 26.15, 25.10, 18.57, 18.25, 16.81.
MS m/z (ESI): 406 $[M+H-18]^+$.

**Example 15**

**1-((2S,3S,5R,8R,9R,10S,13S,14S,15R,17S)-3-Hydroxy-2,13,15-trimethylhexadecahydro-1H-cyclopenta[a]phenanthren-17-yl)-2-(2H-1,2,3-triazol-2-yl)ethan-1-one**

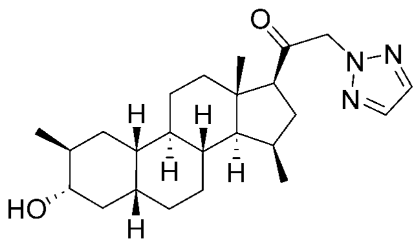

**Example 16**

**1-((2S,3S,5R,8R,9R,10S,13S,14S,15R,17S)-3-Hydroxy-2,13,15-trimethylhexadecahydro-1H-cyclopenta[a]phenanthren-17-yl)-2-(1H-1,2,3-triazol-1-yl)ethan-1-one**

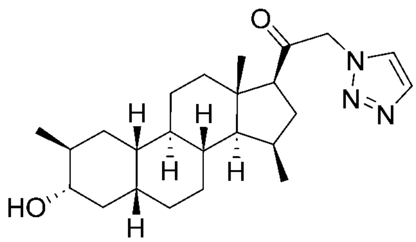

The synthesis method was the same as that in Example 14, except that the 1H-pyrazole-4-carbonitrile in step 9 was replaced by **1H-1,2,3-triazole,** and finally column chromatography separation was performed to give product 1 (compound 15) and product 2 (compound 16).

Compound 15:

$^1$H NMR (400 MHz, $CDCl_3$) δ 7.68 (s, 2H), 5.23 (s, 2H), 3.22 - 3.16 (m, 1H), 2.55 - 2.50 (m, 1H), 2.24 - 2.09 (m, 2H), 2.02- 1.98 (m, 1H), 1.90- 1.80(m, 3H), 1.72 - 1.01 (m, 16H), 0.98- 0.96 (m, 6H), 0.87 (s, 3H).
$^{13}$C NMR (101 MHz, $CDCl_3$) δ 202.63, 135.04, 77.20, 63.87, 60.90, 57.35, 45.36, 41.15, 41.00, 39.83, 39.48, 36.23, 36.08, 35.41, 33.66, 33.63, 31.31, 30.27, 26.18, 25.10, 18.58, 18.26, 16.72.
MS m/z (ESI): 382 $[M+H-18]^+$.

Compound 16:

$^1$H NMR (400 MHz, $CDCl_3$) δ 7.76 (s, 1H), 7.65 (s, 1H), 5.28 - 5.12 (m, 2H), 3.23 - 3.16 (m, 1H), 2.62 - 2.58 (m, 1H), 2.27 - 2.12 (m, 2H), 2.04- 1.98 (m, 1H), 1.92- 1.80(m, 3H), 1.73 - 1.01 (m, 16H), 0.99- 0.97 (m, 6H), 0.84 (s, 3H).
$^{13}$C NMR (101 MHz, $CDCl_3$) δ 201.66, 133.99, 125.10, 77.18, 61.48, 58.94, 57.40, 45.55, 41.26, 40.99, 39.81, 39.49, 36.22, 36.06, 35.39, 33.67, 33.57, 31.30, 30.28, 26.16, 25.10, 18.57, 18.26, 16.82.
MS m/z (ESI): 400 $[M+H]^+$.

**Example 17**

**1-((2S,3S,5R,8R,9R,10S,13S,14S,15R,17S)-3-Hydroxy-2,13,15-trimethylhexadecahydro-1H-cyclopenta[a]phenanthren-17-yl)-2-(2H-tetrazol-2-yl)ethan-1-one**

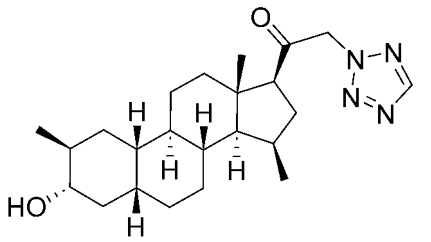

**Example 18**

**1-((2S,3S,5R,8R,9R,10S,13S,14S,15R,17S)-3-Hydroxy-2,13,15-trimethylhexadecahydro-1H-cyclopenta[a]phenanthren-17-yl)-2-(1H-tetrazol-1-yl)ethan-1-one**

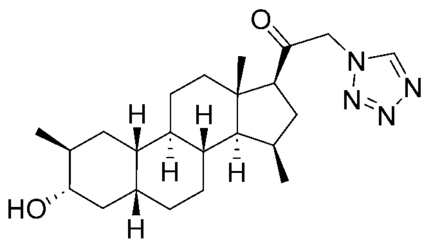

The synthesis method was the same as that in Example 14, except that the 1H-pyrazole-4-carbonitrile in step 9 was replaced by **1H-tetrazole,** and finally column chromatography separation was performed to give product 1 (compound 17) and product 2 (compound 18).

Compound 17:

$^1$H NMR (400 MHz, $CDCl_3$) δ 8.57 (s, 1H), 5.45 (s, 2H), 3.23 - 3.16 (m, 1H), 2.62 - 2.57 (m, 1H), 2.27 - 2.14 (m, 2H), 2.04- 1.99 (m, 1H), 1.92- 1.81(m, 3H), 1.73 - 1.01 (m, 16H), 0.99- 0.97 (m, 6H), 0.88 (s, 3H).
$^{13}$C NMR (101 MHz, $CDCl_3$) δ 200.06, 153.23, 77.16, 61.42, 61.33, 57.39, 45.60, 41.19, 40.98, 39.80, 39.48, 36.21, 36.05, 35.39, 33.66, 33.54, 31.28, 30.28, 26.16, 25.09, 18.56, 18.23, 16.74.
MS m/z (ESI): 401 $[M+H]^+$.

Compound 18:

$^1$H NMR (400 MHz, $CDCl_3$) δ 8.75 (s, 1H), 5.33 - 5.17 (m, 2H), 3.23 - 3.17 (m, 1H), 2.65 - 2.60 (m, 1H), 2.29 - 2.16 (m, 2H), 2.03- 1.81(m, 4H), 1.74 - 1.06 (m, 16H), 1.01 - 0.97 (m, 6H), 0.84 (s, 3H).
$^{13}$C NMR (101 MHz, $CDCl_3$) δ 200.06, 143.63, 77.14, 61.69, 57.43, 56.75, 45.73, 41.30, 40.96, 39.79, 39.47, 36.18, 36.01, 35.36, 33.65, 33.53, 31.26, 30.29, 26.14, 25.07, 18.56, 18.24, 16.85.
MS m/z (ESI): 401 $[M+H]^+$.

**Example 19**

**1-(2-((2S,3S,5R,8R,9R,10S,13S,14S,17S)-3-Hydroxy-2,3,13-trimethylhexadecahydro-1H-cyclopenta[a]phenanthren-17-yl)-2-oxoethyl)-1H-pyrazole-4-carbonitrile**

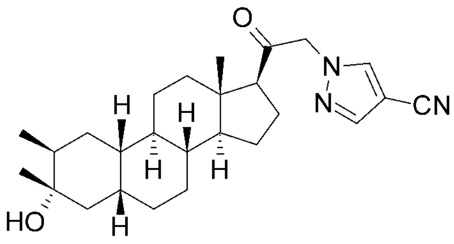

Synthesis scheme

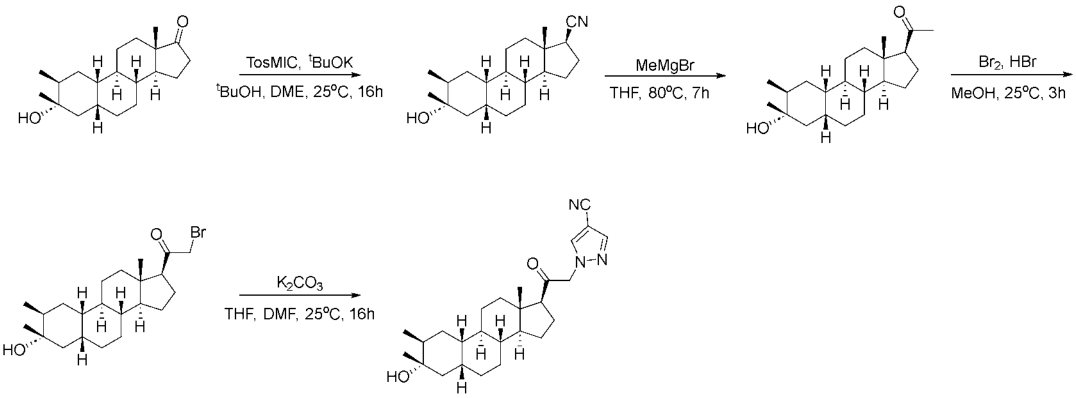


**Step 1: Preparation of (2S,3S,5R,8R,9R,10S,13S,14S,17S)-3-hydroxy-2,3,13-trimethylhexadecahydro-1H-cyclopenta[a]phenanthrene-17-carbonitrile**

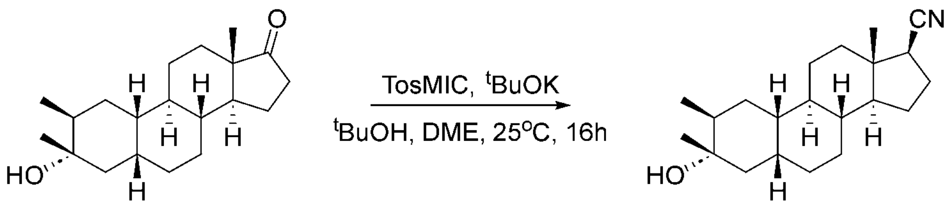


To a dry 100 mL round-bottom flask were added potassium tert-butoxide (4.06 g, 36.2 mmol) and tert-butanol (24.0 mL). The system was purged with nitrogen. (2S,3S,5R,8R,9R,10S,13S,14S)-3-Hydroxy-2,3,13-trimethylhexadecahydro-17H-cyclopenta[a]phenanthren-17-one (1.1 g, 3.62 mmol) was dissolved in 15 mL of ethylene glycol dimethyl ether, and the resulting solution was slowly added dropwise to the reaction system. The reaction system was then stirred for 30 min. p-Toluenesulfonylmethyl isocyanide (1.41 g, 7.24 mmol) was dissolved in 15 mL of ethylene glycol dimethyl ether, and the resulting solution was slowly added dropwise to the reaction system. The reaction system was then stirred at room temperature overnight. After the reaction was completed as detected by TLC, water (50 mL) was added to quench the reaction, and the aqueous phase was extracted with ethyl acetate (50 mL × 2). The organic phases were combined, dried over anhydrous sodium sulfate, filtered, and concentrated to give a crude product, which was then separated and purified by column chromatography (petroleum ether/ethyl acetate: 50/1-3/1) to give (2S,3S,5R,8R,9R,10S,13S,14S,17S)-3-hydroxy-2,3,13-trimethylhexadecahydro-1H-cyclopenta[a]phenanthrene-17-carbonitrile (0.65 g, yield: 57.1%).

**Step 2: preparation of 1-((2S,3S,5R,8R,9R,10S,13S,14S,17S)-3-hydroxy-2,3,13-trimethylhexadecahydro-1H-cyclopenta[a]phenanthren-17-yl)ethan-1-one**

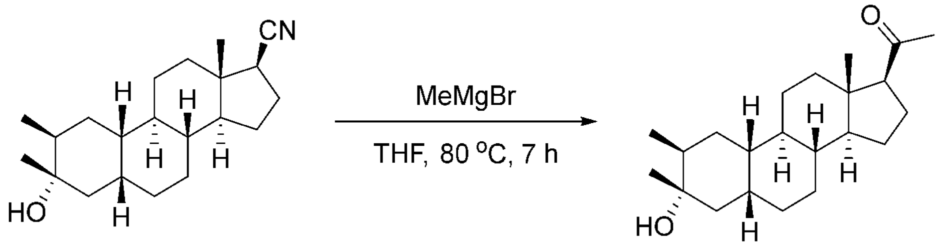


(2S,3S,5R,8R,9R,10S,13S,14S,17S)-3-Hydroxy-2,3,13-trimethylhexadecahydro-1H-cyclopenta[a]phenanthrene-17-carbonitrile (650 mg, 2.06 mmol) was dissolved in a solution of dry tetrahydrofuran (75 mL). The system was purged with nitrogen, and 3.0 M methylmagnesium bromide (20 mL, 61.8 mmol) was slowly added dropwise. The reaction system was then stirred at 80 °C for 7 h. After the reaction was completed as detected by TLC, the reaction was quenched with saturated ammonium chloride, and the mixture was extracted with ethyl acetate. The organic phase was washed with water and saturated brine, dried over anhydrous sodium sulfate, filtered, and concentrated to dryness by rotary evaporation, and the crude product was separated by column chromatography (elution with petroleum ether:ethyl acetate = 3:1) to give 1-((2S,3S,5R,8R,9R,10S,13S,14S,17S)-3-hydroxy-2,3,13-trimethylhexadecahydro-1H-cyclopenta[a]phenanthren-17-yl)ethan-1-one (520 mg, yield: 76.0%).

**Step 3: Preparation of 2-bromo-1-((2S,3S,5R,8R,9R,108,13S,14S,17S)-3-hydroxy-2,3,13-trimethylhexadecahydro-1H-cyclopenta[a]phenanthren-17-yl)ethan-1-one**

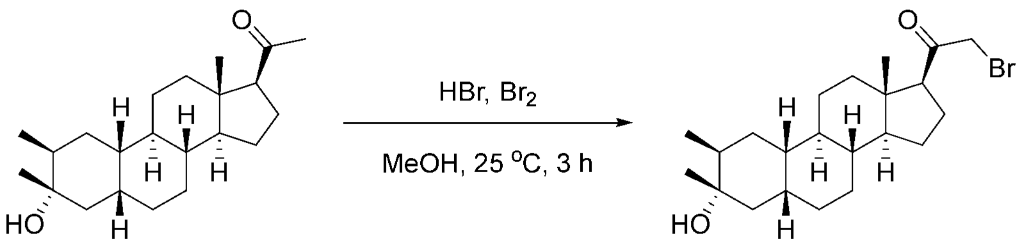


1-((2S,3S,5R,8R,9R,10S,13S,14S,17S)-3-Hydroxy-2,3,13-trimethylhexadecahydro-1H-cyclopenta[a]phenanthren-17-yl)ethan-1-one (520 mg, 1.5 mmol) was dissolved in methanol (20 mL), and a drop of hydrogen bromide was added, followed by the addition of liquid bromine (230 mg, 1.65 mmol). The reaction system was stirred at room temperature for 3 h. Water (50.0 mL) was added, and the aqueous phase was extracted with ethyl acetate (50.0 mL × 2). The organic phases were combined, dried over anhydrous sodium sulfate, filtered, and concentrated to give a crude product (520 mg, crude product), which was directly used in the next step.

**Step 4: Preparation of 1-(2-((2S,3S,5R,8R,9R,10S,13S,14S,17S)-3-hydroxy-2,3,13-trimethylhexadecahydro-1H-cyclopenta[a]phenanthren-17-yl)-2-oxoethyl)-1H-pyrazole-4-carbonitrile**

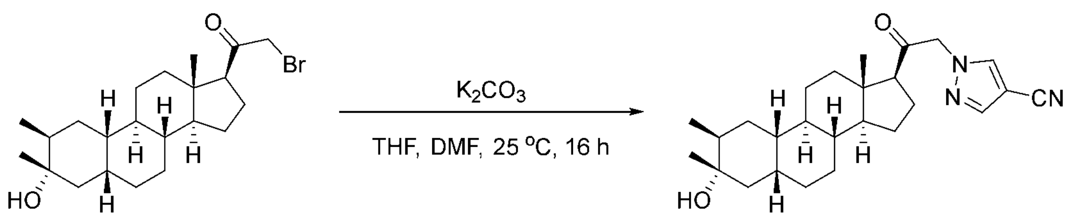


2-Bromo-1-((2S,3S,5R,8R,9R,10S,13S,14S,17S)-3-hydroxy-2,3,13-trimethylhexadecahydro-1H-cyclopenta[a]phenanthren-17-yl)ethan-1-one (220 mg, 0.54 mmol), 1H-pyrazole-4-carbonitrile (74.7 mg, 0.81 mmol), and potassium carbonate (113 mg, 0.81 mmol) were dissolved in anhydrous tetrahydrofuran (7.00 mL) and N,N-dimethylformamide (1.40 mL), and the reaction system was stirred at room temperature overnight. The reaction mixture was then filtered, and the filtrate was concentrated. The crude product was separated by a high-performance liquid chromatography column to give 1-(2-((2S,3S,5R,8R,9R,10S,13S,14S,17S)-3-hydroxy-2,3,13-trimethylhexadecahydro-1H-cyclopenta[a]phenanthren-17-yl)-2-oxoethyl)-1H-pyrazole-4-carbonitrile (120 mg, yield: 52.6%).

$^1$H NMR (400 MHz, $CDCl_3$) δ 7.86 (s, 1H), 7.81 (s, 1H), 5.06 - 4.79 (m, 2H), 2.60 (t, *J* = 8.8 Hz, 1H), 2.23 - 2.16 (m, 1H), 2.06 - 2.00 (m, 1H), 1.82 (d, *J* = 7.5 Hz, 2H), 1.78 - 1.68 (m, 4H), 1.65 - 1.53 (m, 6H), 1.52 - 1.37 (m, 6H), 1.35 - 1.18 (m, 6H), 0.87 (d, *J* = 6.7 Hz, 3H), 0.67 (s, 3H).

$^{13}$C NMR (101 MHz, $CDCl_3$) δ 202.31, 142.58, 136.23, 113.42, 93.32, 74.34, 61.84, 61.31, 56.11, 45.63, 43.10, 41.86, 41.21, 39.37, 38.73, 36.15, 35.14, 34.21, 31.40, 26.26, 25.95, 24.51, 23.28, 20.64, 15.33, 14.00.

MS m/z (ESI): 422.1 [M-H]$^-$.

**Example 20**

**1-((2S,3S,5R,8R,9R,10S,13S,14S,17S)-3-Hydroxy-2,3,13-trimethylhexadecahydro-1H-cyclopenta[a]phenanthren-17-yl)-2-(2H-tetrazol-2-yl)ethan-1-one**

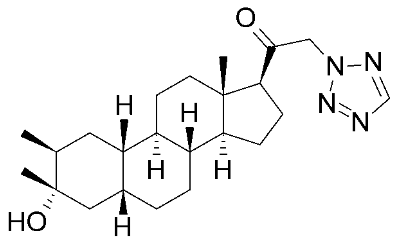

**Example 21**

**1-((2S,3S,5R,8R,9R,10S,13S,14S,17S)-3-Hydroxy-2,3,13-trimethylhexadecahydro-1H-cyclopenta[a]phenanthren-17-yl)-2-(1H-tetrazol-1-yl)ethan-1-one**

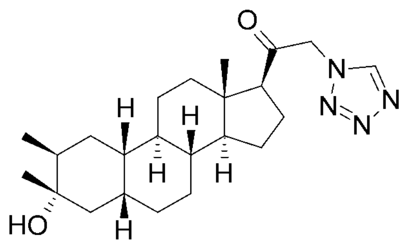

Synthesis scheme

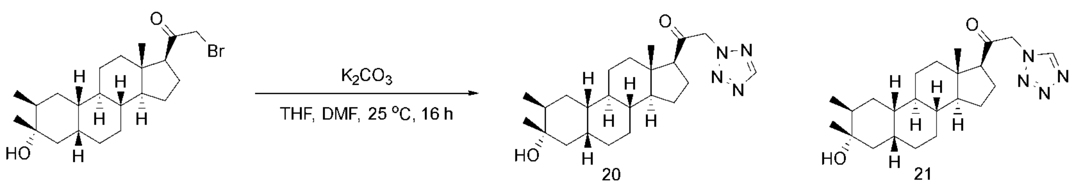

20
21

The synthesis method was the same as that in Example 19, except that the 1H-pyrazole-4-carbonitrile in step 4 was replaced by **1H-tetrazole,** and finally column chromatography separation was performed to give product 1 (compound 20) and product 2 (compound 21).

Product 1 (compound 20):

$^1$H NMR (400 MHz, $CDCl_3$) δ 8.57 (s, 1H), 5.45 (s, 2H), 2.64 (t, *J* = 8.9 Hz, 1H), 2.28 - 2.14 (m, 1H), 2.08 (dt, *J* =

12.1, 3.4 Hz, 1H), 1.91 - 1.68 (m, 8H), 1.63 - 1.38 (m, 10H), 1.33 - 1.24 (m, 6H), 0.87 (d, *J* = 6.7 Hz, 3H), 0.71 (s, 3H).

$^{13}$C NMR (101 MHz, $CDCl_3$) δ 200.38, 153.39, 74.34, 61.56, 61.25, 56.08, 45.69, 43.09, 41.85, 41.20, 39.27, 38.72, 36.15, 35.13, 34.20, 31.39, 26.25, 25.93, 24.50, 23.30, 20.61, 15.31, 13.92.

MS m/z (ESI): 399.1 $[M-H]^-$.

Product 2 (compound 21):

$^1$H NMR (400 MHz, DMSO-$d_6$) δ 9.31 (s, 1H), 5.69 - 5.44 (m, 2H), 4.06 (s, 1H), 2.81 (t, *J* = 8.9 Hz, 1H), 2.12 - 2.01 (m, 2H), 1.81 - 1.50 (m, 8H), 1.49 - 1.28 (m, 7H), 1.27 - 1.11 (m, 4H), 1.11 - 0.97 (m, 4H), 0.78 (d, *J* = 6.7 Hz, 3H), 0.60 (s, 3H).

$^{13}$C NMR (101 MHz, $CDCl_3$) δ 202.45, 145.21, 72.00, 59.92, 56.81, 55.25, 44.63, 42.97, 41.31, 40.78, 38.23, 37.99, 35.43, 34.43, 33.69, 31.08, 25.95, 25.37, 23.97, 22.57, 20.11, 15.40, 13.38.

MS m/z (ESI): 399.1 $[M-H]^-$.

**Example 22**

**1-((2S,3S,5R,8R,9R,10S,13S,14S,17S)-3-Hydroxy-2,3,13-trimethylhexadecahydro-1H-cyclopenta[a]phenanthren-17-yl)-2-(1H-1,2,4-triazol-1-yl)ethan-1-one**

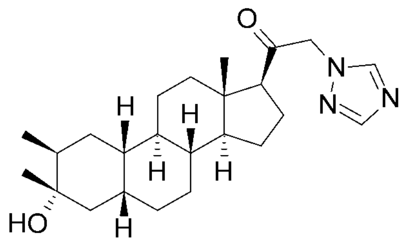

Synthesis scheme

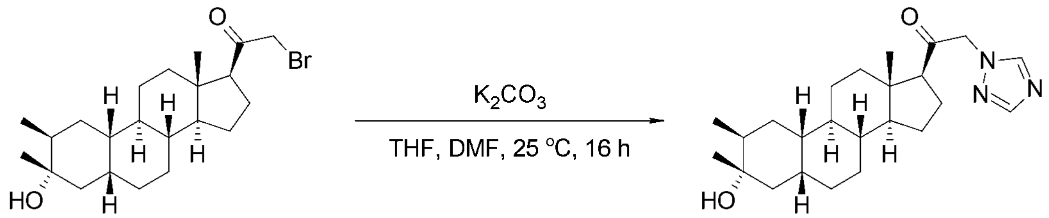


The synthesis method was the same as that in Example 19, except that the 1H-pyrazole-4-carbonitrile in step 4 was replaced by **1H-1,2,4-triazole.**

$^1$H NMR (400 MHz, $CDCl_3$) δ 8.14 (s, 1H), 7.95 (s, 1H), 5.04 - 4.91 (m, 2H), 2.62 (t, *J* = 8.6 Hz, 1H), 2.20 (q, *J* = 10.8, 10.0 Hz, 1H), 2.09 - 2.00 (m, 1H), 1.82-1.62 (m, 8H), 1.55 - 1.35 (m, 6H), 1.34 - 1.19 (m, 10H), 0.87 (d, *J* = 6.7 Hz, 3H), 0.68 (s, 3H).

$^{13}$C NMR (101 MHz, $CDCl_3$) δ 202.26, 152.00, 74.31, 61.27, 58.89, 56.07, 45.55, 43.07, 41.83, 41.18, 39.31, 38.70, 36.13, 35.11, 34.18, 31.37, 29.88, 26.23, 25.92, 24.48, 23.27, 20.58, 15.30, 13.95.

MS m/z (ESI): 400.2 $[M+H]^+$.

**Example 23**

**1-((2S,3S,5R,8R,9R,10S,13S,14S,17S)-3-Hydroxy-2,3,13-trimethylhexadecahydro-1H-cyclopenta[a]phenanthren-17-yl)-2-(5-methyl-2H-tetrazol-2-yl)ethan-1-one**

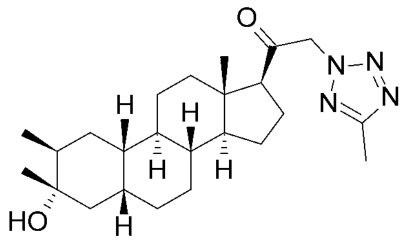

**Example 24**

**1-((2S,3S,5R,8R,9R,10S,13S,14S,17S)-3-Hydroxy-2,3,13-trimethylhexadecahydro-1H-cyclopenta[a]phenanthren-17-yl)-2-(5-methyl-1H-tetrazol-1-yl)ethan-1-one**

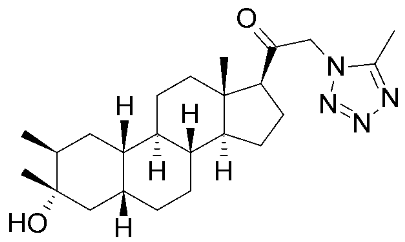

Synthesis scheme

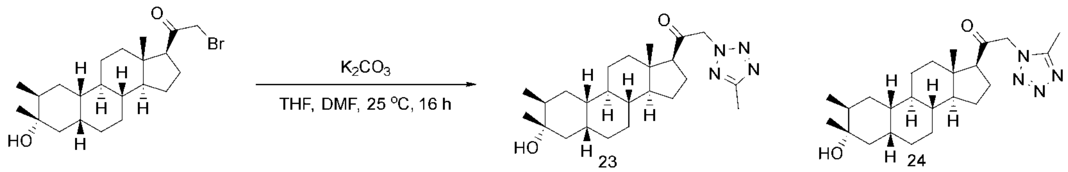


The synthesis method was the same as that in Example 19, except that the 1H-pyrazole-4-carbonitrile in step 4 was replaced by **5-methyl-1H-tetrazole,** and finally column chromatography separation was performed to give product 1 (compound 23) and product 2 (compound 24).

Compound 23:

$^1$H NMR (400 MHz, $CDCl_3$) δ 5.35 (s, 2H), 2.62 (t, *J* = 8.7 Hz, 1H), 2.56 (s, 3H), 2.28 - 2.14 (m, 1H), 2.07 (dt, *J* = 12.2, 3.4 Hz, 1H), 1.83 - 1.74 (m, 7H), 1.59 - 1.40 (m, 10H), 1.34 - 1.26 (m, 7H),0.88 (d, *J* = 6.6 Hz, 3H), 0.71 (s, 3H).
$^{13}$C NMR (101 MHz, $CDCl_3$) δ 200.69, 163.55, 74.30, 61.41, 61.18, 56.07, 45.61, 43.09, 41.84, 41.20, 39.25, 38.70, 36.14, 35.13, 34.18, 31.38, 26.24, 25.92, 24.49, 23.30, 20.61, 15.30, 13.90, 11.11.
MS m/z (ESI): 413.1 $[M-H]^-$.

Compound 24:

$^1$H NMR (400 MHz, $CDCl_3$) δ 5.10 (q, *J* = 18.1 Hz, 2H), 2.66 (t, *J* = 8.7 Hz, 1H), 2.47 (s, 3H), 2.24 - 2.19 (m, 1H), 2.07 (t, *J* = 12.0 Hz, 1H), 1.84 - 1.72 (m, 7H), 1.63 - 1.41 (m, 10H), 1.37 - 1.26 (m, 7H), 0.88 (d, *J* = 6.7 Hz, 3H), 0.68 (s, 3H).
$^{13}$C NMR (101 MHz, $CDCl_3$) δ 200.47, 152.92, 74.29, 61.31, 56.18, 56.12, 45.77, 43.06, 41.83, 41.17, 39.42, 38.69, 36.12, 35.10, 34.17, 31.35, 26.22, 25.93, 24.48, 23.26, 20.63, 15.30, 14.02, 9.08.
MS m/z (ESI): 415.1 $[M+H]^+$.

**Example 25**

**1-(2-((2S,3S,5R,8R,9R,10S,13S,14S,17S)-3-Hydroxy-2,13-dimethylhexadecahydro-1H-cyclopenta[a]phenanthren-17-yl)-2-oxoethyl)-1H-pyrazole-4-carbonitrile**

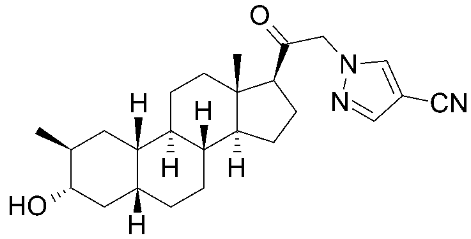

Synthesis scheme

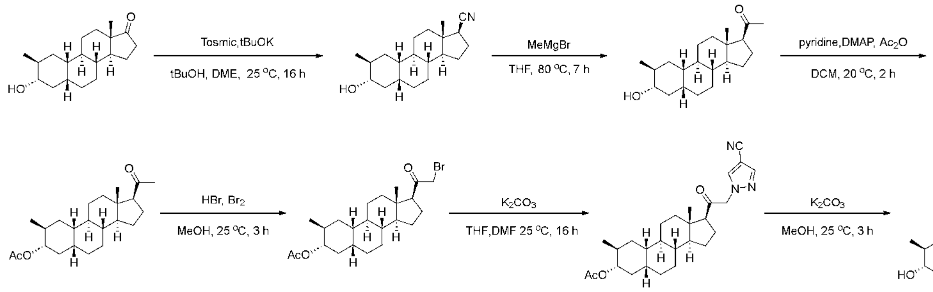


**Step 1: Preparation of (2S,3S,5R,8R,9R,10S,13S,14S,17S)-3-hydroxy-2,13-dimethylhexadecahydro-1H-cyclopenta[a]phenanthrene-17-carbonitrile**

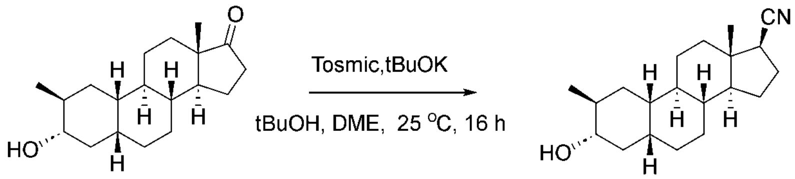


To a dry 100 mL round-bottom flask were added potassium tert-butoxide (3.48 g, 34.4 mmol) and tert-butanol (20.0 mL). The system was purged with nitrogen. (2S,3S,5R,8R,9R,10S,13S,14S)-3-Hydroxy-2,13-dimethylhexadecahydro-17H-cyclopenta[a]phenanthren-17-one (1.00 g, 3.44 mmol) was dissolved in 10 mL of ethylene glycol dimethyl ether, and the resulting solution was slowly added dropwise to the reaction system. The reaction system was then stirred for 30 min. p-Toluenesulfonylmethyl isocyanide (1.21 g, 6.88 mmol) was dissolved in 10 mL of ethylene glycol dimethyl ether, and the resulting solution was slowly added dropwise to the reaction system. The reaction system was then stirred at room temperature overnight. After the reaction was completed as detected by TLC, water (50 mL) was added to quench the reaction, and the aqueous phase was extracted with ethyl acetate (50 mL × 2). The organic phases were combined, dried over anhydrous sodium sulfate, filtered, and concentrated to give a crude product, which was then separated and purified by column chromatography (petroleum ether/ethyl acetate: 50/1-3/1) to give (2S,3S,5R,8R,9R,10S,13S,14S,17S)-3-hydroxy-2,13-dimethylhexadecahydro-1H-cyclopenta[a]phenanthrene-17-carbonitrile (0.6 g, yield: 57.8%).

**Step 2: Preparation of 1-((2S,3S,5R,8R,9R,10S,13S,14S,17S)-3-hydroxy-2,13-dimethylhexadecahydro-1H-cyclopenta[a]phenanthren-17-yl)ethan-1-one**

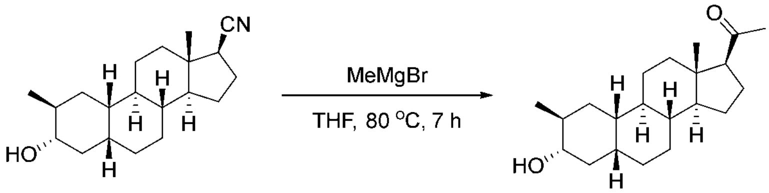


(2S,3S,5R,8R,9R,10S,13S,14S,17S)-3-Hydroxy-2,13-dimethylhexadecahydro-1H-cyclopenta[a]phenanthrene-17-carbonitrile (1.10 g, 3.65 mmol) was dissolved in a solution of dry tetrahydrofuran (124 mL). The system was purged with nitrogen, and 3.0 M methylmagnesium bromide (36.6 mL, 11.0 mmol) was slowly added dropwise. The reaction system was then stirred at 80 °C for 7 h. After the reaction was completed as detected by TLC, the reaction was quenched with saturated ammonium chloride, and the mixture was extracted with ethyl acetate. The organic phase was washed with water and saturated brine, dried over anhydrous sodium sulfate, filtered, and concentrated to dryness by rotary evaporation, and the crude product was separated by column chromatography (elution with petroleum ether:ethyl acetate = 4:1) to give 1-((2S,3S,5R,8R,9R,10S,13S,14S,17S)-3-hydroxy-2,13-dimethylhexadecahydro-1H-cyclopenta [a]phenanthren-17-yl)ethan-1-one (600 mg, yield: 51.8%).

**Step 3: Preparation of (2S,3S,5R,8R,9R,10S,13S,14S,17S)-17-acetyl-2,13-dimethylhexadecahydro-1H-cyclopenta[a]phenanthren-3-yl acetate**

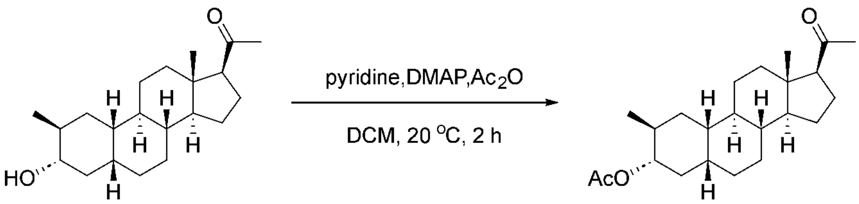


1-((2S,3S,5R,8R,9R,10S,13S,14S,17S)-3-Hydroxy-2,13-dimethylhexadecahydro-1H-cyclopenta[a]phenanthren-17-yl)ethan-1-one (500 mg, 1.57 mmol) was dissolved in dichloromethane (25.0 mL), and DMAP (20.0 mg, 2.36 mmol), pyridine (2.00 mL), and acetic anhydride (1.25 mL) were added. The reaction system was stirred at room temperature for 2 h. The reaction mixture was then filtered, and the organic phase was concentrated to give a crude product of (2S,3S,5R,8R,9R,10S,13S,14S,17S)-17-acetyl-2,13-dimethylhexadecahydro-1H-cyclopenta[a]phenanthren-3-yl acetate (566 mg, crude product).

**Step 4: Preparation of (2S,3S,5R,8R,9R,10S,13S,14S,17S)-17-(2-bromoacetyl)-2,13-dimethylhexadecahydro-1H-cyclopenta[a]phenanthren-3-yl acetate**

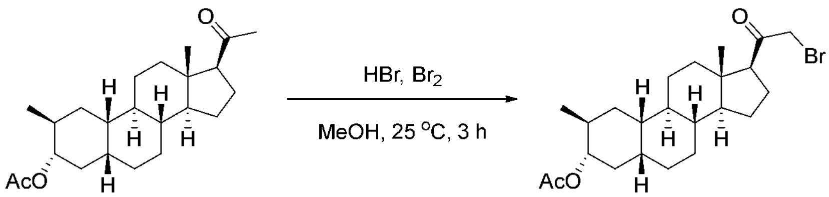


(2S,3S,5R,8R,9R,10S,13S,14S,17S)-17-Acetyl-2,13-dimethylhexadecahydro-1H-cyclopenta[a]phenanthren-3-yl acetate (566 mg, 1.57 mmol) was dissolved in methanol (6.00 mL), and a drop of hydrogen bromide was added, followed by the addition of liquid bromine (0.25 mL, 4.88 mmol). The reaction system was stirred at room temperature for 3 h. Water (20.0 mL) was added, and the aqueous phase was extracted with ethyl acetate (20.0 mL × 2). The organic phases were combined, dried over anhydrous sodium sulfate, filtered, and concentrated to give a crude product (680 mg, crude product), which was directly used in the next step.

**Step 5: Preparation of (2S,3S,5R,8R,9R,10S,13S,14S,17S)-17-(2-(4-cyano-1H-pyrazol-1-yl)acetyl)-2,13-dimethylhexadecahydro-1H-cyclopenta[a]phenanthren-3-yl acetate**

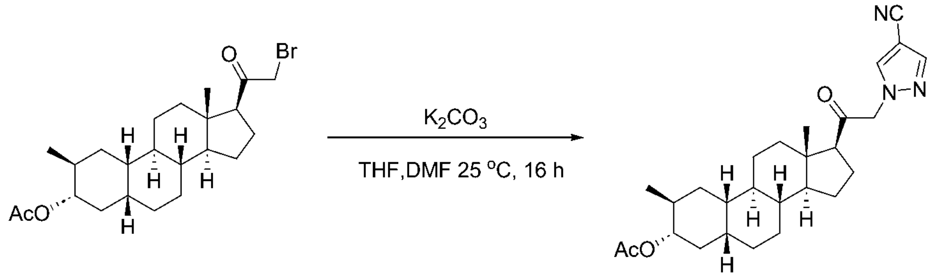


(2S,3S,5R,8R,9R,10S,13S,14S,17S)-17-(2-Bromoacetyl)-2,13-dimethylhexadecahydro-1H-cyclopenta[a]phenanthren-3-yl acetate (680 mg, crude product), 1H-pyrazole-4-carbonitrile (480.0 mg, 5.20 mmol), and potassium carbonate (725 mg, 5.20 mmol) were dissolved in anhydrous tetrahydrofuran (10.0 mL), and the reaction system was stirred at room temperature overnight. The reaction mixture was then filtered, and the filtrate was concentrated to give (2S,3S,5R,8R,9R,10S,13S,14S,17S)-17-(2-(4-cyano-1H-pyrazol-1-yl)acetyl)-2,13-dimethylhexadecahydro-1H-cyclopenta[a]phenanthren-3-yl acetate (600 mg, crude product).

**Step 6: Preparation of 1-(2-((2S,3S,5R,8R,9R,10S,13S,14S,17S)-3-hydroxy-2,13-dimethylhexadecahydro-1H-cyclopenta[a]phenanthren-17-yl)-2-oxoethyl)-1H-pyrazole-4-carbonitrile**

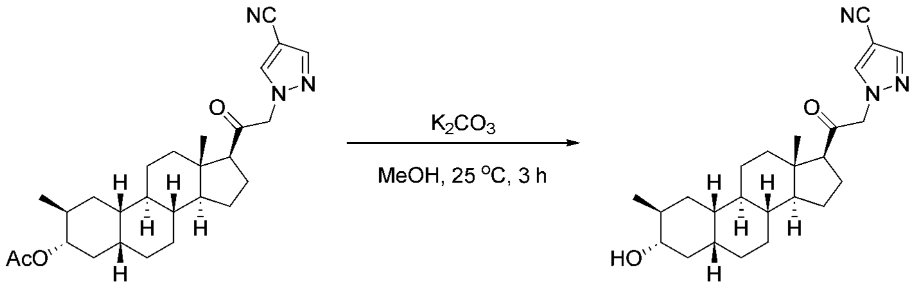


(2S,35,5R,8R,9R,10S,13S,14S,17S)-17-(2-(4-Cyano-1H-pyrazol-1-yl)acetyl)-2,13-dimethylhexadecahydro-1H-cyclopenta[a]phenanthren-3-yl acetate (600 mg, crude product) and potassium carbonate (725 mg, 5.20 mmol) were dissolved in methanol (10.00 mL), and the reaction system was stirred at room temperature for 3 h. The reaction mixture was then filtered, and the filtrate was concentrated. The crude product was separated by a high-performance liquid chromatography column to give 1-(2-((2S,3S,5R,8R,9R,10S,13S,14S,17S)-3-hydroxy-2,13-dimethylhexadecahydro-1H-cyclopenta[a]phenanthren-17-yl)-2-oxoethyl)-1H-pyrazole-4-carbonitrile (172 mg, yield: 31.6%).

$^1H$ NMR (400 MHz, $CDCl_3$) δ 7.86 (s, 1H), 7.81 (s, 1H), 5.05 - 4.87 (m, 2H), 3.18 (dt, *J* = 10.3, 5.1 Hz, 1H), 2.61 (s, 1H), 2.26 - 2.14 (m, 1H), 2.09 - 1.97 (m, 2H), 1.90 - 1.01 (m, 20H), 0.98 (d, *J* = 6.3 Hz, 3H), 0.67 (s, 3H).

$^{13}C$ NMR (101 MHz, $CDCl_3$) δ 202.11, 142.39, 136.04, 113.23, 93.16, 77.20, 61.65, 61.11, 55.89, 45.46, 41.72, 40.80, 39.16, 39.07, 36.03, 35.92, 35.28, 33.66, 31.16, 26.16, 25.64, 24.33, 23.09, 18.59, 13.81.

MS m/z (ESI): 392 $[M+H-18]^+$.

The following target compounds 26-68 were synthesized using synthesis methods similar to those in Examples 1 and 2:

| Example No. | Compound structure | LC-MS (m/z [M+H-18]$^+$) |
|---|---|---|
| 26 | 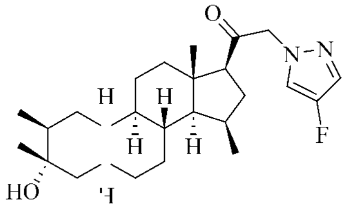 | 413.3 |
| 27 | 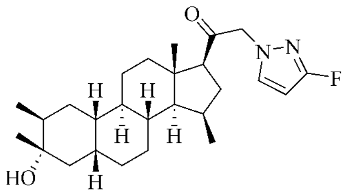 | 413.3 |
| 28 | 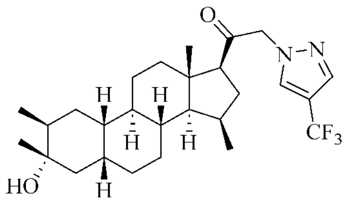 | 463.3 |
| 29 | 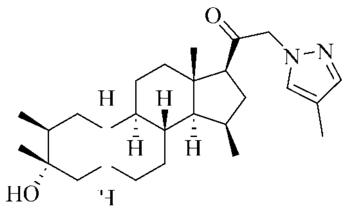 | 409.3 |
| 30 | 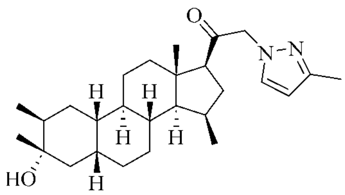 | 409.3 |
| 31 | 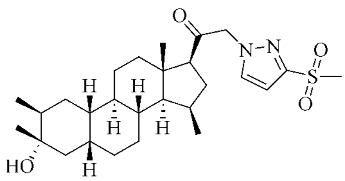 | 473.3 |
| 32 | 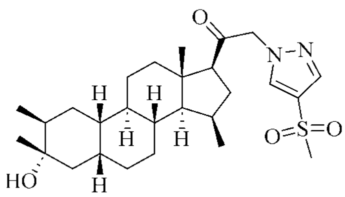 | 473.3 |
| 33 | 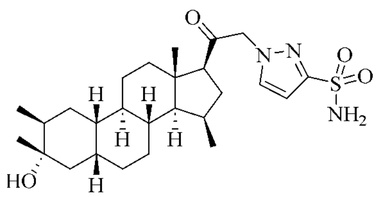 | 474.3 |

(continued)

| Example No. | Compound structure | LC-MS (m/z [M+H-18]+) |
|---|---|---|
| 34 | 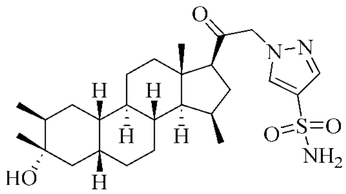 | 474.3 |
| 35 | 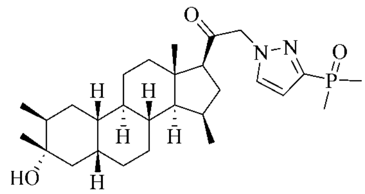 | 471.3 |
| 36 | 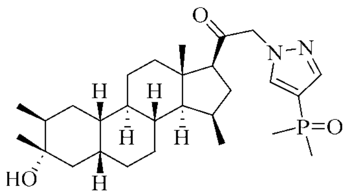 | 471.3 |
| 37 | 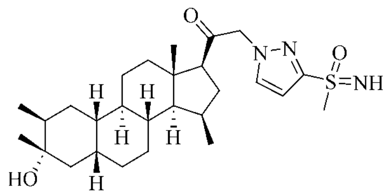 | 472.3 |
| 38 | 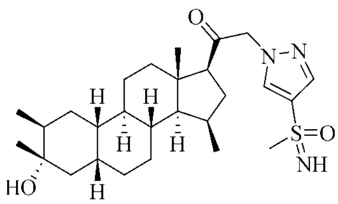 | 472.3 |
| 39 | 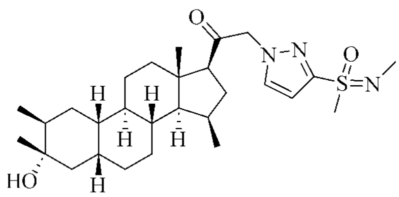 | 486.3 |
| 40 | 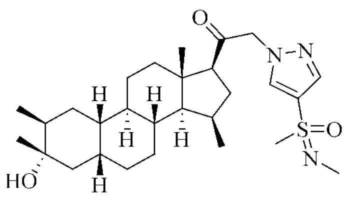 | 486.3 |
| 41 | 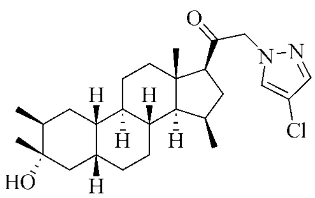 | 429.3 |

(continued)

| Example No. | Compound structure | LC-MS (m/z [M+H-18]+) |
|---|---|---|
| 42 | 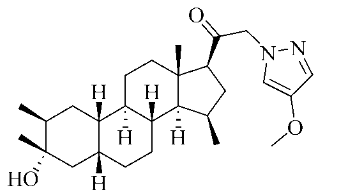 | 425.3 |
| 43 | 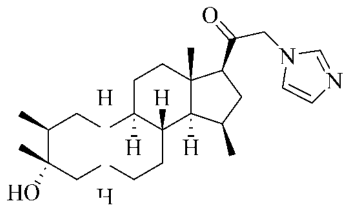 | 395.3 |
| 44 | 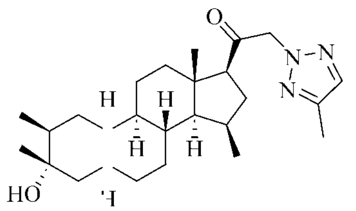 | 410.3 |
| 45 | 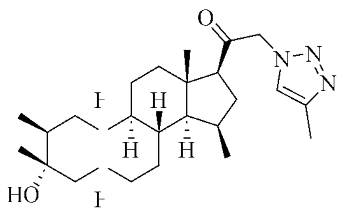 | 410.3 |
| 46 | 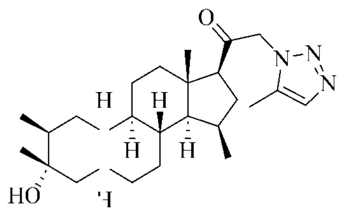 | 410.3 |
| 47 | 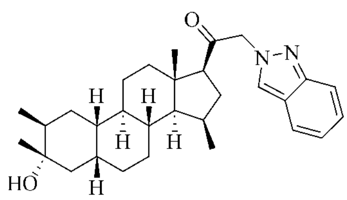 | 445.3 |
| 48 | 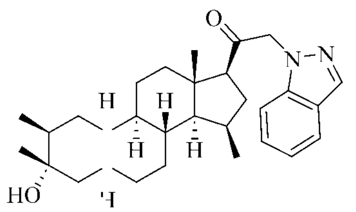 | 445.3 |

(continued)

| Example No. | Compound structure | LC-MS (m/z [M+H-18]+) |
|---|---|---|
| 49 | 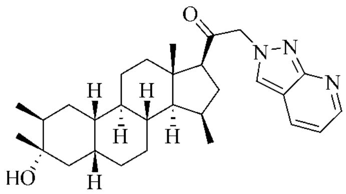 | 446.3 |
| 50 | 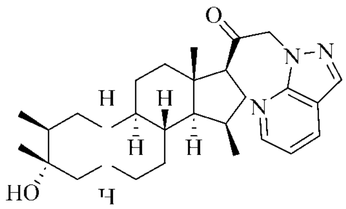 | 446.3 |
| 51 | 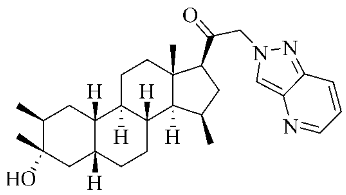 | 446.3 |
| 52 | 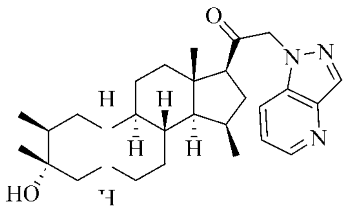 | 446.3 |
| 53 | 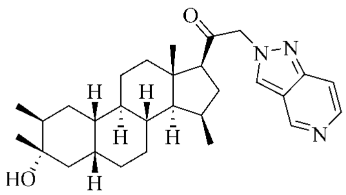 | 446.3 |
| 54 | 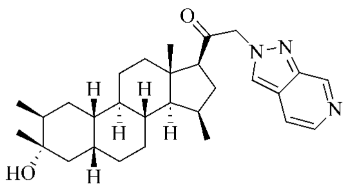 | 446.3 |
| 55 | 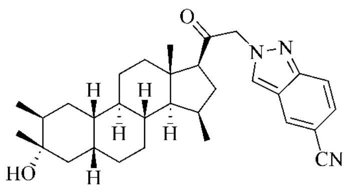 | 470.3 |

(continued)

| Example No. | Compound structure | LC-MS (m/z [M+H-18]+) |
|---|---|---|
| 56 | 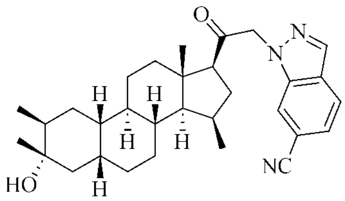 | 470.3 |
| 57 | 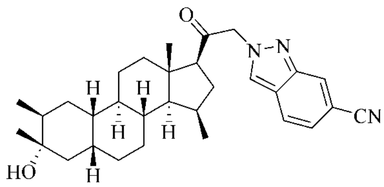 | 470.3 |
| 58 | 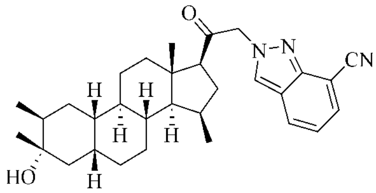 | 470.3 |
| 59 | 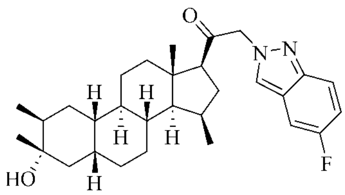 | 463.3 |
| 60 | 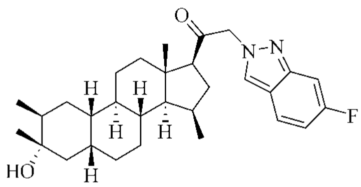 | 463.3 |
| 61 | 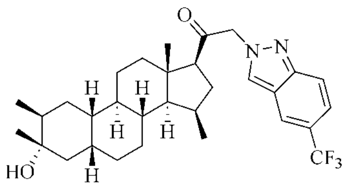 | 513.3 |
| 62 | 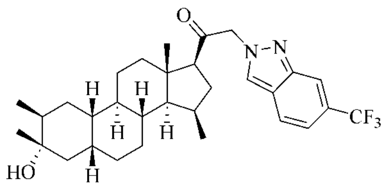 | 513.3 |
| 63 | 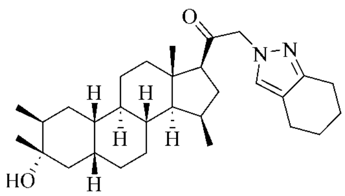 | 449.4 |

(continued)

| Example No. | Compound structure | LC-MS (m/z [M+H-18]$^+$) |
|---|---|---|
| 64 | 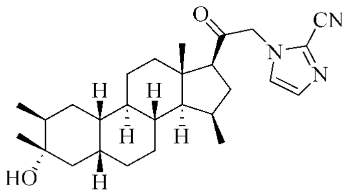 | 420.3 |
| 65 | 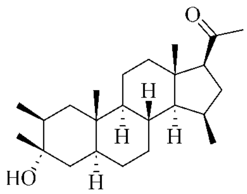 | 343.3 |
| 66 | 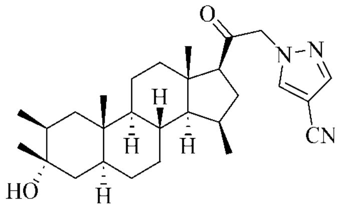 | 434.3 |
| 67 | 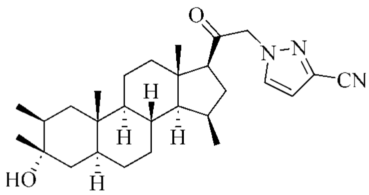 | 434.3 |
| 68 | 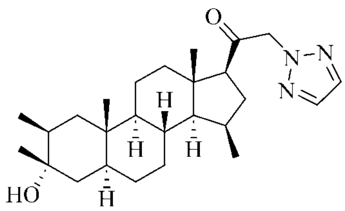 | 410.3 |

## Biological Assay Evaluations

The present disclosure is further described below with reference to test examples, but these examples are not intended to limit the scope of the present disclosure.

The structure of compound Sage-217 is shown below:

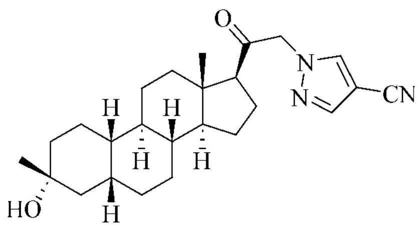

,

which was prepared by referring to the preparation method in Example 20 of patent CN105339381B.

## Test Example 1. Positive Modulatory Effects of Compounds of the Present Disclosure on GABAa Receptor

1. Experimental method:

1.1 Cell culture

The cells used in this experiment were derived from a CHO cell line that stably expressed normal GABAa receptor functions after transfection with human GABAa receptor $\alpha$1, $\beta$2, and $\gamma$2 subunit cDNAs. The cell line was constructed by B'sys GmbH. The cells were cultured in a medium (purchased from Invitrogen) containing the following components: DMEM culture medium; 10% (v/v) inactivated fetal bovine serum; 5 $\mu$g/mL Puromycin; 250 $\mu$g/mL Hygromycin B; and 100 $\mu$g/mL Zeocin. The cells were grown in culture dishes containing the above medium and cultured in an incubator at 37 °C with 5% $CO_2$. 12 h to 24 h before the electrophysiological experiment, the cells were transferred onto circular glass slides placed in culture dishes, and were grown under the same medium and culture conditions as above. The density of cells on each circular glass slide was required to meet the requirement that the overwhelming majority of cells were independent and individual.

1.2 Electrophysiological recording system

In this experiment, a manual patch-clamp system HEKA EPC10 USB signal amplifier and digital conversion system (purchased from HEKA Electronics, Germany) were used for whole-cell current recording. The circular glass slides with surface-grown CHO GABAa cells (see the cell culture and requirement section above) were taken out from the culture dishes and then placed into an electrophysiological recording chamber under an inverted microscope. The recording chamber was continuously perfused with extracellular fluid (approximately 1-2 mL/min). In the experiment, the whole-cell current recording technique was employed to record chloride ion currents of GABAa channels. 1 $\mu$M GABA was used to activate chloride ion currents of GABAa channels on each cell as an initial control (the reason for using 1 $\mu$M GABA concentration was that: this concentration was an effective activation concentration for the GABAa ion channel agonist GABA on the cells used in this experiment, falling between 10% and 20%). Unless otherwise specified, the experiment was performed at conventional room temperature (about 25 °C), and only cells meeting the electrophysiological recording criteria were adopted. In the experiment, the cells were ruptured and sealed, and were clamped at -80 mV after entering a whole-cell state. 1 $\mu$M GABA acted on the cells through the drug perfusion system to induce chloride ion currents of GABAa channels; the action time was about 4 s, and the current magnitude was used as the initial control value. Subsequently, the cells were perfused and incubated with the test concentration compound for 3 min, and then a mixed solution of 1 $\mu$M GABA and the corresponding test concentration compound was applied to act on the cells to induce currents, so as to observe the enhancing effect of the test compound on the currents induced by 1 $\mu$M GABA. When the cell state stabilized, the test compound would be applied from low to high concentrations to act on the same cell. In the experiment, to avoid current reduction caused by desensitization of GABAa ion channels, the interval time for the test substance acting on the cells through the drug perfusion system was at least 180 s.

2. Data analysis

The experimental data were analyzed using data analysis software provided by HEKA Patchmaster, Microsoft Excel, and Graphpad Prism.

3. Experimental results

Table 1-1. Results of *in vitro* GABAa receptor ($\alpha1\beta2\gamma2$) cellular activity assay of compounds of the present disclosure (single concentration)[1]

| Example No. | Agonist ratio[2] |
|---|---|
| 2 | 2108 |
| 4 | 626 |
| 5 | 1919 |
| 6 | 380 |
| 7 | 794 |
| 9 | 1305 |
| 11 | 1313 |

(continued)

| Example No. | Agonist ratio[2] |
|---|---|
| 17 | 217 |
| 19 | 1614 |
| 20 | 970 |

Table 1-2. Results of *in vitro* GABAa receptor ($\alpha1\beta2\gamma2$) cellular activity assay of compounds of the present disclosure

| Example No. | $EC_{50}$ (nM)[3] | $E_{max}$ (% )[4] |
|---|---|---|
| 2 | 22 | 2108 |
| 9 | 40 | 1578 |
| 20 | 110 | 2382 |
| Sage-217 | 120 | 2654 |

[1] The single concentration selected was 0.1 μM.
[2] The agonist ratio is the relative enhancement amplitude of GABAa receptor currents induced by 1 μM GABA, expressed as a percentage.
[3] The $EC_{50}$ (half-maximal effective concentration) value for the test compound acting as a positive modulator to enhance GABAa receptor (chloride ion channel) currents induced by 1 μM GABA.
[4] The $E_{max}$ is the maximum relative enhancement amplitude of GABAa receptor currents induced by 1 μM GABA, expressed as a percentage. The $E_{max}$ and its corresponding test compound concentration values were estimated from the $EC_{50}$ fitting curve.

4. Experimental conclusion

As can be seen from Table 1, the compounds of the examples of the present disclosure had excellent positive modulatory effects on the GABAa receptor. Among them, Examples 2, 9, and 20 exhibited lower $E_{max}$ compared to Sage-217, indicating potentially fewer *in vivo* side effects; in combination with lower $EC_{50}$, they demonstrated potential for a larger therapeutic window.

**Test Example *2. In Vivo* Efficacy Experiment in PTZ-Induced Epilepsy Mouse Model**

1. Experimental method

Experimental animals

Male C57 mice, purchased from SPF (Suzhou) Biotechnology Co., Ltd.

Test compounds

The test compounds were all stored in a refrigerator at 4 °C.
Information on the test samples is shown in the table below:

| Name of test sample | Cat. No. | Batch No. | Supplier |
|---|---|---|---|
| Pentylenetetrazole (PTZ) | P6500-25G | MKCR2882 | SIGMA |
| 0.9% sodium chloride injection | / | F23030705’ | Hebei Tiancheng Pharmaceutical Co., Ltd. |
| Dimethyl sulfoxide (DMSO) | D8418 | SHBP7906 | SIGMA |
| Solutol HS-15 | HY-Y1893 | 159186 | MCE |
| (2-Hydroxypropyl)-β-cyclodextrin | XW12844635 53 | 20221130 | Sinopharm Chemical Reagent Co., Ltd. - Wokai |

Experimental instruments

1 mL Kindly disposable sterile syringe with needle
100-1000 μL pipette
Vortex-Genie2 multifunctional vortex mixer
Numerical control ultrasonic cleaning instrument
MSA series magnetic stirrer
Sartorius SQP electronic balance
Liping precision electronic scale
Plexiglass box, with an opaque design (customized) for sidewalls of adjacent spaces
Grouping of experimental animals

| Group | Dose (mg/kg) | Number of animals (♂) | Number of groups |
|---|---|---|---|
| Vehicle (Vehicle①) | / | 10 | 1 |
| Test compounds | 3/1/0.3 | 10 | 1 |
| Note: ① Vehicle: 5% of DMSO + 5% of HS-15 + 90% of 20% hydroxypropyl-β-cyclodextrin (10 mL/kg, p.o.). | | | |

Solvent preparation: 20% hydroxypropyl-β-cyclodextrin

20.4082 g of hydroxypropyl-β-cyclodextrin (the purity was calculated as 98%) was precisely weighed out and added into a 100 mL solvent flask, and 60 mL of double-distilled water was added. The mixture was vortexed on a vortex mixer for 3-5 min, then placed on a magnetic stirrer, and well mixed with stirring for 1 h to give a homogeneous clear solution. Subsequently, the volume was brought to the mark (100 mL) by adding double-distilled water, and the mixture was vortexed and then magnetically stirred for 10 min to give a homogeneous clear solution.

Test compound preparation

PTZ: a 120 mg/kg PTZ solution (administration volume: 10 mL/kg) was prepared with normal saline as the solvent.

Test compounds: solutions of the test compounds at 3/1/0.3 mg/kg (administration volume: 10 mL/kg) were prepared with Vehicle in section 1.4 as the solvent.

Experimental procedures

One hour before the test, the experimental animals were transferred into an operation room for acclimation.

The mice were randomly grouped, marked, and weighed, with 10 mice in each group. At 60 min before PTZ administration, the test compounds and Vehicle were separately orally administered.

The PTZ solution was administered subcutaneously before experimental observation, and this time point was recorded as the observation starting point.

Immediately after PTZ administration, the animals were placed in observation boxes and observed for 30 min. The following observations of the animals were recorded:

number of animal deaths;

1) latency period of the first clonic seizure, 2) latency period of the first generalized tonic-clonic seizure, 3) number of clonic seizures, and 4) number of generalized tonic-clonic seizures (if the animal did not experience an epileptic seizure during the 30-min observation period, the latency period was recorded as 1800 s, and the number of seizures was recorded as 0).

Clonic seizure: the animal has a generalized myoclonus that lasts for more than 3 s and is accompanied by falling.

Tonic seizure: the limbs are straightened at a 90° angle to the torso.

2. Data analysis

All metrology data were expressed as mean ± standard error of mean (Mean ± SEM), and the Prism 8.0 statistical software was used for data testing and analysis.

3. Experimental results and conclusion

Table 2-1. Mouse survival rate statistics

| Example | Dose (mg/kg) | Survival rate (%) |
|---|---|---|
| Blank group | / | 0 |
| 5 | 3 | 100 |
| 6 | 3 | 100 |
| 7 | 3 | 100 |
| 8 | 3 | 100 |
| 9 | 3 | 100 |
| 10 | 3 | 100 |
| Sage-217 | 3 | 100 |
| 5 | 1 | 90 |
| 6 | 1 | 90 |
| 9 | 1 | 100 |
| 20 | 1 | 100 |
| Sage-217 | 1 | 100 |
| 5 | 0.3 | 10 |
| 9 | 0.3 | 10 |
| 20 | 0.3 | 10 |
| Sage-217 | 0.3 | 0 |

Conclusion: As can be seen from Table 2-1, compared to the blank group, all the compounds of the examples in this experiment prolonged the survival rate of mice with PTZ-induced convulsions, exhibiting protective effects against acute epileptic seizures in the mice. Among them, Examples 5, 6, 9, and 20, as well as Sage-217, still exhibited significant protective effects at a low dose (1 mpk), and Examples 5, 9, and 20 still exhibited partial protective effects at an extremely low dose (0.3 mpk).

Table 2-2. Statistics of clonic and tonic seizures in mice

| Example | Dose (mg/kg) | Clonic seizure latency period (sec) | Number of clonic seizures | Tonic seizure latency period (sec) | Number of tonic seizures |
|---|---|---|---|---|---|
| Blank group | / | 201.6±15.4 | 3.5±0.5 | 530.5±57.4 | 1.0±0.0 |
| 5 | 3 | 1133.7±192.7 | 0.6±0.2 | 1800.0±0.0 | 0.0±0.0 |
| 6 | 3 | 1132.4±195.9 | 0.8±0.2 | 1800.0±0.0 | 0.0±0.0 |
| 9 | 3 | 1649.7±105.0 | 0.2±0.1 | 1800.0±0.0 | 0.0±0.0 |
| 20 | 3 | 1800.0±0.0 | 0.0±0.0 | 1800.0±0.0 | 0.0±0.0 |
| Sage-217 | 3 | 1012.3±181.4 | 0.9±0.3 | 1800.0±0.0 | 0.0±0.0 |

Conclusion: As can be seen from Table 2-2, compared to the blank group, all the compounds of the examples in this experiment significantly prolonged the latency period of clonic seizures and generalized tonic-clonic seizures and significantly reduced their number of occurrences, demonstrating significant antiepileptic effects.

**Test Example *3. In Vivo* Efficacy Experiment by Forced Swimming and Sucrose Preference Tests in Mice**

1. Experimental objective:

The core manifestations of depression are despair, helplessness, and anhedonia. The forced swimming test is commonly used to evaluate despair-like behavior. In this test, the animal is placed in an unpleasant and inescapable environment (a water tank). Initially, the animal shows active behaviors (swimming or struggling); subsequently, the animal begins to remain immobile, and the immobility time gradually increases. Anhedonia means a loss of interest in things that were once pleasurable; in rodents, it is typically manifested as a reduced preference for sweet foods (sucrose). Therefore, the *in vivo* efficacy of the compounds of the examples of the present disclosure in mice can be evaluated by forced swimming and sucrose preference tests.

2. Experimental materials

2.1 Experimental animals

Female/male C57 mice, purchased from SPF (Suzhou) Biotechnology Co., Ltd.

2.2 Test compounds

The test compounds were all stored in a refrigerator at 4 °C.

Information on the test samples is shown in the table below:

| Name of test sample | Cat. No. | Batch No. | Supplier |
|---|---|---|---|
| Purified water | / | 20220821 D | Guangzhou Watson's Food & Beverage Co., Ltd. |
| Dimethyl sulfoxide (DMSO) | D8418 | SHBP7906 | SIGMA |
| Solutol HS-15 | HY-Y1893 | 159186 | MCE |
| (2-Hydroxypropyl)-β-cyclodextrin | XW12844635 53 | 20221130 | Sinopharm Chemical Reagent Co., Ltd. - Wokai |

2.3 Experimental instruments

| **Name** | **Model** | **Manufacturer** |
|---|---|---|
| Precision electronic balance | SECURA225D1CN | Sartorius |
| Electronic balance | YP6001 | Liangping Instrument |
| Magnetic stirrer | LC-MSA-HD | LICHEN-BX |
| Vortex mixer | SI-0256 | Scientific Industries |
| Numerical control ultrasonic cleaner | KQ5200DE | Kunshan Ultrasonic Instruments |
| Experimental four-channel forced swimming system | XR-XQ202 | Shanghai Xinruan |
| Experimental rat shuttle system | XR-XC105-4 | Shanghai Xinruan |
| Learned helplessness LH software | XR-LH01 | Shanghai Xinruan |

2.4 Grouping of experimental animals

| Group | Dose (mg/kg) | Number of animals | Number of groups |
|---|---|---|---|
| Vehicle (Vehicle①) | / | 10 | 1 |

(continued)

| Group | Dose (mg/kg) | Number of animals | Number of groups |
|---|---|---|---|
| Test compounds | 3 | 10 | 1 |

Note: ①Vehicle: 5% of DMSO + 5% of HS-15 + 90% of 20% hydroxypropyl-β-cyclodextrin (10 mL/kg, p.o.).

2.5 Test sample preparation

During test sample preparation, a certain amount of the sample was taken, a vehicle was added, and the mixture was subjected to ultrasonication.

Test sample dilution: A certain volume of the stock solution was taken and serially diluted with the required volume of the vehicle. The prepared drug solution was labeled for later use.

Calculation formulas:

Theoretical concentration (mg/mL) = dose (mg/kg)/administration volume (mL/kg)

Theoretical sample weighing amount (mg) = [preparation volume (mL) × theoretical test sample concentration (mg/mL) × salt coefficient]/purity

2.6 Experimental procedures

Forced swimming: Animals were divided into a control group and various test drug administration dose groups, which were subjected to vehicle and test drug administration, respectively. One hour after the administration, the animals were placed in a 25 °C plexiglass cylinder with a water depth of 20 cm. The swimming status of the animals in the water was recorded by video, and the immobility time of the animals (characterized by floating in the water and stopping struggling) during minutes 2-6 was recorded and analyzed.

Sugar preference: D1: a 1% sucrose solution was provided on both sides; D2/3: drinking water was provided on one side, while a 1% sucrose solution was provided on the other side; D4: water and food were deprived; D5: sucrose preference degree was tested. During the test, the mice with chronic stress were subjected to administration according to the experimental groups. After the administration, pre-weighed sucrose solution and drinking water were provided on D2/3. The animals were allowed free access to drinking for 2 h in a dark environment, and during this period, the positions of the sucrose solution and drinking water were switched at the 1-hour mark.

3. Data processing

Experimental data were expressed as $\bar{\chi}$ ± SEM. One-way analysis of variance was employed, and pairwise comparisons were performed using Dunnett's multiple comparisons tests. The floating immobility time, swimming time, and struggling time were recorded.

Sucrose preference degree (%) = sucrose solution consumption/(sucrose solution consumption + purified water consumption) × 100%.

4. Experimental results and conclusion

Table 3-1. Statistical results of immobility time during minutes 2-6 in mouse forced swimming test

| Example | Mean value (s) | P value |
|---|---|---|
| Blank group | 121.3 | / |
| Sage-217 | 56.38 | 0.008 |
| Example 9 | 54.68 | 0.006 |

Table 3-2. Sucrose preference degree results in mouse sucrose preference test

| Example | Mean value (%) | P value |
|---|---|---|
| Blank group | 65.45 | / |

(continued)

| Example | Mean value (%) | P value |
|---|---|---|
| Sage-217 | 84.31 | 0.0037 |
| Example 9 | 88.37 | 0.0005 |

As can be seen from the above results, compared to the blank group, the compound of Example 9 of the present disclosure could significantly reduce the immobility time of C57 mice in the forced swimming test and significantly improve the sucrose preference degree of C57 mice in the sucrose preference test, demonstrating significant antidepressant effects; additionally, it demonstrated certain advantages compared to Sage-217.

**Test Example 4. *In Vivo* Pharmacokinetic Experiment of Compounds of the Present Disclosure in Mice**

1. Experimental objective:

Male ICR mice were subjected to oral intragastric administration. The plasma concentrations of the compounds of the present disclosure in the mice were measured, the PK parameters were calculated, and pharmacokinetic evaluation was performed on the compounds of the present disclosure.

2. Experimental materials:

(1) Experimental samples: the compounds of the examples of the present disclosure, self-made.
(2) Experimental animals: ICR mice, SPF-grade, male, from Shanghai Lingchang Biotechnology Co., Ltd.
(3) Main experimental instruments:

| **Name** | **Model** | **Manufacturer** |
|---|---|---|
| AB Sciex triple quadrupole liquid chromatography-mass spectrometry instrument | Triple Quad 5500+ QTRAP | SCIEX |
| Sartorius dual-range balance | SECURA225D-ICN | Sartorius Scientific Instruments Co., Ltd. |
| Benchtop high-speed refrigerated centrifuge | Thermo 75009915 | Thermo Scientific |
| RAININ micropipette | 20/200/1000 μL | RAININ®, USA |
| Microplate shaker | 88882006 | Thermo Scientific |
| Benchtop ultrasonic cleaner | KQ5200DE | Kunshan Ultrasonic Instruments Co., Ltd. |
| Vortex oscillator | Vortex genie 2 | Scientific Industries, USA |
| Water purification instrument | Milli-Q IQ7000 | Merck, Germany |
| Precision electronic balance | XSE105DU | METTLER TOLEDO |

3. Experimental scheme:

(1) Administration information:

Drug preparation: according to the weight of the weighed drug, the preparation volume was calculated; 5% of DMSO and 5% of HS-15 were added sequentially; after the drug was fully dissolved, 90% of a 20% aqueous β-cyclodextrin solution was added, and the mixture was well mixed for later use.
Route of administration: oral intragastric administration, at a dose of 10 mg/kg.
Administration frequency and duration: single administration.

(2) Experimental method:

ICR mice were stratified according to body weight and then randomly grouped, with 9 mice per group. The mice were fasted overnight prior to the experiment. The mice were subjected to oral intragastric administration. At 0 h, 0.167 h, 0.333 h, 0.5 h, 1 h, 2 h, 4 h, 6 h, 8 h, 12 h, and 24 h, 50 μL of blood was collected from the fundus vein of each mouse into a sample tube containing heparin sodium as an anticoagulant, and then the tube was placed in wet ice and centrifuged at 4000 $r \cdot min^{-1}$ for 10 min. The plasma was separated, and cryopreserved in a refrigerator at -80 °C for testing.

4. Experimental results and analysis:

The concentrations of the plasma samples were measured by high-performance liquid chromatography-tandem mass spectrometry (LC-MS/MS). The retention times of the compounds and internal standard, chromatographic acquisitions, and integrals of chromatograms were processed using the software Analyst (AB SCIEX), and statistical analysis of the data was performed using the software Microsoft office. The plasma concentrations were processed using a non-compartmental model of the pharmacokinetic software WinNonlin™ Version 6.3 (Pharsight, Mountain View, CA), and the pharmacokinetic parameters were calculated using a linear log-trapezoidal method. The specific results are shown in Table 4.

Table 4. Results of pharmacokinetic experiment in mice

| Sample | $t_{1/2}$ (h) | $C_{max}$ (ng/mL) | $T_{max}$ (h) | $AUC_{(0-t)}$ (ng•h/mL) |
|---|---|---|---|---|
| Sage-217 | 2.17 | 1510 | 1 | 9158 |
| Example 2 | 7.38 | 1237 | 4 | 16583 |
| Example 9 | 2.35 | 3483 | 0.33 | 23615 |
| Example 19 | >8 | 1647 | 8 | 26741 |
| Example 20 | 2.59 | 2576 | 0.5 | 32233 |

5. Experimental conclusion:

As can be seen from the results of the pharmacokinetic experiment in mice shown in the table, the compounds of the present disclosure had longer half-lives $t_{1/2}$, greater exposure AUC, and higher maximum plasma concentrations $C_{max}$, demonstrating good metabolic properties. This result also clearly reflects that such compounds with lower $E_{max}$, owing to their longer half-lives and greater exposure, demonstrated better *in vivo* efficacy compared to Sage-217.

**Test Example 5. Acute Toxicity Experiment and Side Effect Observation of Compounds of the Present Disclosure in Mice**

1. Experimental objective

This experiment aimed to evaluate the acute toxic manifestations of the test compounds following single oral intragastric administration in ICR mice, observe the recovery status of the ICR mice within 14 days post-administration, and determine the maximum tolerated dose of the test compounds.

2. Experimental method

2.1 Experimental animals

65 male and 65 female ICR mice were purchased from Shanghai Jihui Laboratory Animal Care Co., Ltd. After 4-5 days of acclimatization of the experimental animals, the formal experiment was carried out. The mice were housed at 5 mice/cage in a 12/12-hour light/dark cycle at a room temperature of 23±2 °C, with free access to food and water.

2.2 Sample storage

The test compounds were all stored in a refrigerator at 4 °C.

Table 5-1. Information on test samples

| Test sample and vehicle names | Cat. No. | Batch No. | Supplier |
|---|---|---|---|
| Sage-217 | -- | 1 | Biochempartner |
| Example 9 | -- | 1 | Self-made |
| Example 20 | -- | 1 | Self-made |
| Dimethyl sulfoxide (DMSO) | D8418 | SHBP7906 | SIGMA |
| Solutol HS-15 | HY-Y1893 | 159186 | MCE |
| (2-Hydroxypropyl)-β-cyclodextrin | XW1284463553 | 20221130 | Sinopharm Chemical Reagent Co., Ltd. - Wokai |

2.3 Experimental instruments

1 mL Kindly disposable sterile syringe with needle

100-1000 μL pipette

Vortex-Genie2 multifunctional vortex mixer

Numerical control ultrasonic cleaning instrument

MSA series magnetic stirrer

Sartorius SQP electronic balance

Liping precision electronic scale

2.4 Grouping of experimental animals

Table 5-2: Animal grouping information

| Group | Dose (mg/kg) | Number of animals (♂) | Number of animals (♀) |
|---|---|---|---|
| Vehicle (Vehicle①) | - | 5 | 5 |
| Sage-217 | 2.5/5/10/20 | 5 mice/dose | 5 mice/dose |
| Example 9 | 2.5/5/10/20 | 5 mice/dose | 5 mice/dose |
| Example 20 | 2.5/5/10/20 | 5 mice/dose | 5 mice/dose |
| Note: ①Vehicle: 5% of DMSO + 5% of HS-15 + 90% of 20% hydroxypropyl-β-cyclodextrin (10 mL/kg, p.o.). | | | |

3. Experimental procedures

3.1 Solvent preparation: 20% hydroxypropyl-β-cyclodextrin

20.4082 g of hydroxypropyl-β-cyclodextrin (the purity was calculated as 98%) was precisely weighed out and added into a 100 mL solvent flask, and 60 mL of double-distilled water was added. The mixture was vortexed on a vortex mixer for 3-5 min, then placed on a magnetic stirrer, and well mixed with stirring for 1 h to give a homogeneous clear solution. Subsequently, the volume was brought to the mark (100 mL) by adding double-distilled water, and the mixture was vortexed and then magnetically stirred for 10 min to give a homogeneous clear solution.

3.2 Test compound preparation

Based on the Vehicle in section 2.4, a certain weight of the test compound was weighed, 5% of the total volume of a DMSO liquid was first added, and the mixture was vortexed. After the drug was completely dissolved, 5% of the total volume of an HS-15 liquid was added, and the mixture was well mixed and vortexed. Finally, a 20% hydroxypropyl-β-cyclodextrin solution was added according to the remaining volume proportion, forming a 2 mg/mL clear and homogeneous solution. The solution was then proportionally diluted in a gradient to concentrations of 1 mg/mL, 0.5 mg/mL, and 0.25 mg/mL (the administration volume was 10 mL/kg).

3.3 Experimental procedures

a. About 12-18 h before testing, animals were weighed and then grouped, and fasted overnight (water was accessible).
b. The next day, the mice were reweighed according to groups. Ten mice per group (5 males and 5 females/dose) were subjected to oral intragastric administration of the test compound or Vehicle. The day of administration was designated as Day 1.
c. According to experimental process of the acute toxicity test, each group was observed in detail at the cage side at 0.5-1 h and 4-8 h after the administration on Day 1, and the cage-side observation was performed once daily during Day 2 to Day 14.
d. Observation period for Day 2 to Day 14: 9:00 to 12:00.

4. Recording indexes

(1) The clinical symptoms and mortality in each group of ICR mice post-administration were recorded to determine the maximum tolerated dose.

(2) The symptom alleviation and recovery status in each group of ICR mice post-administration were recorded.

5. Experimental results

(1) No death occurred in any group within 0.5-1 h post-administration.
(2) At 4-8 h post-administration, in the 20 mpk group of Sage-217, 1 male and 2 female mice died, while the surviving mice still exhibited prostration symptoms; no death occurred in all dose groups of Examples 9 and 20; the 20 mpk group of Example 9 only showed gait instability, and all lower dose groups returned to normal.
(3) All the remaining mice survived until Day 14.

The detailed cage-side observation results for a certain dose on the first day are shown in Table 5-3.

Table 5-3. Cage-side observation statistics for 10 mpk on the first day

| Group / Condition | | 0-1 h post-administration | | | | | | 4-8 h post-administration | | | | | |
|---|---|---|---|---|---|---|---|---|---|---|---|---|---|
| | | Sage-217 | | Example 9 | | Example 20 | | Sage-217 | | Example 9 | | Example 20 | |
| | | Male | Female | Male | Female | Male | Female | Male | Female | Male | Female | Male | Female |
| Gait instability | Number | | | 3 | 3 | 5 | 5 | | | | | | |
| | Severity | | | **a | ** | *** | * | | | | | | |

| | | | | | | | | | | | | | |
|---|---|---|---|---|---|---|---|---|---|---|---|---|---|
| Hypoactivity | Number | 4 | 5 | | | 2 | | | | | | | |
| | Severity | *** | *** | | | *** | | | | | | | |
| Prostration[b] | Number | 4 | 5 | 3 | 2 | 2 | | 1 | 1 | | | | |
| | Severity | *** | *** | ** | ** | *** | | *** | *** | | | | |
| Note (other symptoms) | | Core manifestations: severe ataxia (***) and severe loss of righting reflex (**)[c] | | Gait instability progressed to prostration, and 2 female and 2 male mice exhibited loss of righting reflex* | | Ataxia (**)[d] | | The rest substantially returned to normal | | Substantially returned to normal | | Substantially returned to normal | |

a. "*" indicates the severity of individual side effects; * represents mild, ** represents moderate, and *** represents severe.

b. Prostration often coincides with the appearance of hypoactivity.

c. Gait instability/prostration/loss of righting reflex can be understood as progressively worsening side effects.

d Ataxia includes gait instability and loss of balance.

6. Experimental conclusion

Under the conditions of this experiment, after the ICR mice were subjected to single oral intragastric administration of Sage-217 at 2.5 mg/kg, 5 mg/kg, 10 mg/kg, and 20 mg/kg, mortality was observed in both male and female mice in the 20 mg/kg group, and the maximum tolerated dose (MTD) was 10 mg/kg; cage-side observations of all dose groups primarily showed ataxia that aggravated with the increase of dose, as well as severe loss of righting reflex observed at high doses. For Example 9, all animals could tolerate it, the MTD was 20 mg/kg, and there was potential to further increase the dose, with the main adverse reactions being only prostration and mild loss of righting reflex observed in some of the mice at high doses. For Example 20, all animals could tolerate it, the MTD was 20 mg/kg, and ataxia that aggravated with the increase of dose was the primary symptom, but no mouse death occurred.

In summary, the compounds of the examples of the present disclosure showed significantly superior safety compared to Sage-217, and weakened the side effects of the GABAa agonist, thereby exhibiting value for further clinical development.

Although specific embodiments of the present disclosure have been described in detail, various modifications

and substitutions can be made to the details of the technical solutions of the present disclosure by those skilled in the art according to all the teachings that have been disclosed, and these changes shall all fall within the protection scope of the present disclosure. The full scope of the present disclosure is given by the appended claims and any equivalents thereof.

## Claims

1. A compound represented by general formula (I), a stereoisomer thereof, or a pharmaceutically acceptable salt thereof:

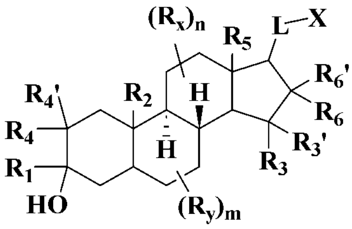

(I)

wherein:

X is hydrogen, $C_{1-6}$ alkyl, $C_{2-6}$ alkenyl, $C_{2-6}$ alkynyl, $C_{1-6}$ alkoxy, $C_{3-8}$ cycloalkyl, 3- to 8-membered heterocyclyl, $C_{6-10}$ aryl, or 5- to 10-membered heteroaryl, wherein the $C_{1-6}$ alkyl, $C_{2-6}$ alkenyl, $C_{2-6}$ alkynyl, $C_{1-6}$ alkoxy, $C_{3-8}$ cycloalkyl, 3- to 8-membered heterocyclyl, $C_{6-10}$ aryl, or 5- to 10-membered heteroaryl is optionally further substituted with one or more $R_A$;

$R_A$ is halogen, hydroxyl, $-NO_2$, -CN, $-NR_{aa}R_{bb}$, $-C(=O)R_{aa}$, $-C(=O)OR_{aa}$, $-OC(=O)R_{aa}$, $-OC(=O)OR_{aa}$, $-C(=O)NR_{aa}R_{bb}$, $-N(R_{aa})C(=O)R_{bb}$, $-OC(=O)NR_{aa}R_{bb}$, $-N(R_{aa})C(=O)OR_{bb}$, $-N(R_{cc})C(=O)NR_{aa}R_{bb}$, $-SR_{aa}$, $-S(=O)R_{aa}$, $-S(=O)_2R_{aa}$, $-S(=O)_2OR_{aa}$, $-OS(=O)_2R_{aa}$, $-S(=O)_2NR_{aa}R_{bb}$, $-S(=O)(=NR_{aa})R_{bb}$, $-N(R_{aa})S(=O)_2R_{bb}$, $-P(=O)R_{aa}R_{bb}$, $C_{1-6}$ alkyl, $C_{2-6}$ alkenyl, $C_{2-6}$ alkynyl, $C_{1-6}$ haloalkyl, $C_{1-6}$ alkoxy, $C_{1-6}$ haloalkoxy, $C_{3-8}$ cycloalkyl, or 3- to 8-membered heterocyclyl, wherein the $C_{1-6}$ alkyl, $C_{2-6}$ alkenyl, $C_{2-6}$ alkynyl, $C_{1-6}$ haloalkyl, $C_{1-6}$ alkoxy, $C_{1-6}$ haloalkoxy, $C_{3-8}$ cycloalkyl, or 3- to 8-membered heterocyclyl is optionally further substituted with one or more substituents selected from deuterium, halogen, hydroxyl, amino, sulfhydryl, nitryl, cyano, $C_{1-6}$ alkyl, $C_{1-6}$ haloalkyl, $C_{1-6}$ alkoxy, $C_{1-6}$ haloalkoxy, $C_{1-6}$ alkylthio, $C_{1-6}$ alkylamino, $C_{3-8}$ cycloalkyl, 3- to 8-membered heterocyclyl, $C_{6-10}$ aryl, and 5- to 10-membered heteroaryl;

$R_{aa}$, $R_{bb}$, and $R_{cc}$ are each independently hydrogen, $C_{1-6}$ alkyl, $C_{1-6}$ haloalkyl, $C_{3-8}$ cycloalkyl, 3- to 8-membered heterocyclyl, $C_{6-10}$ aryl, or 5- to 10-membered heteroaryl, or any two of $R_{aa}$, $R_{bb}$, and $R_{cc}$, together with the atom(s) to which they are attached, form 3- to 8-membered heterocyclyl or a 5- to 10-membered heteroaryl ring;

L is $-(CH_2)_{n1}-$, $-(CH_2)_{n1}O(CH_2)_{n2}-$, $-(CH_2)_{n1}S(CH_2)_{n2}-$, $-(CH_2)_{n1}NH(CH_2)_{n2}-$, $-(CH_2)_{n1}C(O)(CH_2)_{n2}-$, $-(CH_2)_{n1}S(O)(CH_2)_{n2}-$, $-(CH_2)_{n1}C(O)O(CH_2)_{n2}-$, $-(CH_2)_{n1}S(O)_2(CH_2)_{n2}-$, $-(CH_2)_{n1}C(O)NH(CH_2)_{n2}-$, $-(CH_2)_{n1}C(O)(CH_2)_{n2}NH-$, $-(CH_2)_{n1}NHC(O)(CH_2)_{n2}-$, or $-(CH_2)_{n1}NHS(O)_2(CH_2)_{n2}-$;

$R_x$ and $R_y$ are each independently hydrogen, deuterium, halogen, hydroxyl, amino, sulfhydryl, nitryl, cyano, $C_{1-6}$ alkyl, $C_{1-6}$ haloalkyl, $C_{1-6}$ alkoxy, $C_{1-6}$ haloalkoxy, $C_{3-8}$ cycloalkyl, 3- to 8-membered heterocyclyl, $C_{6-10}$ aryl, or 5- to 6-membered heteroaryl, wherein the $C_{1-6}$ alkyl, $C_{1-6}$ haloalkyl, $C_{1-6}$ alkoxy, $C_{1-6}$ haloalkoxy, $C_{3-8}$ cycloalkyl, 3- to 8-membered heterocyclyl, $C_{6-10}$ aryl, or 5- to 6-membered heteroaryl is optionally further substituted with one or more substituents selected from deuterium, halogen, hydroxyl, amino, sulfhydryl, nitryl, cyano, $C_{1-6}$ alkyl, $C_{1-6}$ haloalkyl, $C_{1-6}$ alkoxy, and $C_{1-6}$ haloalkoxy;

$R_1$, $R_2$, $R_3$, $R_3$', $R_5$, $R_6$, and $R_6$' are each independently hydrogen, deuterium, halogen, hydroxyl, amino, sulfhydryl, nitryl, cyano, $C_{1-6}$ alkyl, $C_{2-6}$ alkenyl, $C_{2-6}$ alkynyl, $C_{1-6}$ haloalkyl, $C_{1-6}$ alkoxy, $C_{1-6}$ haloalkoxy, $-(CH_2)_{n3}R_{dd}$, $-(CH_2)_{n3}OR_{dd}$, $-(CH_2)_{n3}SR_{dd}$, $-(CH_2)_{n3}C(O)R_{dd}$, $-(CH_2)_{n3}C(O)OR_{dd}$, $-(CH_2)_{n3}S(O)R_{dd}$, $-(CH2)_{n3}S(O)_2R_{dd}$, $-(CH_2)_{n3}NR_{dd}R_{ee}$, $-(CH_2)_{n3}C(O)NR_{dd}R_{ee}$, $-(CH_2)_{n3}S(O)NR_{dd}R_{ee}$, $-(CH2)_{n3}NR_{dd}C(O)R_{ee}$, $-(CH_2)_{n3}NR_{dd}S(O)R_{ee}$, or $-(CH_2)_{n3}NR_{dd}S(O)_2R_{ee}$;

$R_{dd}$ and $R_{ee}$ are each independently hydrogen, $C_{1-6}$ alkyl, $C_{1-6}$ haloalkyl, $C_{3-8}$ cycloalkyl, 3- to 8-membered heterocyclyl, $C_{6-10}$ aryl, or 5- to 10-membered heteroaryl;

$R_4$ and $R_4$' are each independently hydrogen, halogen, hydroxyl, amino, sulfhydryl, nitryl, cyano, $C_{1-6}$ alkyl, $C_{2-6}$ alkenyl, $C_{2-6}$ alkynyl, $C_{1-6}$ haloalkyl, $C_{1-6}$ alkoxy, $C_{1-6}$ haloalkoxy, or $C_{3-8}$ cycloalkyl; and $R_4$ and $R_4$' are not both hydrogen;

n is an integer from 0 to 5;

m is an integer from 0 to 5;
n1 is an integer from 0 to 3;
n2 is an integer from 0 to 3; and
n3 is an integer from 0 to 3.

**2.** The compound, the stereoisomer thereof, or the pharmaceutically acceptable salt thereof according to claim 1, wherein one or more of the following conditions are met:

(1) X is hydrogen, $C_{1-6}$ alkyl, $C_{6-10}$ aryl, or 5- to 10-membered heteroaryl, wherein the $C_{1-6}$ alkyl, $C_{6-10}$ aryl, or 5- to 10-membered heteroaryl is optionally further substituted with one or more $R_A$; preferably, X is hydrogen, $C_{6-10}$ aryl, or 5- to 10-membered heteroaryl, wherein the $C_{6-10}$ aryl or 5- to 10-membered heteroaryl is optionally further substituted with one or more $R_A$;
(2) $R_A$ is halogen, -CN, $-NR_{aa}R_{bb}$, $-C(=O)R_{aa}$, $-C(=O)OR_{aa}$, $-C(=O)NR_{aa}R_{bb}$, $-S(=O)_2R_{aa}$, - $S(=O)_2NR_{aa}R_{bb}$, $-S(=O)(=NR_{aa})R_{bb}$, $-P(=O)R_{aa}R_{bb}$, $C_{1-6}$ alkyl, $C_{1-6}$ haloalkyl, $C_{1-6}$ alkoxy, $C_{3-8}$ cycloalkyl, or 3- to 8-membered heterocyclyl, preferably halogen, -CN, $-S(=O)_2R_{aa}$, $-S(=O)_2NR_{aa}R_{bb}$, $-S(=O)(=NR_{aa})R_{bb}$, $-P(=O)R_{aa}R_{bb}$, $C_{1-6}$ alkyl, $C_{1-6}$ haloalkyl, or $C_{1-6}$ alkoxy, more preferably halogen, -CN, $-S(=O)_2R_{aa}$, - $S(=O)_2NR_{aa}R_{bb}$, $-S(=O)(=NR_{aa})R_{bb}$, $-P(=O)R_{aa}R_{bb}$, $C_{1-3}$ alkyl, $C_{1-3}$ haloalkyl, or $C_{1-3}$ alkoxy, and further preferably fluorine, chlorine, -CN, methyl, trifluoromethyl, methoxy, $-SO_2CH_3$, $-SO_2NH_2$, $-SCH_3(=O)(=NH)$, $-SCH_3(=O)(=NCH_3)$, or $-P(=O)(CH_3)_2$;
(3) $R_{aa}$, $R_{bb}$, and $R_{cc}$ are each independently hydrogen or $C_{1-6}$ alkyl, preferably hydrogen or $C_{1-3}$ alkyl, and more preferably hydrogen or methyl;
(4) L is $-(CH_2)_{n1}-$, $-(CH_2)_{n1}C(O)(CH_2)_{n2}-$, $-(CH_2)_{n1}(O)_2(CH_2)_{n2}-$, $-(CH_2)_{n1}C(O)NH(CH_2)_{n2}-$, - $(CH_2)_{n1}C(O)(CH_2)_{n2}NH-$, $-(CH_2)_{n1}NHC(O)(CH_2)_{n2}-$, or $-(CH_2)_{n1}NHS(O)_2(CH_2)_{n2}-$, preferably - $C(O)(CH_2)_{n2}-$, $-C(O)NH(CH_2)_{n2}-$, $-C(O)(CH_2)_{n2}NH-$, $-(CH_2)_{n1}NHC(O)-$, or $-(CH_2)_{n1}NHS(O)_2-$, and more preferably $-C(O)(CH_2)_{n2}-$ or $-C(O)(CH_2)_{n2}NH-$;
(5) $R_x$ and $R_y$ are each independently hydrogen, deuterium, halogen, hydroxyl, $C_{1-6}$ alkyl, $C_{1-6}$ haloalkyl, $C_{1-6}$ alkoxy, or $C_{3-8}$ cycloalkyl, preferably hydrogen, deuterium, halogen, hydroxyl, or $C_{1-6}$ alkyl, and more preferably hydrogen;
(6) $R_1$, $R_2$, and $R_5$ are each independently hydrogen, deuterium, halogen, hydroxyl, $C_{1-6}$ alkyl, $C_{2-6}$ alkenyl, $C_{2-6}$ alkynyl, $C_{1-6}$ haloalkyl, $C_{1-6}$ alkoxy, $-(CH_2)_{n3}OR_{dd}$, $-(CH_2)_{n3}SR_{dd}$, or $-(CH_2)_{n3}C(O)R_{dd}$, preferably hydrogen, deuterium, $C_{1-3}$ alkyl, $C_{2-4}$ alkenyl, or $C_{2-4}$ alkynyl, more preferably hydrogen or $C_{1-3}$ alkyl, and further preferably hydrogen or methyl;
(7) $R_{dd}$ and $R_{ee}$ are each independently hydrogen, $C_{1-6}$ alkyl, or $C_{1-6}$ haloalkyl, preferably hydrogen or $C_{1-3}$ alkyl;
(8) $R_3$, $R_3$', $R_6$, and $R_6$' are each independently hydrogen, deuterium, or $C_{1-6}$ alkyl, preferably hydrogen, deuterium, or $C_{1-3}$ alkyl, more preferably hydrogen or $C_{1-3}$ alkyl, and further preferably hydrogen or methyl;
(9) $R_4$ and $R_4$' are each independently hydrogen or $C_{1-6}$ alkyl, preferably hydrogen or $C_{1-3}$ alkyl, and more preferably hydrogen or methyl; and $R_4$ and $R_4$' are not both hydrogen;
(10) n is selected from 0, 1, 2, 3, and 4, preferably 0, 1, or 2;
(11) m is selected from 0, 1, 2, 3, and 4, preferably 0, 1, or 2;
(12) n1 is selected from 0, 1, and 2, preferably 0 or 1;
(13) n2 is selected from 0, 1, and 2, preferably 0 or 1; and
(14) n3 is selected from 0, 1, and 2, preferably 0 or 1.

**3.** The compound, the stereoisomer thereof, or the pharmaceutically acceptable salt thereof according to claim 1 or 2, wherein general formula (I) further has a structure represented by general formula (II):

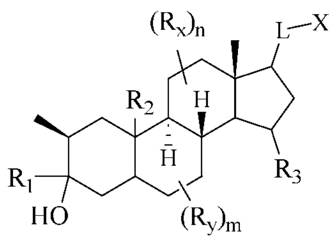

**(II)**

wherein:
X, L, $R_x$, $R_y$, $R_1$, $R_2$, $R_3$, n, and m are as defined in claim 1 or 2.

**4.** The compound, the stereoisomer thereof, or the pharmaceutically acceptable salt thereof according to any one of claims 1-3, wherein general formula (I) further has a structure represented by general formula (III):

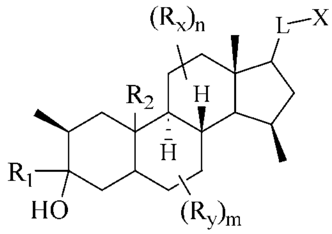

**(III)**

wherein:

X is hydrogen, $C_{1-6}$ alkyl, $C_{2-6}$ alkenyl, $C_{2-6}$ alkynyl, $C_{1-6}$ alkoxy, $C_{3-8}$ cycloalkyl, 3- to 8-membered heterocyclyl, $C_{6-10}$ aryl, or 5- to 10-membered heteroaryl, wherein the $C_{1-6}$ alkyl, $C_{2-6}$ alkenyl, $C_{2-6}$ alkynyl, $C_{1-6}$ alkoxy, $C_{3-8}$ cycloalkyl, 3- to 8-membered heterocyclyl, $C_{6-10}$ aryl, or 5- to 10-membered heteroaryl is optionally further substituted with one or more $R_A$;

$R_A$ is halogen, hydroxyl, $-NO_2$, -CN, $-NR_{aa}R_{bb}$, $-C(=O)R_{aa}$, $-C(=O)OR_{aa}$, $-OC(=O)R_{aa}$, $-OC(=O)OR_{aa}$, $-C(=O)NR_{aa}R_{bb}$, $-N(R_{aa})C(=O)R_{bb}$, $-OC(=O)NR_{aa}R_{bb}$, $-N(R_{aa})C(=O)OR_{bb}$, $-N(R_{cc})C(=O)NR_{aa}R_{bb}$, $-SR_{aa}$, $-S(=O)R_{aa}$, $-S(=O)_2R_{aa}$, $-S(=O)_2OR_{aa}$, $-OS(=O)_2R_{aa}$, $-S(=O)_2NR_{aa}R_{bb}$, $-S(=O)(=NR_{aa})R_{bb}$, $-N(R_{aa})S(=O)_2R_{bb}$, $-P(=O)R_{aa}R_{bb}$, $C_{1-6}$ alkyl, $C_{2-6}$ alkenyl, $C_{2-6}$ alkynyl, $C_{1-6}$ haloalkyl, $C_{1-6}$ alkoxy, $C_{1-6}$ haloalkoxy, $C_{3-8}$ cycloalkyl, or 3- to 8-membered heterocyclyl, wherein the $C_{1-6}$ alkyl, $C_{2-6}$ alkenyl, $C_{2-6}$ alkynyl, $C_{1-6}$ haloalkyl, $C_{1-6}$ alkoxy, $C_{1-6}$ haloalkoxy, $C_{3-8}$ cycloalkyl, or 3- to 8-membered heterocyclyl is optionally further substituted with one or more substituents selected from deuterium, halogen, hydroxyl, amino, sulfhydryl, nitryl, cyano, $C_{1-6}$ alkyl, $C_{1-6}$ haloalkyl, $C_{1-6}$ alkoxy, $C_{1-6}$ haloalkoxy, $C_{1-6}$ alkylthio, $C_{1-6}$ alkylamino, $C_{3-8}$ cycloalkyl, 3- to 8-membered heterocyclyl, $C_{6-10}$ aryl, and 5- to 10-membered heteroaryl;

$R_{aa}$, $R_{bb}$, and $R_{cc}$ are each independently hydrogen, $C_{1-6}$ alkyl, $C_{1-6}$ haloalkyl, $C_{3-8}$ cycloalkyl, 3- to 8-membered heterocyclyl, $C_{6-10}$ aryl, or 5- to 10-membered heteroaryl, or any two of $R_{aa}$, $R_{bb}$, and $R_{cc}$, together with the atom(s) to which they are attached, form 3- to 8-membered heterocyclyl or a 5- to 10-membered heteroaryl ring;

L is $-(CH_2)_{n1}-$, $-(CH_2)_{n1}O(CH_2)_{n2}-$, $-(CH_2)_{n1}S(CH_2)_{n2}-$, $-(CH_2)_{n1}NH(CH_2)_{n2}-$, $-(CH_2)_{n1}C(O)(CH_2)_{n2}-$, $-(CH_2)_{n1}S(O)(CH_2)_{n2}-$, $-(CH_2)_{n1}C(O)O(CH2)_{n2}-$, $-(CH_2)_{n1}S(O)_2(CH_2)_{n2}-$, $-(CH_2)_{n1}C(O)NH(CH_2)_{n2}-$, $-(CH_2)_{n1}NHC(O)(CH_2)_{n2}-$, or $-(CH_2)_{n1}NHS(O)_2(CH_2)_{n2}-$;

$R_x$ and $R_y$ are each independently hydrogen, deuterium, halogen, hydroxyl, amino, sulfhydryl, nitryl, cyano, $C_{1-6}$ alkyl, $C_{1-6}$ haloalkyl, $C_{1-6}$ alkoxy, $C_{1-6}$ haloalkoxy, $C_{3-8}$ cycloalkyl, 3- to 8-membered heterocyclyl, $C_{6-10}$ aryl, or 5- to 6-membered heteroaryl, wherein the $C_{1-6}$ alkyl, $C_{1-6}$ haloalkyl, $C_{1-6}$ alkoxy, $C_{1-6}$ haloalkoxy, $C_{3-8}$ cycloalkyl, 3- to 8-membered heterocyclyl, $C_{6-10}$ aryl, or 5- to 6-membered heteroaryl is optionally further substituted with one or more substituents selected from deuterium, halogen, hydroxyl, amino, sulfhydryl, nitryl, cyano, $C_{1-6}$ alkyl, $C_{1-6}$ haloalkyl, $C_{1-6}$ alkoxy, and $C_{1-6}$ haloalkoxy;

$R_1$ and $R_2$ are each independently hydrogen, deuterium, halogen, hydroxyl, amino, sulfhydryl, nitryl, cyano, $C_{1-6}$ alkyl, $C_{2-6}$ alkenyl, $C_{2-6}$ alkynyl, $C_{1-6}$ haloalkyl, $C_{1-6}$ alkoxy, $C_{1-6}$ haloalkoxy, $-(CH_2)_{n3}R_{dd}$, $-(CH_2)_{n3}OR_{dd}$, $-(CH_2)_{n3}SR_{dd}$, $-(CH_2)_{n3}C(O)R_{dd}$, $-(CH_2)_{n3}C(O)OR_{dd}$, $-(CH_2)_{n3}S(O)R_{dd}$, $-(CH_2)_{n3}S(O)_2R_{dd}$, $-(CH_2)_{n3}NR_{dd}R_{ee}$, $-(CH_2)_{n3}C(O)NR_{dd}R_{ee}$, $-(CH_2)_{n3}S(O)NR_{dd}R_{ee}$, $-(CH_2)_{n3}NR_{dd}C(O)R_{ee}$, $-(CH_2)_{n3}NR_{dd}S(O)R_{ee}$, or $-(CH_2)_{n3}NR_{dd}S(O)_2R_{ee}$;

$R_{dd}$ and $R_{ee}$ are each independently hydrogen, $C_{1-6}$ alkyl, $C_{1-6}$ haloalkyl, $C_{3-8}$ cycloalkyl, 3- to 8-membered heterocyclyl, $C_{6-10}$ aryl, or 5- to 10-membered heteroaryl;

n is an integer from 0 to 5;

m is an integer from 0 to 5;

n1 is an integer from 0 to 3;

n2 is an integer from 0 to 3; and

n3 is an integer from 0 to 3.

**5.** The compound, the stereoisomer thereof, or the pharmaceutically acceptable salt thereof according to any one of claims 1-4, wherein one or more of the following conditions are met:

(1) X is hydrogen, $C_{1-6}$ alkyl, $C_{6-10}$ aryl, or 5- to 10-membered heteroaryl, wherein the $C_{1-6}$ alkyl, $C_{6-10}$ aryl, or 5- to

10-membered heteroaryl is optionally further substituted with one or more $R_A$; preferably, X is hydrogen, $C_6$ in aryl, or 5- to 10-membered heteroaryl, wherein the $C_{6-10}$ aryl or 5- to 10-membered heteroaryl is optionally further substituted with one or more $R_A$;

(2) $R_A$ is halogen, -CN, $-NR_{aa}R_{bb}$, $-C(=O)R_{aa}$, $-C(=O)OR_{aa}$, $-C(=O)NR_{aa}R_{bb}$, $-S(=O)_2R_{aa}$, - $S(=O)_2NR_{aa}R_{bb}$, $-S(=O)(=NR_{aa})R_{bb}$, $-P(=O)R_{aa}R_{bb}$, $C_{1-6}$ alkyl, $C_{1-6}$ haloalkyl, $C_{1-6}$ alkoxy, $C_{3-8}$ cycloalkyl, or 3- to 8-membered heterocyclyl, preferably halogen, -CN, $-S(=O)_2R_{aa}$, $-S(=O)_2NR_{aa}R_{bb}$, $-S(=O)(=NR_{aa})R_{bb}$, $-P(=O)R_{aa}R_{bb}$, $C_{1-6}$ alkyl, $C_{1-6}$ haloalkyl, or $C_{1-6}$ alkoxy, more preferably halogen, -CN, $-S(=O)_2R_{aa}$, - $S(=O)_2NR_{aa}R_{bb}$, $-S(=O)(=NR_{aa})R_{bb}$, $-P(=O)R_{aa}R_{bb}$, $C_{1-3}$ alkyl, $C_{1-3}$ haloalkyl, or $C_{1-3}$ alkoxy, and further preferably fluorine, chlorine, -CN, methyl, trifluoromethyl, methoxy, $-SO_2CH_3$, $-SO_2NH_2$, $-SCH_3(=O)(=NH)$, $-SCH_3(=O)(=NCH_3)$, or $-P(=O)(CH_3)_2$;

(3) $R_{aa}$, $R_{bb}$, and $R_{cc}$ are each independently hydrogen or $C_{1-6}$ alkyl, preferably hydrogen or $C_{1-3}$ alkyl, and more preferably hydrogen or methyl;

(4) L is $-(CH_2)_{n1}-$, $-(CH_2)_{n1}C(O)(CH_2)_{n2}-$, $-(CH_2)_{n1}S(O)_2(CH_2)_{n2}-$, $-(CH_2)_{n1}C(O)NH(CH_2)_{n2}-$, - $(CH_2)_{n1}NHC(O)(CH_2)_{n2}-$, or $-(CH_2)_{n1}NHS(O)_2(CH_2)_{n2}-$, preferably $-C(O)(CH_2)_{n2}-$, $-C(O)NH(CH_2)_{n2}-$, - $(CH_2)_{n1}NHC(O)-$, or $-(CH_2)_{n1}NHS(O)_2-$, and more preferably $-C(O)(CH_2)_{n2}-$;

(5) $R_x$ and $R_y$ are each independently hydrogen, deuterium, halogen, hydroxyl, $C_{1-6}$ alkyl, $C_{1-6}$ haloalkyl, $C_{1-6}$ alkoxy, or $C_{3-8}$ cycloalkyl, preferably hydrogen, deuterium, halogen, hydroxyl, or $C_{1-6}$ alkyl, and more preferably hydrogen;

(6) $R_1$ and $R_2$ are each independently hydrogen, deuterium, halogen, hydroxyl, $C_{1-6}$ alkyl, $C_{2-6}$ alkenyl, $C_{2-6}$ alkynyl, $C_{1-6}$ haloalkyl, $C_{1-6}$ alkoxy, $-(CH_2)_{n3}OR_{dd}$, $-(CH_2)_{n3}SR_{dd}$, or $-(CH_2)_{n3}C(O)R_{dd}$, preferably hydrogen, deuterium, $C_{1-3}$ alkyl, $C_{2-4}$ alkenyl, or $C_{2-4}$ alkynyl, more preferably hydrogen or $C_{1-3}$ alkyl, and further preferably hydrogen or methyl;

(7) $R_{dd}$ and $R_{cc}$ are each independently $C_{1-6}$ alkyl or $C_{1-6}$ haloalkyl, preferably $C_{1-3}$ alkyl;

(8) n is selected from 0, 1, 2, 3, and 4, preferably 0, 1, or 2;

(9) m is selected from 0, 1, 2, 3, and 4, preferably 0, 1, or 2;

(10) n1 is selected from 0, 1, and 2, preferably 0 or 1;

(11) n2 is selected from 0, 1, and 2, preferably 0 or 1; and

(12) n3 is selected from 0, 1, and 2, preferably 0 or 1.

**6.** The compound, the stereoisomer thereof, or the pharmaceutically acceptable salt thereof according to any one of claims 1-5, wherein general formula (I) further has a structure represented by general formula (IV):

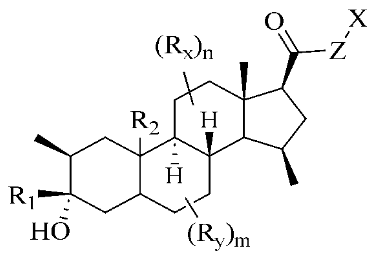

**(IV)**

wherein:

Z is $-(CH_2)_{n4}-$ or $-NH(CH_2)_{n4}-$, preferably $-(CH_2)_{n4}-$, and more preferably $-CH_2-$;

$R_1$ is hydrogen, deuterium, halogen, hydroxyl, $C_{1-6}$ alkyl, $C_{2-6}$ alkenyl, $C_{2-6}$ alkynyl, $C_{1-6}$ haloalkyl, $C_{1-6}$ alkoxy, $-(CH_2)_{n3}OR_{dd}$, $-(CH_2)_{n3}SR_{dd}$, or $-(CH_2)_{n3}C(O)R_{dd}$, preferably $C_{1-6}$ alkyl, more preferably $C_{1-3}$ alkyl, and further preferably methyl;

$R_2$ is hydrogen, deuterium, halogen, hydroxyl, $C_{1-6}$ alkyl, $C_{2-6}$ alkenyl, $C_{2-6}$ alkynyl, $C_{1-6}$ haloalkyl, $C_{1-6}$ alkoxy, $-(CH_2)_{n3}OR_{dd}$, $-(CH_2)_{n3}SR_{dd}$, or $-(CH_2)_{n3}C(O)R_{dd}$, preferably hydrogen or $C_{1-6}$ alkyl, more preferably hydrogen or $C_{1-3}$ alkyl, and further preferably hydrogen or methyl;

$R_{dd}$ is $C_{1-6}$ alkyl or $C_{1-6}$ haloalkyl, preferably $C_{1-3}$ alkyl;

n3 is 0, 1, or 2, preferably 0 or 1;

n4 is 0, 1, or 2, preferably 0 or 1;

X, $R_x$, $R_y$, n, and m are as described in any one of claims 1-5.

**7.** The compound, the stereoisomer thereof, or the pharmaceutically acceptable salt thereof according to any one of claims 1-6, wherein general formula (I) further has a structure represented by general formula (V):

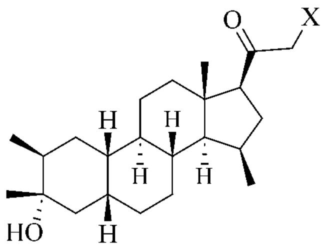

**(V)**

wherein:

X is hydrogen or 5- to 10-membered heteroaryl optionally substituted with one or more $R_A$, preferably hydrogen or 5- to 10-membered heteroaryl containing 1-4 nitrogen atoms and optionally substituted with one or more $R_a$;
$R_A$ is halogen, hydroxyl, -CN, -$NR_{aa}R_{bb}$, -C(=O)$R_{aa}$, -C(=O)$OR_{aa}$, -OC(=O)$R_{aa}$, -OC(=O)$OR_{aa}$, - C(=O)$NR_{aa}R_{bb}$, -N($R_{aa}$)C(=O)$R_{bb}$, -OC(=O)$NR_{aa}R_{bb}$, -N($R_{aa}$)C(=O)$OR_{bb}$, -N($R_{cc}$)C(=O)$NR_{aa}R_{bb}$, -$SR_{aa}$, - S(=O)$R_{aa}$, -S(=O)$_2R_{aa}$, -S(=O)$_2OR_{aa}$, -OS(=O)$_2R_{aa}$, -S(=O)$_2NR_{aa}R_{bb}$, -S(=O)(=$NR_{aa}$)$R_{bb}$, -N($R_{aa}$)S(=O)$_2R_{bb}$, -P(=O) $R_{aa}R_{bb}$, $C_{1-6}$ alkyl, $C_{2-6}$ alkenyl, $C_{2-6}$ alkynyl, $C_{1-6}$ haloalkyl, $C_{1-6}$ alkoxy, $C_{1-6}$ haloalkoxy, $C_{3-8}$ cycloalkyl, or 3- to 8-membered heterocyclyl, preferably halogen, -CN, -S(=O)$_2R_{aa}$, -S(=O)$_2NR_{aa}R_{bb}$, - S(=O)(=$NR_{aa}$)$R_{bb}$, -P(=O)$R_{aa}R_{bb}$, $C_{1-6}$ alkyl, $C_{1-6}$ haloalkyl, or $C_{1-6}$ alkoxy, more preferably halogen, -CN, - S(=O)2$R_{aa}$, -S(=O)$_2$N-$R_{aa}R_{bb}$, -S(=O)(=$NR_{aa}$)$R_{bb}$, -P(=O)$R_{aa}R_{bb}$, $C_{1-3}$ alkyl, $C_{1-3}$ haloalkyl, or $C_{1-3}$ alkoxy, and further preferably fluorine, chlorine, -CN, methyl, trifluoromethyl, methoxy, -$SO_2CH_3$, -$SO_2NH_2$, - $SCH_3$(=O)(=NH), -$SCH_3$(=O) (=$NCH_3$), or -P(=O)$(CH_3)_2$;
$R_{aa}$, $R_{bb}$, and $R_{cc}$ are each independently hydrogen, $C_{1-6}$ alkyl, or $C_{1-6}$ haloalkyl, preferably hydrogen or $C_{1-6}$ alkyl, more preferably hydrogen or $C_{1-3}$ alkyl, and further preferably hydrogen or methyl.

**8.** The compound, the stereoisomer thereof, or the pharmaceutically acceptable salt thereof according to any one of claims 1-7, wherein X is hydrogen or the following group:

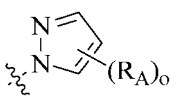 , 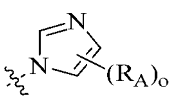 , 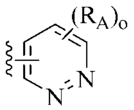 , 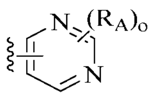 , 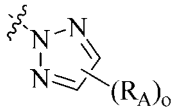 , 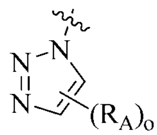 ,

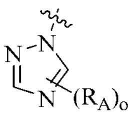 , 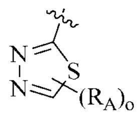 , 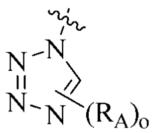 , 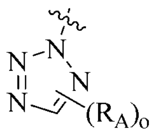 , 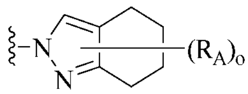 , 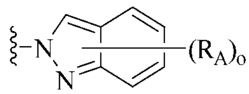 ,

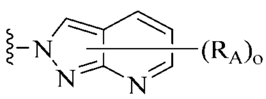 , 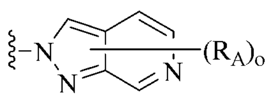 , 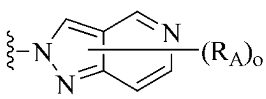 , 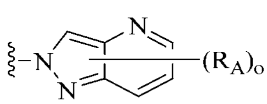 ,

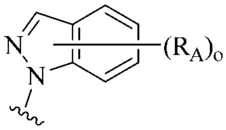 , 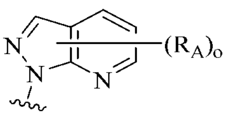 , 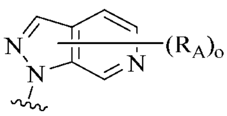 , 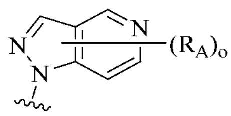 ,

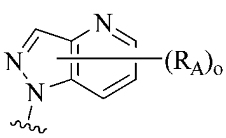 , 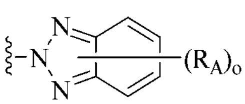 , 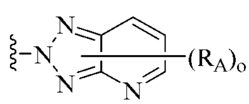 , 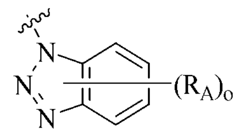 ,

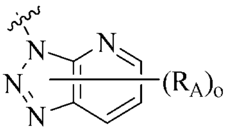, 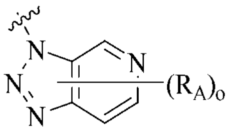, 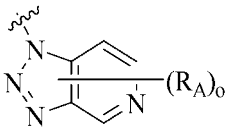, or 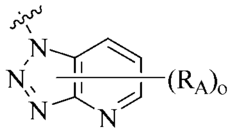,

preferably hydrogen,

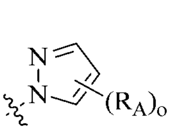, 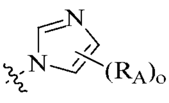, 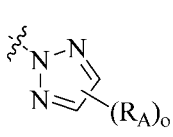, 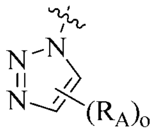, 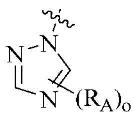,

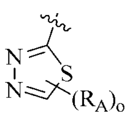, 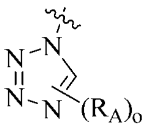, 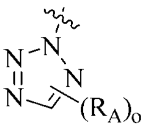, 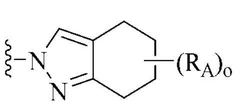, 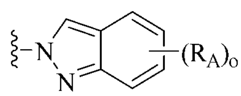,

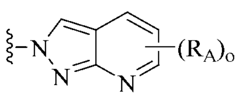, 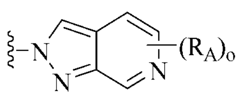, 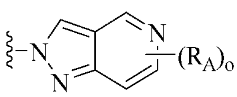, 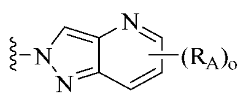,

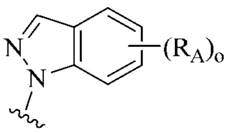, 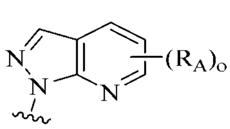, or 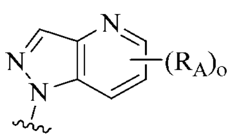;

$R_A$ is halogen, hydroxyl, -CN, $-NR_{aa}R_{bb}$, $-C(=O)R_{aa}$, $-C(=O)OR_{aa}$, $-OC(=O)R_{aa}$, $-OC(=O)OR_{aa}$, $-C(=O)NR_{aa}R_{bb}$, $-N(R_{aa})C(=O)R_{bb}$, $-OC(=O)NR_{aa}R_{bb}$, $-N(R_{aa})C(=O)OR_{bb}$, $-N(R_{cc})C(=O)NR_{aa}R_{bb}$, $-SR_{aa}$, $-S(=O)R_{aa}$, $-S(=O)_2R_{aa}$, $-S(=O)_2OR_{aa}$, $-OS(=O)_2R_{aa}$, $-S(=O)_2NR_{aa}R_{bb}$, $-S(=O)(=NR_{aa})R_{bb}$, $-N(R_{aa})S(=O)_2R_{bb}$, $-P(=O)R_{aa}R_{bb}$, $C_{1-6}$ alkyl, $C_{2-6}$ alkenyl, $C_{2-6}$ alkynyl, $C_{1-6}$ haloalkyl, $C_{1-6}$ alkoxy, $C_{1-6}$ haloalkoxy, $C_{3-8}$ cycloalkyl, or 3- to 8-membered heterocyclyl, preferably halogen, -CN, $-S(=O)_2R_{aa}$, $-S(=O)_2NR_{aa}R_{bb}$, $-S(=O)(=NR_{aa})R_{bb}$, $-P(=O)R_{aa}R_{bb}$, $C_{1-6}$ alkyl, $C_{1-6}$ haloalkyl, or $C_{1-6}$ alkoxy, more preferably halogen, -CN, - S(=O)2$R_{aa}$, $-S(=O)_2N-R_{aa}R_{bb}$, $-S(=O)(=NR_{aa})R_{bb}$, $-P(=O)R_{aa}R_{bb}$, $C_{1-3}$ alkyl, $C_{1-3}$ haloalkyl, or $C_{1-3}$ alkoxy, and further preferably fluorine, chlorine, -CN, methyl, trifluoromethyl, methoxy, $-SO_2CH_3$, $-SO_2NH_2$, - $SCH_3(=O)(=NH)$, $-SCH_3(=O)(=NCH_3)$, or $-P(=O)(CH_3)_2$;

$R_{aa}$, $R_{bb}$, and $R_{cc}$ are each independently hydrogen, $C_{1-6}$ alkyl, or $C_{1-6}$ haloalkyl, preferably hydrogen or $C_{1-6}$ alkyl, more preferably hydrogen or $C_{1-3}$ alkyl, and further preferably hydrogen or methyl;

o is selected from 0, 1, 2, 3, and 4, preferably 0 or 1.

**9.** The compound, the stereoisomer thereof, or the pharmaceutically acceptable salt thereof according to any one of claims 1-6, wherein general formula (I) further has a structure represented by general formula (VI):

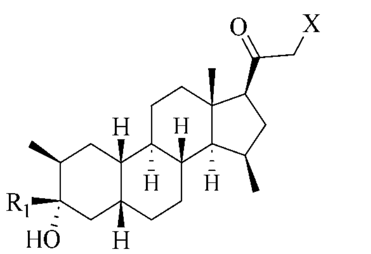

**(VI)**

wherein:

$R_1$ is hydrogen, deuterium, halogen, hydroxyl, $C_{1-6}$ alkyl, $C_{2-6}$ alkenyl, $C_{2-6}$ alkynyl, $C_{1-6}$ haloalkyl, or $C_{1-6}$ alkoxy, preferably hydrogen, deuterium, or $C_{1-6}$ alkyl, more preferably hydrogen, deuterium, or $C_{1-3}$ alkyl, and further preferably hydrogen, deuterium, or methyl;

X is hydrogen or 5- to 10-membered heteroaryl optionally substituted with one or more $R_A$, preferably hydrogen or 5- to 10-membered heteroaryl containing 1-4 nitrogen atoms and optionally substituted with one or more $R_A$, and more preferably hydrogen,

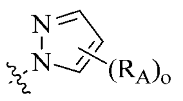, 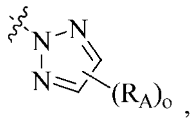, 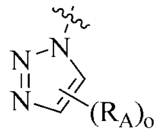,

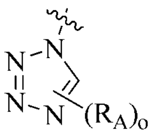, 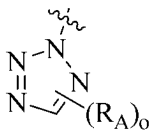, 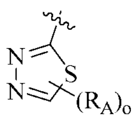, or 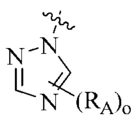;

$R_A$ is halogen, hydroxyl, cyano, $C_{1-6}$ alkyl, $C_{2-6}$ alkenyl, $C_{2-6}$ alkynyl, $C_{1-6}$ haloalkyl, $C_{1-6}$ alkoxy, or $C_{1-6}$ haloalkoxy, preferably cyano, $C_{1-6}$ alkyl, or $C_{1-6}$ haloalkyl, more preferably cyano, $C_{1-3}$ alkyl, or $C_{1-3}$ haloalkyl, and further preferably cyano, methyl, or trifluoromethyl;

o is selected from 0, 1, and 2, preferably 0 or 1.

**10.** The compound, the stereoisomer thereof, or the pharmaceutically acceptable salt thereof according to any one of claims 1-3, wherein general formula (I) further has a structure represented by general formula (VII):

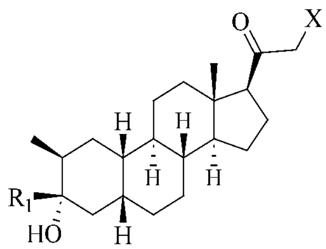

**(VII)**

wherein:

$R_1$ is hydrogen, deuterium, halogen, hydroxyl, $C_{1-6}$ alkyl, $C_{2-6}$ alkenyl, $C_{2-6}$ alkynyl, $C_{1-6}$ haloalkyl, or $C_{1-6}$ alkoxy, preferably hydrogen, deuterium, or $C_{1-6}$ alkyl, more preferably hydrogen, deuterium, or $C_{1-3}$ alkyl, and further preferably hydrogen, deuterium, or methyl;

X is 5- to 10-membered heteroaryl optionally substituted with one or more $R_A$, preferably 5- to 10-membered heteroaryl containing 1-4 nitrogen atoms and optionally substituted with one or more $R_A$, and more preferably

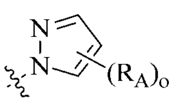, 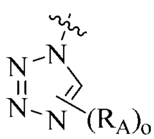, 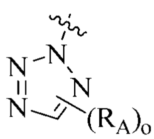, or 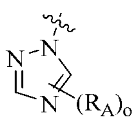;

$R_A$ is halogen, hydroxyl, cyano, $C_{1-6}$ alkyl, $C_{2-6}$ alkenyl, $C_{2-6}$ alkynyl, $C_{1-6}$ haloalkyl, $C_{1-6}$ alkoxy, or $C_{1-6}$ haloalkoxy, preferably cyano or $C_{1-6}$ alkyl, more preferably cyano or $C_{1-3}$ alkyl, and further preferably cyano or methyl;

o is selected from 0, 1, and 2, preferably 0 or 1.

**11.** The compound, the stereoisomer thereof, or the pharmaceutically acceptable salt thereof according to claim 1 or 2, wherein general formula (I) further has a structure represented by general formula (VIII):

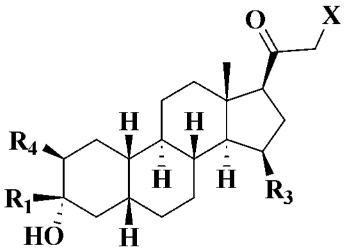

(VIII)

wherein:

$R_1$ is hydrogen, deuterium, or $C_{1-6}$ alkyl, preferably hydrogen, deuterium, or $C_{1-3}$ alkyl, and more preferably hydrogen or methyl;
$R_3$ is hydrogen, deuterium, or $C_{1-6}$ alkyl, preferably hydrogen, deuterium, or $C_{1-3}$ alkyl, and more preferably hydrogen or methyl;
$R_4$ is $C_{1-6}$ alkyl, preferably $C_{1-3}$ alkyl, and more preferably methyl;
X is 5-membered heteroaryl containing 1-4 nitrogen atoms and optionally substituted with one or more $R_A$, preferably

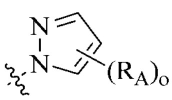 , 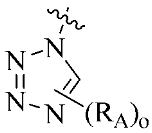 , 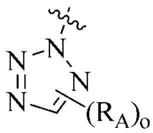 , 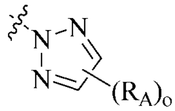 , 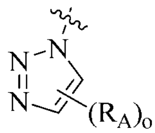 , or

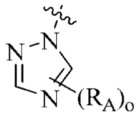 ;

$R_A$ is halogen, -CN, $C_{1-6}$ alkyl, $C_{1-6}$ haloalkyl, $C_{1-6}$ alkoxy, or $C_{3-8}$ cycloalkyl, preferably halogen, -CN, $C_{1-3}$ alkyl, $C_{1-3}$ haloalkyl, or $C_{1-3}$ alkoxy, and more preferably fluorine, chlorine, -CN, methyl, ethyl, trifluoromethyl, or methoxy;
o is selected from 0, 1, and 2.

**12.** The compound, the stereoisomer thereof, or the pharmaceutically acceptable salt thereof according to any one of claims 1, 2, and 11, wherein general formula (I) further has a structure represented by general formula (VIII-A) or (VIII-B):

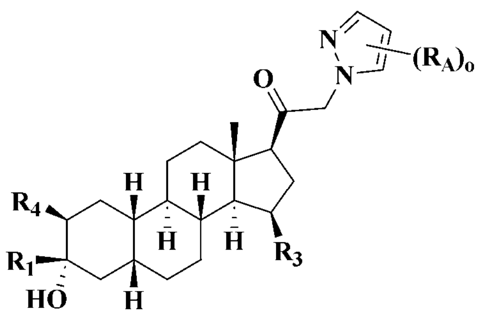

(VIII-A)

or

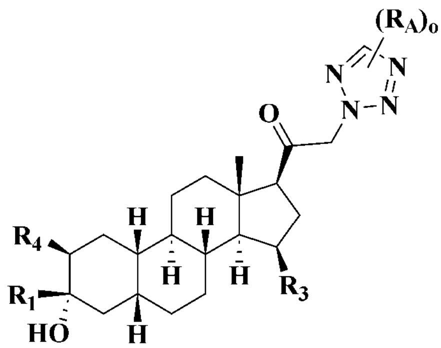

(VIII-B)

,

wherein:

$R_1$ is hydrogen, deuterium, or $C_{1-6}$ alkyl, preferably hydrogen, deuterium, or $C_{1-3}$ alkyl, and more preferably hydrogen or methyl;

$R_3$ is hydrogen, deuterium, or $C_{1-6}$ alkyl, preferably hydrogen, deuterium, or $C_{1-3}$ alkyl, and more preferably hydrogen or methyl;

$R_4$ is $C_{1-6}$ alkyl, preferably $C_{1-3}$ alkyl, and more preferably methyl;

$R_A$ is halogen, -CN, $C_{1-6}$ alkyl, $C_{1-6}$ haloalkyl, $C_{1-6}$ alkoxy, or $C_{3-8}$ cycloalkyl, preferably halogen, -CN, $C_{1-3}$ alkyl, $C_{1-3}$ haloalkyl, or $C_{1-3}$ alkoxy, and more preferably fluorine, chlorine, -CN, methyl, ethyl, trifluoromethyl, or methoxy;

o is selected from 0, 1, and 2.

**13.** The compound, the stereoisomer thereof, or the pharmaceutically acceptable salt thereof according to claim 1, which is selected from the following compounds:

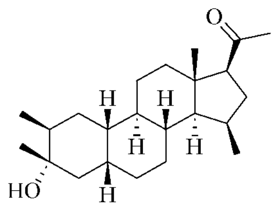
1 ,

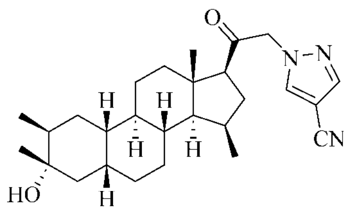
2 ,

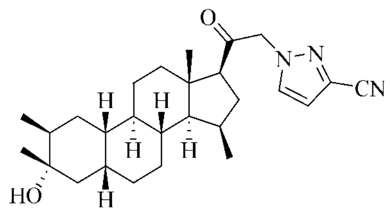
3 ,

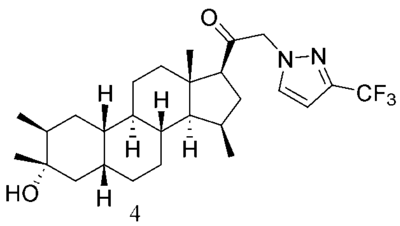
4 ,

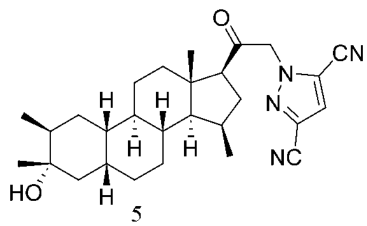
5 ,

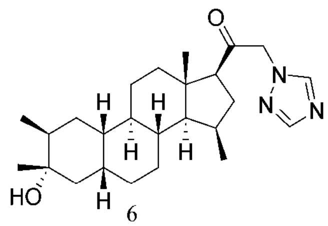
6 ,

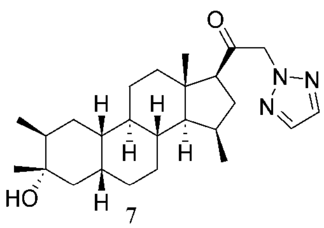
7 ,

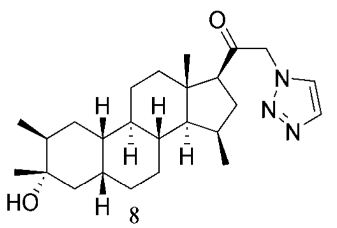
8 ,

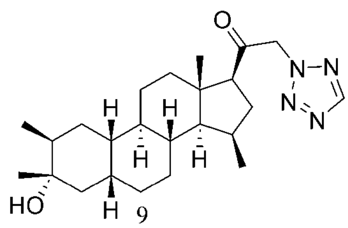
9 ,

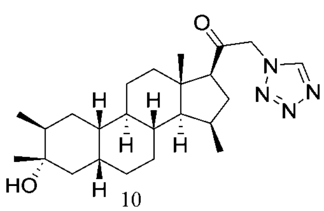
10 ,

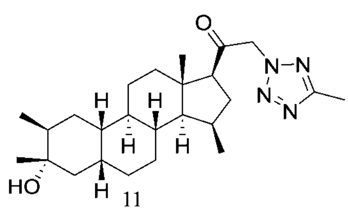
11 ,

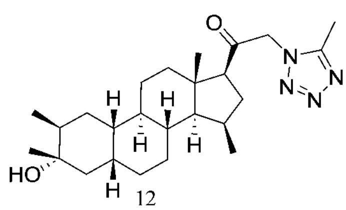
12 ,

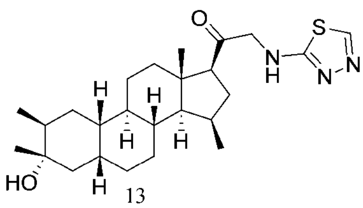
13 ,

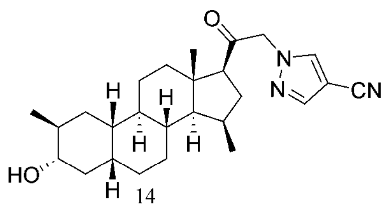
14 ,

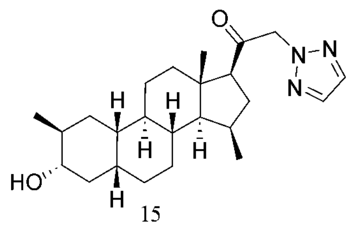
15 ,

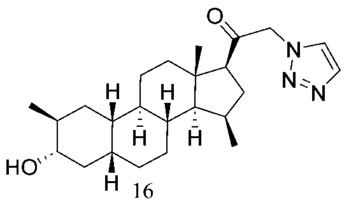
16,

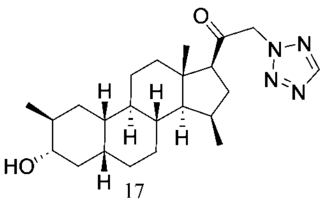
17,

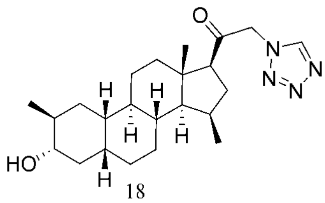
18,

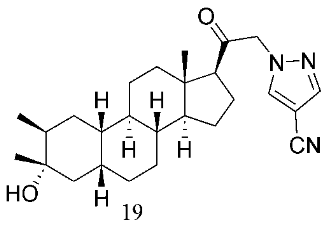
19,

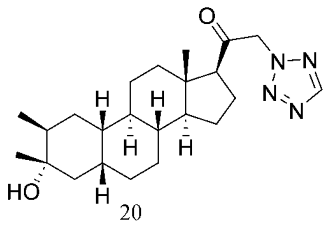
20,

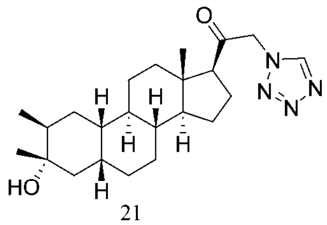
21,

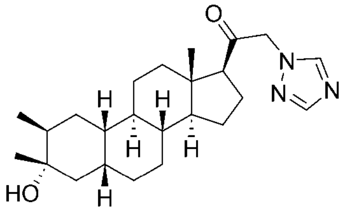
22,

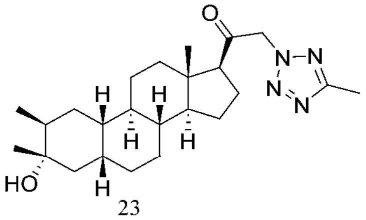
23,

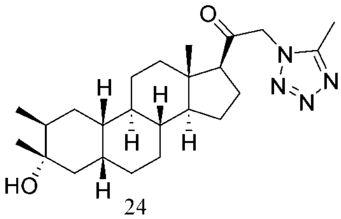
24,

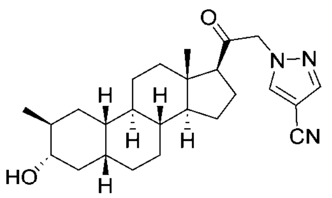
25,

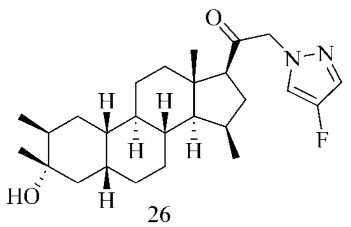
26,

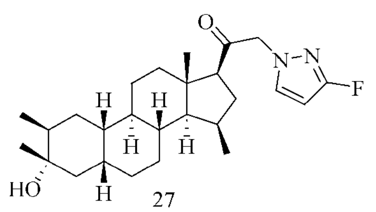
27,

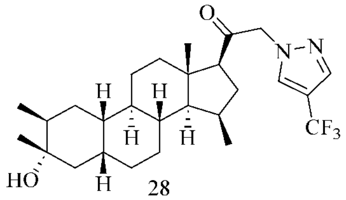
28,

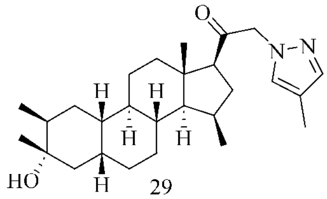
29,

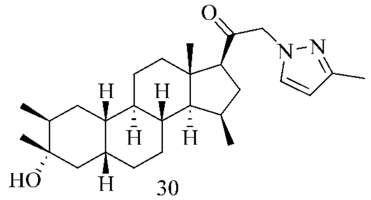
30,

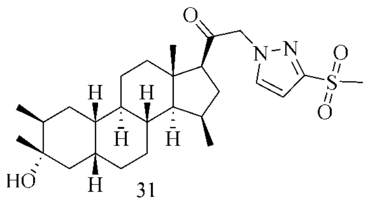
31,

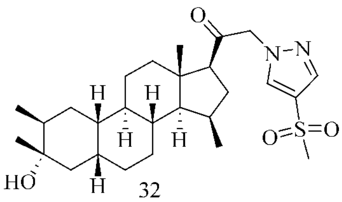
32,

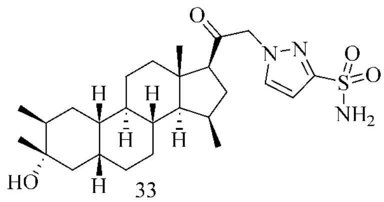
33,

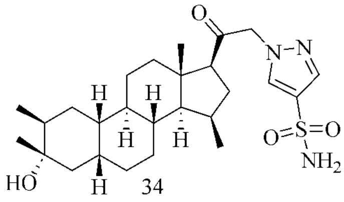
34 ,

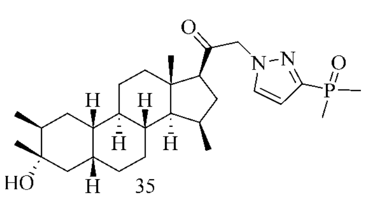
35 ,

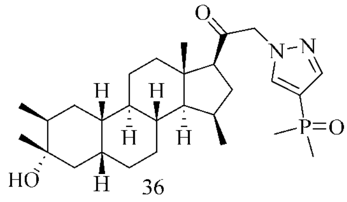
36 ,

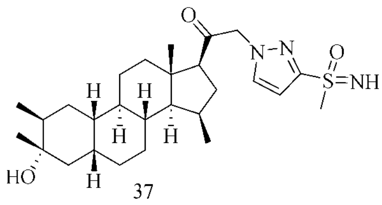
37 ,

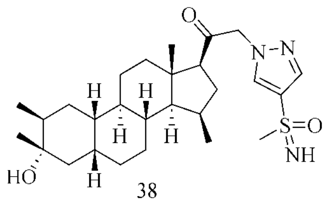
38 ,

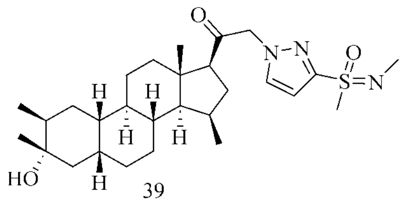
39 ,

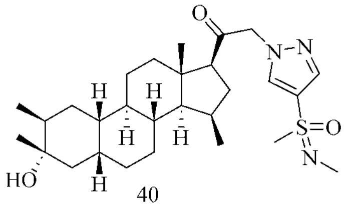
40 ,

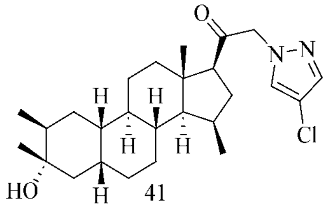
41 ,

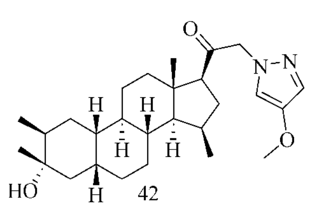
42 ,

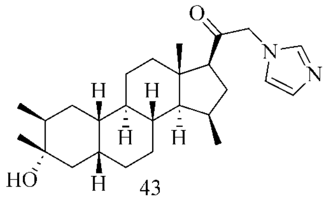
43 ,

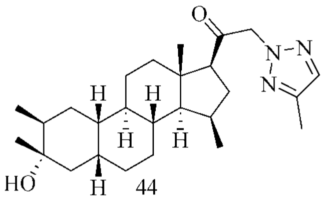
44 ,

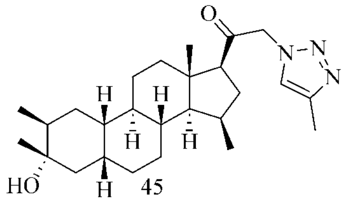
45 ,

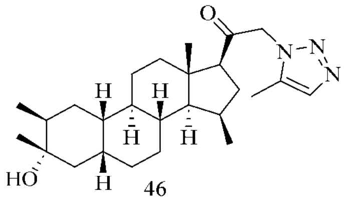
46 ,

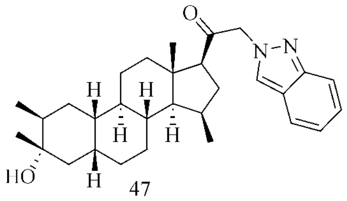
47 ,

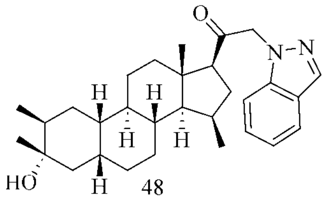
48 ,

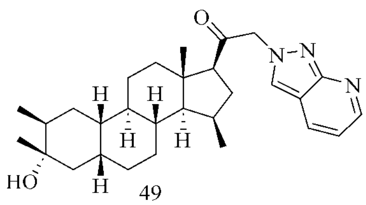
49 ,

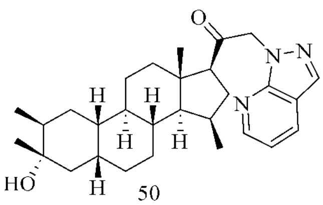
50 ,

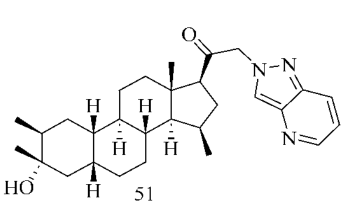
51 ,

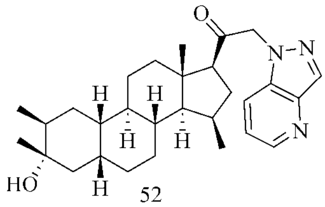
52 ,

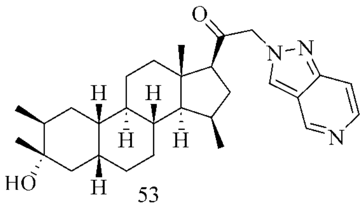
53 ,

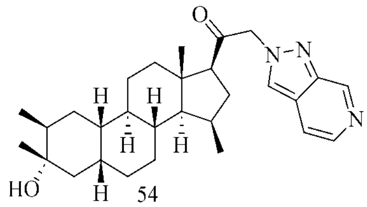
54 ,

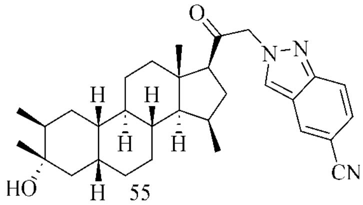 55,

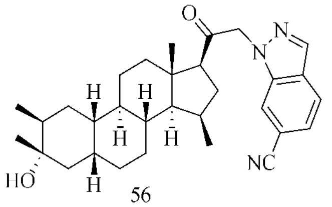 56,

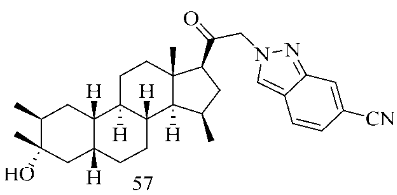 57,

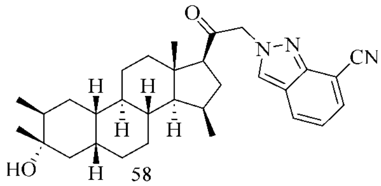 58,

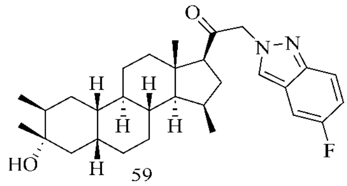 59,

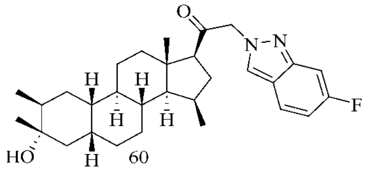 60,

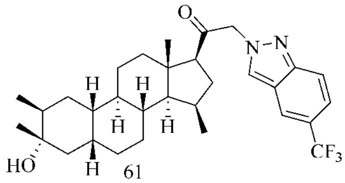 61,

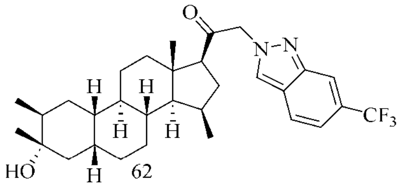 62,

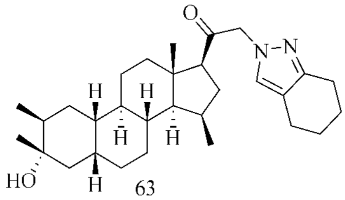 63,

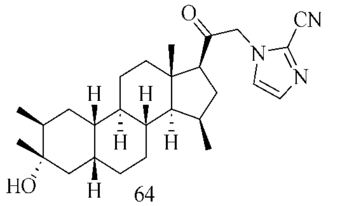 64,

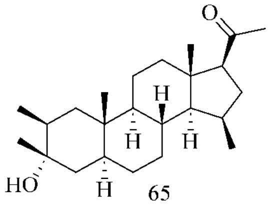 65,

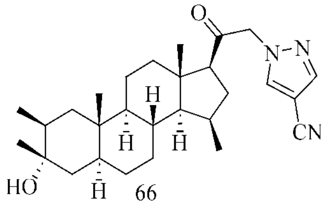 66,

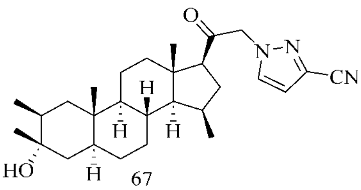 67, and

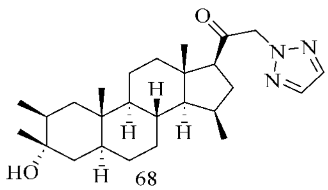 68.

**14.** A pharmaceutical composition, comprising a therapeutically effective amount of the compound, the stereoisomer thereof, or the pharmaceutically acceptable salt thereof according to any one of claims 1-13, and at least one pharmaceutical adjuvant among pharmaceutically acceptable carriers, diluents or excipients.

**15.** Use of the compound, the stereoisomer thereof, or the pharmaceutically acceptable salt thereof according to any one of claims 1-13 or the pharmaceutical composition according to claim 14 in the preparation of a GABAa receptor modulator medicament.

**16.** Use of the compound, the stereoisomer thereof, or the pharmaceutically acceptable salt thereof according to any one of claims 1-13 or the pharmaceutical composition according to claim 14 in the preparation of a medicament for treating a CNS-related disease, wherein preferably, the CNS-related disease is: sleep disorder, mood disorder, premenstrual dysphoric disorder, schizophrenia spectrum disorder, convulsive disorder, memory disorder and/or cognitive disorder, movement disorder, personality disorder, autism spectrum disorder, depression, postpartum depression, major depressive disorder, perimenopausal depression, anxiety disorder, premenstrual anxiety disorder, epilepsy, pain, traumatic brain injury, vascular disease, substance use disorder and/or withdrawal syndrome, or tinnitus; more preferably, the CNS-related disease is depression, postpartum depression, major depressive disorder, perimenopausal depression, anxiety disorder, premenstrual dysphoric disorder, or epilepsy.

**INTERNATIONAL SEARCH REPORT**

International application No.
**PCT/CN2024/092185**

**A. CLASSIFICATION OF SUBJECT MATTER**

C07J7/00(2006.01)i; C07J5/00(2006.01)i; C07J9/00(2006.01)i; A61K31/58(2006.01)i; A61K31/57(2006.01)i; A61K31/56(2006.01)i; A61P25/00(2006.01)i

According to International Patent Classification (IPC) or to both national classification and IPC

**B. FIELDS SEARCHED**

Minimum documentation searched (classification system followed by classification symbols)

C07J A61K A61P

Documentation searched other than minimum documentation to the extent that such documents are included in the fields searched

Electronic data base consulted during the international search (name of data base and, where practicable, search terms used)

CNABS, CNTXT, ENTXT, ISI web of knowledge, STN: 枢境生物科技, 恩华药业, 甾体, 类固醇, SHUJING BIOPHARMA, NHWA PHARMACEUTICAL, STEROIDS, GABAa, CNS, 结构式检索, structural formula search.

**C. DOCUMENTS CONSIDERED TO BE RELEVANT**

| Category* | Citation of document, with indication, where appropriate, of the relevant passages | Relevant to claim No. |
|---|---|---|
| X | WO 2015180679 A1 (SAGE THERAPEUTICS, INC. et al.) 03 December 2015 (2015-12-03) claims 1-34 | 1-16 |
| X | WO 2022040545 A1 (INTRA-CELLULAR THERAPIES, INC.) 24 February 2022 (2022-02-24) embodiment 1 and claims 1-23 | 1-16 |
| X | WO 0066614 A1 (COCENSYS, INC. et al.) 09 November 2000 (2000-11-09) embodiments 1-4 and claims 1-13 | 1-16 |
| X | WO 2021113068 A1 (SPARX THERAPEUTICS, INC.) 10 June 2021 (2021-06-10) embodiments 6-8 and 15-16, and claims 1-48 | 1-16 |
| X | WO 2017156103 A1 (SAGE THERAPEUTICS, INC. et al.) 14 September 2017 (2017-09-14) claims 1-65 | 1-16 |
| X | WO 2022177718 A1 (SAGE THERAPEUTICS, INC.) 25 August 2022 (2022-08-25) claim 1 | 1-16 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 July 2024** | **19 July 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/092185**

**C. DOCUMENTS CONSIDERED TO BE RELEVANT**

| Category* | Citation of document, with indication, where appropriate, of the relevant passages | Relevant to claim No. |
|---|---|---|
| A | WO 2020150210 A1 (BEIJING XUANYI PHARMASCIENCES CO., LTD.) 23 July 2020 (2020-07-23)<br>claims 1-40 | 1-16 |
| A | WO 2022115381 A1 (SAGE THERAPEUTICS, INC.) 02 June 2022 (2022-06-02)<br>claims 1-196 | 1-16 |

Form PCT/ISA/210 (second sheet) (July 2022)

**INTERNATIONAL SEARCH REPORT**
**Information on patent family members**

International application No.
**PCT/CN2024/092185**

| Patent document cited in search report | Publication date (day/month/year) | Patent family member(s) | Publication date (day/month/year) |
|---|---|---|---|
| WO 2015180679 A1 | 03 December 2015 | ES 2927007 T3 | 31 October 2022 |
| | | AU 2015266447 A1 | 01 December 2016 |
| | | RU 2016151727 A | 02 July 2018 |
| | | RU 2016151727 A3 | 03 December 2018 |
| | | RU 2699359 C2 | 05 September 2019 |
| | | HRP 20201126 T1 | 11 December 2020 |
| | | BR 112016027508 A2 | 26 June 2018 |
| | | US 2020165291 A1 | 28 May 2020 |
| | | AU 2019279910 A1 | 16 January 2020 |
| | | AU 2019279910 B2 | 22 July 2021 |
| | | NZ 726260 A | 24 November 2023 |
| | | NZ 764921 A | 24 November 2023 |
| | | MX 2021005099 A | 15 June 2021 |
| | | KR 20170003706 A | 09 January 2017 |
| | | KR 102494061 B1 | 31 January 2023 |
| | | HUE 059829 T2 | 28 December 2022 |
| | | US 2021061849 A1 | 04 March 2021 |
| | | US 2018179247 A1 | 28 June 2018 |
| | | KR 20240066304 A | 14 May 2024 |
| | | AR 100691 A1 | 26 October 2016 |
| | | KR 20230021165 A | 13 February 2023 |
| | | KR 102664412 B1 | 21 May 2024 |
| | | PL 3705488 T3 | 05 December 2022 |
| | | TW 201625661 A | 16 July 2016 |
| | | JP 2020073577 A | 14 May 2020 |
| | | JP 6892936 B2 | 23 June 2021 |
| | | SI 3705488 T1 | 28 October 2022 |
| | | LT 3705488 T | 26 September 2022 |
| | | PH 12016502342 A1 | 13 February 2017 |
| | | JO 3691 B1 | 27 August 2020 |
| | | TW 202231652 A | 16 August 2022 |
| | | SG 11201609813 XA | 29 December 2016 |
| | | JP 2017516793 A | 22 June 2017 |
| | | JP 6652934 B2 | 26 February 2020 |
| | | EP 3149018 A1 | 05 April 2017 |
| | | EP 3149018 A4 | 06 December 2017 |
| | | EP 3149018 B1 | 29 April 2020 |
| | | IL 304033 A | 01 August 2023 |
| | | MX 2021005106 A | 15 June 2021 |
| | | US 2021061850 A1 | 04 March 2021 |
| | | RS 60529 B1 | 31 August 2020 |
| | | AU 2021204410 A1 | 22 July 2021 |
| | | AU 2021204410 B2 | 21 September 2023 |
| | | ES 2805598 T3 | 12 February 2021 |
| | | NZ 764941 A | 24 November 2023 |
| | | MX 2016015532 A | 27 February 2017 |
| | | IL 248948 A0 | 31 January 2017 |
| | | IL 248948 B | 01 September 2022 |
| | | SG 10201803812 TA | 28 June 2018 |
| | | HUE 050028 T2 | 28 December 2021 |

Form PCT/ISA/210 (patent family annex) (July 2022)

**INTERNATIONAL SEARCH REPORT**
**Information on patent family members**

International application No.
**PCT/CN2024/092185**

| Patent document cited in search report | Publication date (day/month/year) | Patent family member(s) | Publication date (day/month/year) |
|---|---|---|---|
| | | RS 63554 B1 | 30 September 2022 |
| | | EP 4144746 A1 | 08 March 2023 |
| | | PL 3149018 T3 | 25 January 2021 |
| | | LT 3149018 T | 10 August 2020 |
| | | DK 3149018 T3 | 20 July 2020 |
| | | DK 3705488 T3 | 03 October 2022 |
| | | PT 3149018 T | 21 July 2020 |
| | | IL 292461 A | 01 June 2022 |
| | | IL 292461 B1 | 01 November 2023 |
| | | IL 292461 B2 | 01 March 2024 |
| | | CY 1124109 T1 | 24 March 2022 |
| | | RU 2019126637 A | 03 October 2019 |
| | | US 2024076310 A1 | 07 March 2024 |
| | | JP 2023123878 A | 05 September 2023 |
| | | HRP 20221294 T1 | 03 March 2023 |
| | | SI 3149018 T1 | 30 October 2020 |
| | | PT 3705488 T | 30 September 2022 |
| | | AU 2023285755 A1 | 18 January 2024 |
| | | AR 121661 A2 | 29 June 2022 |
| | | MX 2023004352 A | 08 May 2023 |
| | | CA 2949720 A1 | 03 December 2015 |
| | | EP 3705488 A1 | 09 September 2020 |
| | | EP 3705488 B1 | 03 August 2022 |
| | | JP 2021130696 A | 09 September 2021 |
| | | JP 7386204 B2 | 24 November 2023 |
| WO 2022040545 A1 | 24 February 2022 | JP 2023539125 A | 13 September 2023 |
| | | EP 4200312 A1 | 28 June 2023 |
| | | US 2023416300 A1 | 28 December 2023 |
| WO 0066614 A1 | 09 November 2000 | JP 2002543218 A | 17 December 2002 |
| | | US 2005171074 A1 | 04 August 2005 |
| | | HK 1047594 A1 | 28 February 2003 |
| | | AU 4810400 A | 17 November 2000 |
| | | AU 780989 B2 | 28 April 2005 |
| | | RU 2243232 C2 | 27 December 2004 |
| | | CZ 20013867 A3 | 17 July 2002 |
| | | IL 146230 A0 | 25 July 2002 |
| | | PL 351438 A1 | 22 April 2003 |
| | | BR 0010060 A | 15 January 2002 |
| | | KR 20020013530 A | 20 February 2002 |
| | | WO 0066614 A8 | 15 March 2001 |
| | | NO 20015262 D0 | 26 October 2001 |
| | | NO 20015262 L | 19 December 2001 |
| | | NO 321536 B1 | 22 May 2006 |
| | | HUP 0201818 A2 | 28 October 2002 |
| | | HUP 0201818 A3 | 28 April 2004 |
| | | EP 1177206 A1 | 06 February 2002 |
| | | US 2004034002 A1 | 19 February 2004 |
| | | CA 2372342 A1 | 09 November 2000 |
| | | ZA 200109847 B | 26 February 2003 |
| | | YU 77701 A | 19 July 2005 |

Form PCT/ISA/210 (patent family annex) (July 2022)

**INTERNATIONAL SEARCH REPORT**
**Information on patent family members**

International application No.
**PCT/CN2024/092185**

| Patent document cited in search report | Publication date (day/month/year) | Patent family member(s) | Publication date (day/month/year) |
|---|---|---|---|
| | | NZ 515779 A | 28 November 2003 |
| | | MXPA 01010915 A | 07 November 2002 |
| | | UA 73736 C2 | 15 September 2005 |
| WO 2021113068 A1 | 10 June 2021 | US 2021188897 A1 | 24 June 2021 |
| WO 2017156103 A1 | 14 September 2017 | CA 3017172 A1 | 14 September 2017 |
| | | CA 3017172 C | 14 March 2023 |
| | | RU 2018135079 A | 08 April 2020 |
| | | RU 2018135079 A3 | 16 April 2020 |
| | | RU 2766155 C2 | 08 February 2022 |
| | | US 2023355639 A1 | 09 November 2023 |
| | | KR 20180115797 A | 23 October 2018 |
| | | KR 102408399 B1 | 13 June 2022 |
| | | US 2020171049 A1 | 04 June 2020 |
| | | US 10940156 B2 | 09 March 2021 |
| | | AU 2017229656 A1 | 11 October 2018 |
| | | AU 2017229656 B2 | 29 September 2022 |
| | | AU 2022283769 A1 | 02 February 2023 |
| | | IL 300422 A | 01 April 2023 |
| | | EP 3426257 A1 | 16 January 2019 |
| | | EP 3426257 A4 | 13 November 2019 |
| | | JP 2019511488 A | 25 April 2019 |
| | | JP 6838074 B2 | 03 March 2021 |
| | | JP 2023093723 A | 04 July 2023 |
| | | TW 201733592 A | 01 October 2017 |
| | | TWI 798173 B | 11 April 2023 |
| | | MA 43815 A | 07 April 2021 |
| | | US 2020306265 A1 | 01 October 2020 |
| | | JOP 20170059 B1 | 17 August 2021 |
| | | KR 20220084418 A | 21 June 2022 |
| | | JP 2021073309 A | 13 May 2021 |
| | | HK 1258616 A1 | 15 November 2019 |
| | | BR 112018067998 A2 | 16 April 2019 |
| | | MX 2022003896 A | 19 April 2022 |
| | | IL 261658 A | 31 October 2018 |
| | | IL 261658 B1 | 01 March 2023 |
| | | IL 261658 B2 | 01 July 2023 |
| | | TW 202400182 A | 01 January 2024 |
| | | US 2022023313 A1 | 27 January 2022 |
| | | US 11554125 B2 | 17 January 2023 |
| | | ZA 201805905 B | 31 July 2019 |
| | | CA 3158448 A1 | 14 September 2017 |
| | | SG 11201807785 VA | 30 October 2018 |
| | | MX 2018010902 A | 06 March 2019 |
| | | PH 12018501923 A1 | 01 July 2019 |
| | | RU 2022102537 A | 30 March 2022 |
| WO 2022177718 A1 | 25 August 2022 | US 2024148756 A1 | 09 May 2024 |
| WO 2020150210 A1 | 23 July 2020 | US 2022089636 A1 | 24 March 2022 |
| | | EP 3911331 A1 | 24 November 2021 |
| | | EP 3911331 A4 | 18 January 2023 |
| | | TW 202043204 A | 01 December 2020 |

Form PCT/ISA/210 (patent family annex) (July 2022)

**INTERNATIONAL SEARCH REPORT**
**Information on patent family members**

International application No.
**PCT/CN2024/092185**

| Patent document cited in search report | | | Publication date (day/month/year) | Patent family member(s) | | | Publication date (day/month/year) |
|---|---|---|---|---|---|---|---|
| | | | | KR | 20210116523 | A | 27 September 2021 |
| | | | | CA | 3124703 | A1 | 23 July 2020 |
| | | | | CA | 3124703 | C | 17 October 2023 |
| | | | | JP | 2022518359 | A | 15 March 2022 |
| | | | | JP | 7340023 | B2 | 06 September 2023 |
| WO | 2022115381 | A1 | 02 June 2022 | JP | 2023550653 | A | 04 December 2023 |
| | | | | EP | 4251634 | A1 | 04 October 2023 |
| | | | | KR | 20230124916 | A | 28 August 2023 |
| | | | | CA | 3202870 | A1 | 02 June 2022 |
| | | | | TW | 202235089 | A | 16 September 2022 |
| | | | | IL | 303101 | A | 01 July 2023 |
| | | | | WO | 2022115381 | A8 | 06 July 2023 |
| | | | | MX | 2023006063 | A | 09 August 2023 |
| | | | | AU | 2021385335 | A1 | 29 June 2023 |
| | | | | AU | 2021385335 | A9 | 30 May 2024 |
| | | | | US | 2024101592 | A1 | 28 March 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310527779
- CN 202311387436
- CN 202410117316
- CN 105339381 B